# EUROPEAN PATENT SPECIFICATION

(11) **EP 1 735 319 B1**
(45) Date of publication and mention of the grant of the patent: **03.05.2017**
(21) Application number: 05714678.9
(22) Date of filing: 29.03.2005
(51) Int. Cl.: C07D 495/04, C07D 403/12, C07D 213/74, C07D 231/38, C07D 333/38, C07D 401/04, C07C 311/12, C07C 311/21, C07D 235/16, A61K 31/167, A61K 31/40

(54) **INHIBITORS OF HISTONE DEACETYLASE**
HISTONDEACETYLASE-HEMMER
INHIBITEURS D'HISTONE DESACETYLASE

(30) Priority: 26.03.2004 US 556828 P; 25.03.2005 US 90713; 25.03.2005 WO PCT/IB2005/000802
(43) Date of publication of application: 27.12.2006
(73) Proprietor: MethylGene Inc., Montreal, QC H4S 2A1 (CA)
(72) Inventor: DELORME, Daniel, Saint-Lazare, Quebec J7T 2B2 (CA); VAISBURG, Arkadii, Kirkland, Quebec H9J 2X2 (CA); MORADEI, Oscar, Kirkland, Quebec H9J 4A5 (CA); LEIT, Silvana, Kirkland, Quebec H9J 4A5 (CA); RAEPPEL, Stephane, St. Lazare, Quebec J7T 3L8 (CA); FRECHETTE, Sylvie, Verdun, Quebec H4G 1S2 (CA); BOUCHAIN, Giliane, Cedar Valley, Ontario L0G 1E0 (CA); ZHOU, Zhihong, Kirkland, Québec H9J 1N9 (CA); PAQUIN, Isabelle, La Salle, Québec H8P 2K6 (CA); GAUDETTE, Frederic, Verdun, Quebec H4H 1X9 (CA); ISAKOVIC, Ljubomir, Montreal, Quebec H3H 1H4 (CA)
(74) Representative: D Young & Co LLP
(86) International application number: PCT/CA2005/000454
(87) International publication number: WO 2005/092899

(56) References cited:
- WO-A-03/078390
- WO-A-03/092686
- WO-A-2004/005513
- WO-A2-03/024448
- CA-A1- 2 480 356
- CA-A1- 2 484 065
- CA-A1- 2 490 579
- JP-A- 11 269 146
- DATABASE CROSSFIRE BEILSTEIN BEILSTEIN INSTITUT ZUR FOERDERUNG DER CHEMISCHEN WISSENSCHAFTEN, FRANKFURT AM MAIN, DE; XP002578745 Database accession no. 5438997 & JOURNAL OF ORGANIC CHEMISTRY, vol. 57, no. 10, 1992, pages 2883-2887, ISSN: 0022-3263
- DATABASE CROSSFIRE BEILSTEIN BEILSZEIN INSTITUT ZUR FOERDERUNG DER CHEMISCHEN WISSENSCHAFTEN, FRANKFURT AM MAIN, DE; XP002578746 Database accession no. 8853038 & MOLECULES, vol. 6, no. 7, 2001, pages 603-613, ISSN: 1420-3049
- DATABASE CROSSFIRE BEILSTEIN BEILSTEIN INSTITUT ZUR FOERDERUNG DER CHEMISCHEN WISSENSCHAFTEN, FRANKFURT AM MAIN, DE; XP002578747 Database accession no. 323265 & US 2 754 286 A (DU PONT DE NEMOURS AND CO.) 10 July 1956 (1956-07-10)
- DATABASE CROSSFIRE BEILSTEIN BEILSTEIN INSTITUT ZUR FOERDERUNG DER CHEMISCHEN WISSENSCHAFTEN, FRANKFURT AM MAIN, DE; XP002578748 Database accession no. 1675779 & US 4 994 479 A (YAMANOUCHI PHARMACEUTICAL CO., LTD) 19 February 1991 (1991-02-19)
- DATABASE CROSSFIRE BEILSTEIN BEILSTEIN INSTITUT ZUR FOERDERUNG DER CHEMISCHEN WISSENSCHAFTEN, FRANKFURT AM MAIN, DE; XP002578749 Database accession no. 7606264 & JOURNAL OF THE CHEMICAL SOCIETY, DALTON TRANSACTIONS: INORGANIC CHEMISTRY, vol. 11, 1996, pages 2341-2346, ISSN: 0300-9246
- ROBERT J. PERRY ET AL: 'Synthesis of 2-arylbenzoxazoles via the palladium-catalyzed carbonylation and condensation of aromatic halides and o-aminophenols' THE JOURNAL OF ORGANIC CHEMISTRY vol. 57, no. 10, 01 May 1992, pages 2883 - 2887, XP055014282 DOI: 10.1021/jo00036a025 ISSN: 0022-3263
- MIROSLAV MILETÍN ET AL: 'Some Anilides of 2-Alkylthio- and 2-Chloro-6-Alkylthio-4-Pyridinecarboxylic Acids: Synthesis and Photosynthesis-Inhibiting Activity' MOLECULES vol. 6, no. 7, 30 June 2001, pages 603 - 613, XP055014285 DOI: 10.3390/60700603

### SEQUENCE LISTING

<110> Methylgene, Inc.
<120> INHIBITORS OF HISTONE DEACETYLASE
<130> P027152EP
<140> 05714678.9
   <141> 2005-03-29
<150> US 60/556,828
   <151> 2004-03-26
<150> US 11/090,713
   <151> 2005-03-25
<150> PCT/IB05/00802
   <151> 2005-03-25
<160> 17
<170> PatentIn version 3.3
<210> 1
   <211> 20
   <212> DNA
   <213> Artificial
<220>
   <223> Synthetic
<400> 1
   gaaacgtgag ggactcagca 20
<210> 2
   <211> 20
   <212> DNA
   <213> Artificial
<220>
   <223> Synthetic
<400> 2
   ggaagccaga gctggagagg 20
<210> 3
   <211> 20
   <212> DNA
   <213> Artificial
<220>
   <223> Synthetic
<400> 3
   gttaggtgag gcactgagga 20
<210> 4
   <211> 20
   <212> DNA
   <213> Artificial
<220>
   <223> Synthetic
<400> 4
   gctgagctgt tctgatttgg 20
<210> 5
   <211> 20
   <212> DNA
   <213> Artificial
<220>
   <223> Synthetic
<400> 5
   cgtgagcact tctcatttcc 20
<210> 6
   <211> 20
   <212> DNA
   <213> Artificial
<220>
   <223> Synthetic
<400> 6
   cgctttcctt gtcattgaca 20
<210> 7
   <211> 20
   <212> DNA
   <213> Artificial
<220>
   <223> Synthetic
<400> 7
   gcctttccta ctcattgtgt 20
<210> 8
   <211> 20
   <212> DNA
   <213> Artificial
<220>
   <223> Synthetic
<400> 8
   gctgcctgcc gtgcccaccc 20
<210> 9
   <211> 20
   <212> DNA
   <213> Artificial
<220>
   <223> Synthetic
<400> 9
   cgtgcctgcg ctgcccacgg 20
<210> 10
   <211> 20
   <212> DNA
   <213> Artificial
<220>
   <223> Synthetic
<400> 10
   tacagtccat gcaacctcca 20
<210> 11
   <211> 20
   <212> DNA
   <213> Artificial
<220>
   <223> Synthetic
<400> 11
   atcagtccaa ccaacctcgt 20
<210> 12
   <211> 20
   <212> DNA
   <213> Artificial
<220>
   <223> Synthetic
<400> 12
   cttcggtctc acctgcttgg 20
<210> 13
   <211> 20
   <212> DNA
   <213> Artificial
<220>
   <223> Synthetic
<400> 13
   caggctggaa tgagctacag 20
<210> 14
   <211> 20
   <212> DNA
   <213> Artificial
<220>
   <223> Synthetic
<400> 14
   gacgctgcaa tcaggtagac 20
<210> 15
   <211> 20
   <212> DNA
   <213> Artificial
<220>
   <223> Synthetic
<400> 15
   cttcagccag gatgcccaca 20
<210> 16
   <211> 20
   <212> DNA
   <213> Artificial
<220>
   <223> Synthetic
<400> 16
   ctccggctcc tccatcttcc 20
<210> 17
   <211> 20
   <212> DNA
   <213> Artificial
<220>
   <223> Synthetic
<400> 17
   agccagctgc cacttgatgc 20

## Description

### BACKGROUND OF THE INVENTION

### Field of the Invention

This invention relates to the inhibition of histone deacetylase. More particularly, the invention relates to compounds and their use in inhibiting histone deacetylase enzymatic activity.

### Summary of the Related Art

In eukaryotic cells, nuclear DNA associates with histones to form a compact complex called chromatin. The histones constitute a family of basic proteins which are generally highly conserved across eukaryotic species. The core histones, termed H2A, H2B, H3, and H4, associate to form a protein core. DNA winds around this protein core, with the basic amino acids of the histones interacting with the negatively charged phosphate groups of the DNA. Approximately 146 base pairs of DNA wrap around a histone core to make up a nucleosome particle, the repeating structural motif of chromatin.

Csordas, Biochem. J., 286: 23-38 (1990) teaches that histones are subject to posttranslational acetylation of the ε-amino groups of *N*-terminal lysine residues, a reaction that is catalyzed by histone acetyl transferase (HAT1). Acetylation neutralizes the positive charge of the lysine side chain, and is thought to impact chromatin structure. Indeed, Taunton et al., Science, 272: 408-411 (1996), teaches that access of transcription factors to chromatin templates is enhanced by histone hyperacetylation. Taunton et al. further teaches that an enrichment in underacetylated histone H4 has been found in transcriptionally silent regions of the genome.

Histone acetylation is a reversible modification, with deacetylation being catalyzed by a family of enzymes termed histone deacetylases (HDACs). The molecular cloning of gene sequences encoding proteins with HDAC activity has established the existence of a set of discrete HDAC enzyme isoforms. Grozinger et al., Proc. Natl. Acad. Sci. USA, 96: 4868-4873 (1999), teaches that HDACs is divided into two classes, the first represented by yeast Rpd3-like proteins, and the second represented by yeast Hda1-like proteins. Grozinger *et al*. also teaches that the human HDAC1, HDAC2, and HDAC3 proteins are members of the first class of HDACs, and discloses new proteins, named HDAC4, HDAC5, and HDAC6, which are members of the second class of HDACs. Kao et al., Genes & Dev., 14: 55-66 (2000), discloses HDAC7, a new member of the second class of HDACs. Van den Wyngaert, FEBS, 478: 77-83 (2000) discloses HDAC8, a new member of the first class of HDACs. Zhou,X. et al., Proc. Natl. Acad. Sci. U.S.A. 98 (19), 10572-10577 (2001) discloses cloning and characterization of HDAC9. Kao,H.Y. et al., J. Biol. Chem. 277 (1), 187-193 (2002) discloses isolation and characterization of mammalian HDAC10. Gao L. et al., J Biol Chem. 277(28): 25748-55 (2002) discloses cloning and functional characterization of HDAC11.

Richon et al., Proc. Natl. Acad. Sci. USA, 95: 3003-3007 (1998), discloses that HDAC activity is inhibited by trichostatin A (TSA), a natural product isolated from Streptomyces hygroscopicus, and by a synthetic compound, suberoylanilide hydroxamic acid (SAHA). Yoshida and Beppu, Exper. Cell Res., 177: 122-131 (1988), teaches that TSA causes arrest of rat fibroblasts at the G₁ and G₂ phases of the cell cycle, implicating HDAC in cell cycle regulation. Indeed, Finnin et al., Nature, 401: 188-193 (1999), teaches that TSA and SAHA inhibit cell growth, induce terminal differentiation, and prevent the formation of tumors in mice. Suzuki et al., U.S. Pat. No. 6,174,905, EP 0847992, JP 258863/96, and Japanese Application No. 10138957, disclose benzamide derivatives that induce cell differentiation and inhibit HDAC. Delorme et al., WO 01/38322 and PCT IB01/00583, disclose additional compounds that serve as HDAC inhibitors.
CA 2 480 356 A1 discloses benzamide derivatives which are useful as histone deacetylase inhibitors.

These findings suggest that inhibition of HDAC activity represents a novel approach for intervening in cell cycle regulation and that HDAC inhibitors have great therapeutic potential in the treatment of cell proliferative diseases or conditions. To date, few inhibitors of histone deacetylase are known in the art. There is thus a need to identify additional HDAC inhibitors and to identify the structural features required for potent HDAC inhibitory activity.

### BRIEF SUMMARY OF THE INVENTION

The invention provides compounds and their use in treating cell proliferative diseases. The invention provides new inhibitors of histone deacetylase enzymatic activity.

In a first aspect, the invention provides compounds that are useful as inhibitors of histone deacetylase.

In a second aspect, the invention provides a composition comprising an inhibitor of histone deacetylase according to the invention and a pharmaceutically acceptable carrier.

In a third aspect, the invention provides the *in vitro* use of an inhibitor of histone deacetylase of the invention for treating a cell proliferative disease or condition in a cell, comprising contacting a cell in which inhibition of histone deacetylase is desired with the inhibitor of histone deacetylase of the invention.

In a fourth aspect, the invention provides an inhibitor of histone deacetylase of the invention for use in treating cell proliferative diseases.

The foregoing merely summarizes certain aspects of the invention and is not intended to be limiting in nature. These aspects and other aspects and embodiments are described more fully below.

### DETAILED DESCRIPTION OF THE PREFERRED EMBODIMENTS

The invention provides compounds and their use in inhibiting histone deacetylase enzymatic activity. The invention also provides compositions and the use of compounds/compositions for use in treating cell proliferative diseases and conditions. The patent and scientific literature referred to herein establishes knowledge that is available to those with skill in the art.

For purposes of the present invention, the following definitions will be used (unless expressly stated otherwise):

As used herein, the terms "histone deacetylase" and "HDAC" are intended to refer to any one of a family of enzymes that remove acetyl groups from the ε-amino groups of lysine residues at the N-terminus of a histone. Unless otherwise indicated by context, the term "histone" is meant to refer to any histone protein, including H1, H2A, H2B, H3, H4, and H5, from any species. Preferred histone deacetylases include class I and class II enzymes. Preferably the histone deacetylase is a human HDAC, including, but not limited to, HDAC-1, HDAC-2, HDAC-3, HDAC-4, HDAC-5, HDAC-6, HDAC-7, HDAC-8, HDAC-9, HDAC-10, and HDAC-11. In some other preferred embodiments, the histone deacetylase is derived from a protozoal or fungal source.

The terms "histone deacetylase inhibitor" and "inhibitor of histone deacetylase" are used to identify a compound having a structure as defined herein, which is capable of interacting with a histone deacetylase and inhibiting its enzymatic activity. "Inhibiting histone deacetylase enzymatic activity" means reducing the ability of a histone deacetylase to remove an acetyl group from a histone. In some preferred embodiments, such reduction of histone deacetylase activity is at least about 50%, more preferably at least about 75%, and still more preferably at least about 90%. In other preferred embodiments, histone deacetylase activity is reduced by at least 95% and more preferably by at least 99%.

Preferably, such inhibition is specific, i.e., the histone deacetylase inhibitor reduces the ability of a histone deacetylase to remove an acetyl group from a histone at a concentration that is lower than the concentration of the inhibitor that is required to produce another, unrelated biological effect. Preferably, the concentration of the inhibitor required for histone deacetylase inhibitory activity is at least 2-fold lower, more preferably at least 5-fold lower, even more preferably at least 10-fold lower, and most preferably at least 20-fold lower than the concentration required to produce an unrelated biological effect.

For simplicity, chemical moieties are defined and referred to throughout primarily as univalent chemical moieties (*e.g.,* alkyl, aryl, etc.). Nevertheless, such terms are also used to convey corresponding multivalent moieties under the appropriate structural circumstances clear to those skilled in the art. For example, while an "alkyl" moiety generally refers to a monovalent radical (*e.g.* CH₃-CH₂-), in certain circumstances a bivalent linking moiety can be "alkyl," in which case those skilled in the art will understand the alkyl to be a divalent radical (*e.g.,* -CH₂-CH₂-), which is equivalent to the term "alkylene." (Similarly, in circumstances in which a divalent moiety is required and is stated as being "aryl," those skilled in the art will understand that the term "aryl" refers to the corresponding divalent moiety, arylene.) All atoms are understood to have their normal number of valences for bond formation (*i*.e., 4 for carbon, 3 for N, 2 for 0, and 2, 4, or 6 for S, depending on the oxidation state of the S). On occasion a moiety may be defined, for example, as (A)ₐ-B-, wherein a is 0 or 1. In such instances, when a is 0 the moiety is B- and when a is 1 the moiety is A-B-.

The term "hydrocarbyl" refers to a straight, branched, or cyclic alkyl, alkenyl, or alkynyl, each as defined herein. A "C₀" hydrocarbyl is used to refer to a covalent bond. Thus, "C₀-C₃-hydrocarbyl" includes a covalent bond, methyl, ethyl, ethenyl, ethynyl, propyl, propenyl, propynyl, and cyclopropyl.

The term "alkyl" as employed herein refers to straight and branched chain aliphatic groups having from 1 to 12 carbon atoms, preferably 1-8 carbon atoms, and more preferably 1-6 carbon atoms, which is optionally substituted with one, two or three substituents. Preferred alkyl groups include, without limitation, methyl, ethyl, propyl, isopropyl, butyl, isobutyl, sec-butyl, tert-butyl, pentyl, and hexyl. A "C₀" alkyl (as in "C₀-C₃.alkyl") is a covalent bond (like "C₀" hydrocarbyl).

The term "alkenyl" as used herein means an unsaturated straight or branched chain aliphatic group with one or more carbon-carbon double bonds, having from 2 to 12 carbon atoms, preferably 2-8 carbon atoms, and more preferably 2-6 carbon atoms, which is optionally substituted with one, two or three substituents. Preferred alkenyl groups include, without limitation, ethenyl, propenyl, butenyl, pentenyl, and hexenyl.

The term "alkynyl" as used herein means an unsaturated straight or branched chain aliphatic group with one or more carbon-carbon triple bonds, having from 2 to 12 carbon atoms, preferably 2-8 carbon atoms, and more preferably 2-6 carbon atoms, which is optionally substituted with one, two or three substituents. Preferred alkynyl groups include, without limitation, ethynyl, propynyl, butynyl, pentynyl, and hexynyl.

An "alkylene," "alkenylene," or "alkynylene" group is an alkyl, alkenyl, or alkynyl group, as defined hereinabove, that is positioned between and serves to connect two other chemical groups. Preferred alkylene groups include, without limitation, methylene, ethylene, propylene, and butylene. Preferred alkenylene groups include, without limitation, ethenylene, propenylene, and butenylene. Preferred alkynylene groups include, without limitation, ethynylene, propynylene, and butynylene.

The term "cycloalkyl" as employed herein includes saturated and partially unsaturated cyclic hydrocarbon groups having 3 to 12 carbons, preferably 3 to 8 carbons, and more preferably 3 to 6 carbons, wherein the cycloalkyl group additionally is optionally substituted. Preferred cycloalkyl groups include, without limitation, cyclopropyl, cyclobutyl, cyclopentyl, cyclopentenyl, cyclohexyl, cyclohexenyl, cycloheptyl, and cyclooctyl.

The term "heteroalkyl" refers to an alkyl group, as defined hereinabove, wherein one or more carbon atoms in the chain are replaced by a heteroatom selected from the group consisting of 0, S, and N.

An "aryl" group is a C₆-C₁₄ aromatic moiety comprising one to three aromatic rings, which is optionally substituted. Preferably, the aryl group is a C₆-C₁₀ aryl group. Preferred aryl groups include, without limitation, phenyl, naphthyl, anthracenyl, and fluorenyl. An "aralkyl" or "arylalkyl" group comprises an aryl group covalently linked to an alkyl group, either of which may independently be optionally substituted or unsubstituted. Preferably, the aralkyl group is (C₁-C₆)alk(C₆-C₁₀)aryl, including, without limitation, benzyl, phenethyl, and naphthylmethyl.

A "heterocyclic" group (or "heterocyclyl) is an optionally substituted non-aromatic mono-, bi-, or tricyclic structure having from about 3 to about 14 atoms, wherein one or more atoms are selected from the group consisting of N, 0, and S. One ring of a bicyclic heterocycle or two rings of a tricyclic heterocycle may be aromatic, as in indan and 9,10-dihydro anthracene. The heterocyclic group is optionally substituted on carbon with oxo or with one of the substituents listed above. The heterocyclic group may also independently be substituted on nitrogen with alkyl, aryl, aralkyl, alkylcarbonyl, alkylsulfonyl, arylcarbonyl, arylsulfonyl, alkoxycarbonyl, aralkoxycarbonyl, or on sulfur with oxo or lower alkyl. Preferred heterocyclic groups include, without limitation, epoxy, aziridinyl, tetrahydrofuranyl, pyrrolidinyl, piperidinyl, piperazinyl, thiazolidinyl, oxazolidinyl, oxazolidinonyl, and morpholino. In certain preferred embodiments, the heterocyclic group is fused to an aryl, heteroaryl, or cycloalkyl group. Examples of such fused heterocycles include, without limitation, tetrahydroquinoline and dihydrobenzofuran. Specifically excluded from the scope of this term are compounds where an annular 0 or S atom is adjacent to another 0 or S atom.

As used herein, the term "heteroaryl" refers to optionally substituted groups having 5 to 14 ring atoms, preferably 5, 6, 9, or 10 ring atoms; having 6, 10, or 14 pi electrons shared in a cyclic array; and having, in addition to carbon atoms, between one or more heteroatoms selected from the group consisting of N, 0, and S. For example, a heteroaryl group may be pyrimidinyl, pyridinyl, benzimidazolyl, thienyl, benzothiazolyl, benzofuranyl and indolinyl. Preferred heteroaryl groups include, without limitation, thienyl, benzothienyl, furyl, benzofuryl, dibenzofuryl, pyrrolyl, imidazolyl, pyrazolyl, pyridyl, pyrazinyl, pyrimidinyl, indolyl, quinolyl, isoquinolyl, quinoxalinyl, tetrazolyl, oxazolyl, thiazolyl, triazolyl, and isoxazolyl.

A "heteroaralkyl" or "heteroarylalkyl" group comprises a heteroaryl group covalently linked to an alkyl group, either of which is independently optionally substituted or unsubstituted. Preferred heteroalkyl groups comprise a C₁-C₆ alkyl group and a heteroaryl group having 5, 6, 9, or 10 ring atoms. Specifically excluded from the scope of this term are compounds having adjacent annular 0 and/or S atoms. Examples of preferred heteroaralkyl groups include pyridylmethyl, pyridylethyl, pyrrolylmethyl, pyrrolylethyl, imidazolylmethyl, imidazolylethyl, thiazolylmethyl, and thiazolylethyl.

An "arylene," "heteroarylene," or "heterocyclylene" group is an aryl, heteroaryl, or heterocyclyl group, as defined hereinabove, that is positioned between and serves to connect two other chemical groups.

Preferred heterocyclyls and heteroaryls include, but are not limited to, acridinyl, azocinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, benzimidazolinyl, carbazolyl, 4aH-carbazolyl, carbolinyl, chromanyl, chromenyl, cinnolinyl, decahydroquinolinyl, 2H,6H-1,5,2-dithiazinyl, dihydrofuro[2,3-b]tetrahydrofuran, furanyl, furazanyl, imidazolidinyl, imidazolinyl, imidazolyl, 1H-indazolyl, indolenyl, indolinyl, indolizinyl, indolyl, 3H-indolyl, isobenzofuranyl, isochromanyl, isoindazolyl, isoindolinyl, isoindolyl, isoquinolinyl, isothiazolyl, isoxazolyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, pyrimidinyl, phenanthridinyl, phenanthrolinyl, phenazinyl, phenothiazinyl, phenoxathiinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl.

As employed herein, when a moiety (*e.g.,* cycloalkyl, hydrocarbyl, aryl, heteroaryl, heterocyclic, urea, etc.) is described as "optionally substituted" it is meant that the group optionally has from one to four, preferably from one to three, more preferably one or two, non-hydrogen substituents. Suitable substituents include, without limitation, halo, hydroxy, oxo (*e.g.,* an annular -CH- substituted with oxo is -C(O)-) nitro, halohydrocarbyl, hydrocarbyl, aryl, aralkyl, alkoxy, haloalkoxy, aryloxy, heteroaryloxy, amino, acylamino, alkylcarbamoyl, arylcarbamoyl, aminoalkyl, acyl, carboxy, hydroxyalkyl, alkanesulfonyl, arenesulfonyl, sulfonamido, alkanesulfonamido, arenesulfonamido, aralkylsulfonamido, alkylcarbonyl, acyloxy, cyano, alkylthio, ureido, and ureidoalkyl groups. Preferred substituents, which are themselves not further substituted (unless expressly stated otherwise) are:
(a) halo, cyano, oxo, alkyl, alkoxy, alkylthio, haloalkoxy, aminoalkyl, aminoalkoxy, carboxy, formyl, nitro, amino, amidino, carbamoyl, guanidino, C₃-C₇ heterocycle, heterocyclylalkyl, heterocyclylcarbonyl, hydroxyalkyl, alkoxyalkyl,
(b) C₁-C₅ alkyl or alkenyl or arylalkyl imino, carbamoyl, carbamate, azido, carboxamido, mercapto, hydroxy, hydroxyalkyl, alkylaryl, arylalkyl, C₁-C₈ alkyl, C₁-C₈ alkenyl, C₁-C₈ alkoxy, C₁-C₈ alkoxycarbonyl, aryloxycarbonyl, C₂-C₈ acyl, C₂-C₈ acylamino, C₁-C₈ alkylthio, arylalkylthio, arylthio, heteroarylthio, C₁-C₈ alkylsulfinyl, arylalkylsulfinyl, arylsulfinyl, C₁-C₈ alkylsulfonyl, arylalkylsulfonyl, arylsulfonyl, C₀-C₆ *N*-alkyl carbamoyl, C₂-C₁₅ *N,N*-dialkylcarbamoyl, C₃-C₇ cycloalkyl, aroyl, aryloxy, heteroaryloxy, arylalkyl ether, C₃-C₇ heterocyclylalkylether, aryl, aryl fused to a cycloalkyl or heterocycle or another aryl ring, C₃-C₇ heterocycle, heteroaryl, arylcarbamoyl, or any of these rings fused or spiro-fused to a cycloalkyl, heterocyclyl, or aryl, wherein any of the foregoing which are additionally substitutable is further optionally substituted with one more moieties listed in (a), above; and
(c) -(CH₂)₅-NR³⁰R³¹, wherein s is from 0 (in which case the nitrogen is directly bonded to the moiety that is substituted) to 6, and R³⁰ and R³¹ are each independently hydrogen, cyano, oxo, carboxamido, amidino, C₁-C₈ hydroxyalkyl, C₁-C₃ alkylaryl, aryl-C₁-C₃ alkyl, C₁-C₈ alkyl, C₁-C₈ alkenyl, C₁-C₈ alkoxy, C₁-C₈ alkoxycarbonyl, aryloxycarbonyl, aryl-C₁-C₃ alkoxycarbonyl, C₂-C₈ acyl, C₁-C₈ alkylsulfonyl, arylalkylsulfonyl, arylsulfonyl, aroyl, aryl, cycloalkyl, heterocyclyl, or heteroaryl, wherein each of the foregoing is further optionally substituted with one more moieties listed in (a), above; or
R³⁰ and R³¹ taken together with the N to which they are attached form a heterocyclyl or heteroaryl, each of which is optionally substituted with from 1 to 3 substituents from (a), above.

In addition, substituents on cyclic moieties (i.e., cycloalkyl, heterocyclyl, aryl, heteroaryl) include 5-6 membered mono- and 9-14 membered bi-cyclic moieties fused to the parent cyclic moiety to form a bi- or tri-cyclic fused ring system. For example, an optionally substituted phenyl includes, but not limited to, the following:

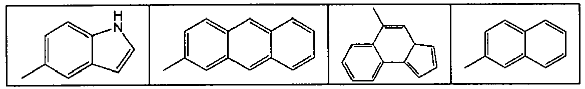

Preferred substituents on cyclic moieties (i.e., cycloalkyl, heterocyclyl, aryl, heteroaryl) also include groups of the formula -K¹-N(H)(R¹⁰), wherein
K¹ is a chemical bond or C₁-C₄ alkylene;
R¹⁰ is selected from the group consisting of Z' and -Ak²-Z', wherein
Ak² is C₁-C₄ alkylene; and
Z' is cycloalkyl, aryl, heteroaryl, or heterocyclyl, each of which optionally is substituted, and each of which optionally is fused to one or more aryl or heteroaryl rings, or to one or more saturated or partially unsaturated cycloalkyl or heterocyclic rings.

Particularly preferred substituents on cyclic moieties (such as aryl, heteroaryl, cycloalkyl, heterocyclyl, or any of these rings fused to one or more aryl or heteroaryl rings, or to one or more saturated or partially unsaturated cycloalkyl or heterocyclic rings), include 1, 2, or 3 groups independently selected from the following:
a) alkoxy, cyano, amino, oxo, haloalkyl, halo, alkyl, R₅₀-C(O)-N(R₃₂)-, R₅₀-O-C(O)-N(R₃₂)-, R₅₀-NH-C(O)-N(R₃₂)-, R₅₀-NH-C(O)-O-, (R₃₂)(R₃₃)N-alkyl-, (R₃₂)(R₃₃)N-alkyl-O-, (R₃₂)(R₃₃)N-alkenylene-N(R₃₂)-, N(R₃₂)-aryl-N(R₃₂)-C(O)-aryl-alkyl-N(R₃₂)-;
   wherein R₅₀ is cycloakyl, heterocyclyl-C₁-C₆ alkyl-, R₃₂R₃₃N-alkyl-, or alkyl;
b) aryl-C₀-C₆ alkyl-, heteroaryl-C₀-C₆ alkyl-, cycloalkyl-C₀-C₆ alkyl-, heterocyclyl-C₀-C₆ alkyl-, aryl-C₀-C₆ alkyl-N(R₃₂)-, aryl-C(O)-, heteroaryl-C₀-C₆ alkyl-N(R₃₂)-, heterocyclyl-C₀-C₆ alkyl-N(R₃₂)-, aryl-O-, heteroaryl-O-, aryl-S-, heteroaryl-S-, aryl-SO₂-, heteroaryl-SO₂, aryl-C(O)N(R₃₂)-, heteroaryl-C(O)N(R₃₂)-, heteroaryl-C(H)(SO₂-heteroaryl)-N(R₃₂)-;
wherein R₃₂ and R₃₃ are independently H or C₁-C₆ alkyl, or R₃₂ and R₃₃ taken together with the N to which they are attached form a heterocyclyl or heteroaryl;
and wherein any of the rings described in paragraphs [0045]-[0048] are further optionally substituted with 1, 2, or 3 groups independently selected from alkyl, alkoxy, thioalkoxy, alkyl-SO₂-, amino, halo, cyano, haloalkyl, hydroxyalkyl, alkoxyalkoxyalkyl, COOH, alkanoyl, alkanoate, NO₂, hydroxy, haloalkoxy, (R₃₂)(R₃₃)N-C₀-C₆ alkyl-, (R₃₂)(R₃₃)N-C₀-C₆ alkyl-O-, (R₃₂)(R₃₃)N-C(O)-, heteroaryl, alkyl-C(O)N(R₃₂)-, aryl-O-, (R₃₂)(R₃₃)N-SO₂-, aryl, and (R₃₂)(R₃₃)N-alkyl-C(O)N(R₃₂)-.

A "halohydrocarbyl" is a hydrocarbyl moiety in which from one to all hydrogens have been replaced with one or more halo.

The term "halogen" or "halo" as employed herein refers to chlorine, bromine, fluorine, or iodine. As herein employed, the term "acyl" refers to an alkylcarbonyl or arylcarbonyl substituent. The term "acylamino" refers to an amide group attached at the nitrogen atom (*i.e*., R-CO-NH-). The term "carbamoyl" refers to an amide group attached at the carbonyl carbon atom *(i.e.,* NH₂-CO-). The nitrogen atom of an acylamino or carbamoyl substituent is additionally optionally substituted. The term "sulfonamido" refers to a sulfonamide substituent attached by either the sulfur or the nitrogen atom. The term "amino" is meant to include NH₂, alkylamino, arylamino, and cyclic amino groups. The term "ureido" as employed herein refers to a substituted or unsubstituted urea moiety.

The term "radical" as used herein means a chemical moiety comprising one or more unpaired electrons.

A moiety that is substituted is one in which one or more hydrogens have been independently replaced with another chemical substituent. As a non-limiting example, substituted phenyls include 2-flurophenyl, 3,4-dichlorophenyl, 3-chloro-4-fluoro-phenyl, 2-fluoro-3-propylphenyl. As another non-limiting example, substituted N-octyls include 2,4 dimethyl-5-ethyl-octyl and 3-cyclopentyl-octyl. Included within this definition are methylenes (-CH₂-) substituted with oxygen to form carbonyl -CO-).

An "unsubstituted" moiety as defined above (*e.g*., unsubstituted cycloalkyl, unsubstituted heteroaryl, etc.) means that moiety as defined above that does not have any of the optional substituents for which the definition of the moiety (above) otherwise provides. Thus, for example, while an "aryl" includes phenyl and phenyl substituted with a halo, "unsubstituted aryl" does not include phenyl substituted with a halo.

Throughout the specification preferred embodiments of one or more chemical substituents are identified. Also preferred are combinations of preferred embodiments.

Some compounds of the invention may have chiral centers and/or geometric isomeric centers (E- and Z- isomers), and it is to be understood that the invention encompasses all such optical, diastereoisomers and geometric isomers. The invention also comprises all tautomeric forms of the compounds disclosed herein. Where compounds of the invention include chiral centers, the invention encompasses the enantiomerically pure isomers of such compounds, the enantiomerically enriched mixtures of such compounds, and the racemic mixtures of such compounds.

The compounds of the invention may be administered in the form of an *in vivo* hydrolyzable ester or in *vivo* hydrolyzable amide. An in vivo hydrolyzable ester of a compound of the invention containing carboxy or hydroxy group is, for example, a pharmaceutically acceptable ester which is hydrolyzed in the human or animal body to produce the parent acid or alcohol. Suitable pharmaceutically acceptable esters for carboxy include C₁-₆-alkoxymethyl esters (*e.g.*, methoxymethyl), C₁₋₆-alkanoyloxymethyl esters (*e.g.,* for example pivaloyloxymethyl), phthalidyl esters, C₃₋₈-cycloalkoxycarbonyloxyC₁₋₆-alkyl esters (*e.g.,* 1-cyclohexylcarbonyloxyethyl); 1,3-dioxolen-2-onylmethyl esters (*e.g*., 5-methyl-1,3-dioxalen-2-onylmethyl; and C₁₋₆-alkoxycarbonyloxyethyl esters (*e.g.,* 1-methoxycarbonyloxyethyl) and may be formed at any carboxy group in the compounds of this invention.

An *in vivo* hydrolyzable ester of a compound of the invention containing a hydroxy group includes inorganic esters such as phosphate esters and a-acyloxyalkyl ethers and related compounds which as a result of the in vivo hydrolysis of the ester breakdown to give the parent hydroxy group, Examples of α-acyloxyalkyl ethers include acetoxymethoxy and 2,2-dimethylpropionyloxy-methoxy. A selection of in vivo hydrolyzable ester forming groups for hydroxy include alkanoyl, benzoyl, phenylacetyl and substituted benzoyl and phenylacetyl, alkoxycarbonyl (to give alkyl carbonate esters), dialkylcarbamoyl and *N*-(*N*,*N-*dialkylaminoethy))-*N-*alkylcarbamoyl (to give carbamates), *N,N*-dialkylaminoacetyl and carboxyacetyl. Examples of substituents on benzoyl include morpholino and piperazino linked from a ring nitrogen atom via a methylene group to the 3- or 4- position of the benzoyl ring. A suitable value for an *in vivo* hydrolyzable amide of a compound of the invention containing a carboxy group is, for example, a. N-C₁₋₆-alkyl or N,N-di-C₁₋₆-alkyl amide such as *N*-methyl, *N*-ethyl, *N*-propyl, *N,N*-dimethyl, *N*-ethyl-*N-*methyl or *N,N*-diethyl amide.

### Compounds

In a first aspect, the invention provides novel inhibitors of histone deacetylase. In a first embodiment, the novel inhibitors of histone deacetylase are represented by formula (1f-1), 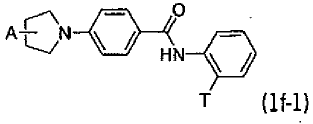 and pharmaceutical acceptable salts thereof, wherein T is OH or CH₂ and A is as defined below.

Preferred compounds according to paragraph [0152] include those wherein T is NH₂.

In a preferred embodiment, the novel histone deacetylase inhibitors of the invention are compounds of formula (2) 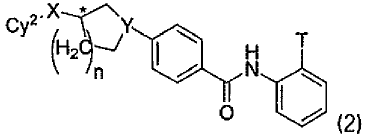 and pharmaceutically acceptable salts thereof, wherein
Cy² is aryl or heteroaryl, each of which is optionally substituted and wherein each of aryl, and heteroaryl is optionally fused to one or more aryl or heteroaryl rings, or to one or more saturated or partially unsaturated cycloalkyl or heterocyclic rings, each of which rings is optionally substituted;
X is selected from the group consisting of: a covalent bond, C₀-C₄-hydrocarbyl, C₀-C₄-hydrocarbyl-(CO)-C₀-C₄-hydrocarbyl, C₀-C₄-hydrocarbyl-(NR⁷)-C₀-C₄-hydrocarbyl, C₀-C₄-hydrocarbyl-(S)-C₀-C₄-hydrocarbyl, C₀-C₄-hydrocarbyl -(O)-C₀-C₄-hydrocarbyl, C₀-C₄-hydrocarbyl -(SO)-C₀-C₄-hydrocarbyl, C₀-C₄-hydrocarbyl -(SO₂)-C₀-C₄-hydrocarbyl, C₀-C₄-hydrocarbyl-(NH)-(CO)-C₀-C₄-hydrocarbyl, C₀-C₄-hydrocarbyl -(CO)-(NH)-C₀-C₄-hydrocarbyl, -NN-CO-NH-, -NH-CS-NH-, -O-CO-O-, -O-CS-O-, -NH-C(NH)-NH-, -S(O)₂-N(R⁷)-, -N(R⁷)-S(O)₂-, -NH-C(O)-O-, and -O-C(O)-NH-,
   wherein R⁷ is selected from the group consisting of hydrogen, C₁-C₅-alkyl, aryl, aralkyl, acyl, heterocyclyl, heteroaryl, SO₂-alkyl, SO₂-aryl, CO-alkyl, CO-aryl, CO-NH-alkyl, CO-NH-aryl, CO-O-alkyl and CO-O-aryl, each of which is optionally substituted,
n is 0 to 4,
Y is N or CH,
T is NH₂ or OH.

Compounds of formula (2) contain a chiral center (indicated by the asterisk (*)). The invention encompasses both racemic mixtures and enantiomerically enriched mixtures of compounds of formula (2), as well as the enantiomerically pure isomers of compounds of formula (2). Preferably in enantiomerically enriched mixtures there is greater or equal to 80% of one enantiomer, more preferably greater than 90%, 95%, or 98%.

Group A is independently selected from H, halogen, C₁-C₄ alkyl, optionally substituted alkoxy including aminoalkoxy, haloalkoxy and heteroarylalkoxy, alkoxyalkyl, haloalkyl, amino, nitro, alkylthio, acylamino, carbamoyl, or the following:

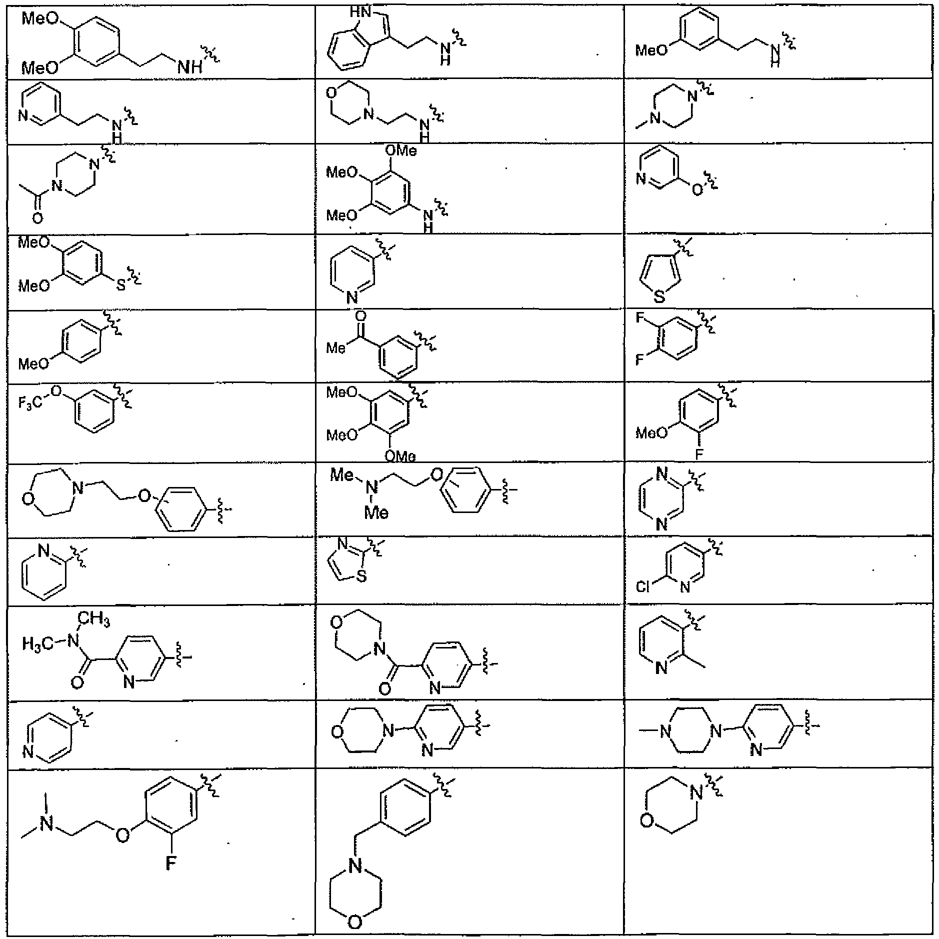

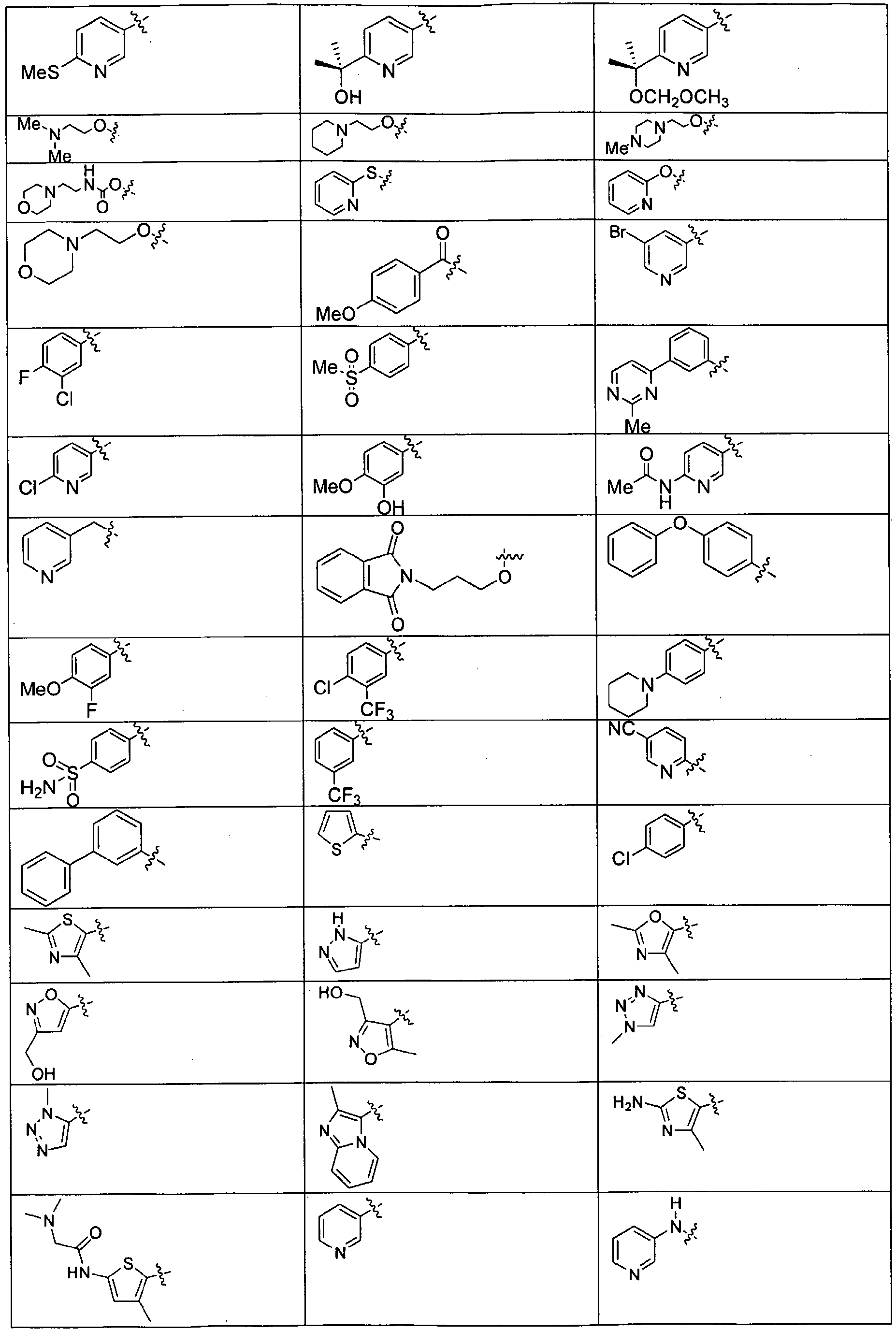

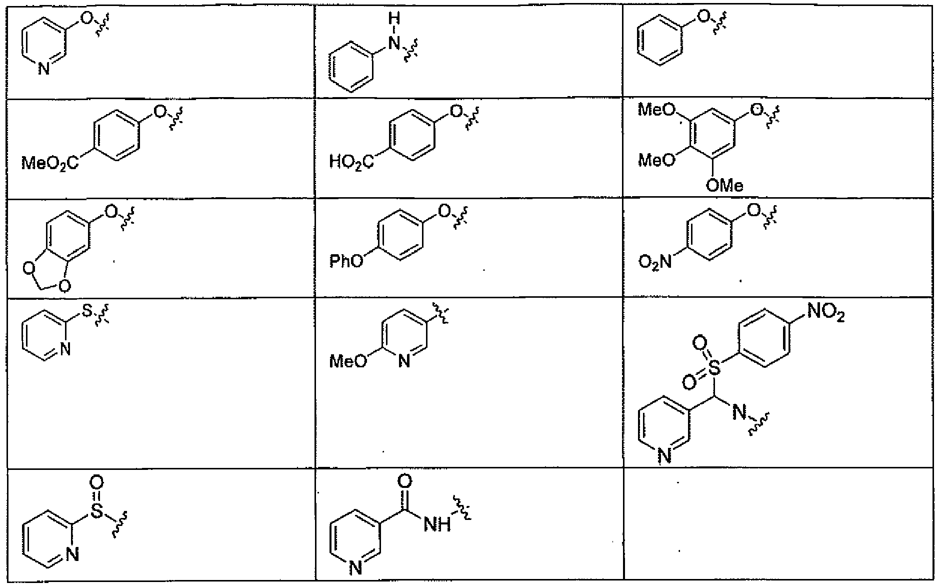

### Pharmaceutical Compositions

In a second aspect, the invention provides pharmaceutical compositions comprising an inhibitor of histone deacetylase according to the invention and a pharmaceutically acceptable carrier. Compounds of the invention may be formulated by any method well known in the art and may be prepared for administration by any route, including, without limitation, parenteral, oral, sublingual, transdermal, topical, intranasal, intratracheal, or intrarectal. In certain preferred embodiments, compounds of the invention are administered intravenously in a hospital setting. In certain other preferred embodiments, administration may preferably be by the oral route.

The characteristics of the carrier will depend on the route of administration. As used herein, the term "pharmaceutically acceptable" means a non-toxic material that is compatible with a biological system such as a cell, cell culture, tissue, or organism, and that does not interfere with the effectiveness of the biological activity of the active ingredient(s). Thus, compositions according to the invention may contain, in addition to the inhibitor, diluents, fillers, salts, buffers, stabilizers, solubilizers, and other materials well known in the art. The preparation of pharmaceutically acceptable formulations is described in, e.g., Remington's Pharmaceutical Sciences, 18th Edition, ed. A. Gennaro, Mack Publishing Co., Easton, PA, 1990.

As used herein, the term pharmaceutically acceptable salts refers to salts that retain the desired biological activity of the above-identified compounds and exhibit minimal or no undesired toxicological effects. Examples of such salts include, but are not limited to acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, pamoic acid, alginic acid, polyglutamic acid, naphthalenesulfonic acid, naphthalenedisulfonic acid, and polygalacturonic acid. The compounds can also be administered as pharmaceutically acceptable quaternary salts known by those skilled in the art, which specifically include the quaternary ammonium salt of the formula -NR + Z-, wherein R is hydrogen, alkyl, or benzyl, and Z is a counterion, including chloride, bromide, iodide, -O-alkyl, toluenesulfonate, methylsulfonate, sulfonate, phosphate, or carboxylate (such as benzoate, succinate, acetate, glycolate, maleate, malate, citrate, tartrate, ascorbate, benzoate, cinnamoate, mandeloate, benzyloate, and diphenylacetate).

The active compound is included in the pharmaceutical acceptable carrier or diluent in an amount sufficient to deliver to a patient a therapeutically effective amount without causing serious toxic effects in the patient treated. A preferred dose of the active compound for all of the above-mentioned conditions is in the range from about 0.01 to 300 mg/kg, preferably 0.1 to 100 mg/kg per day, more generally 0.5 to about 25 mg per kilogram body weight of the recipient per day. A typical topical dosage will range from 0.01-3% wt/wt in a suitable carrier. The effective dosage range of the pharmaceutically acceptable derivatives can be calculated based on the weight of the parent compound to be delivered. If the derivative exhibits activity in itself, the effective dosage can be estimated as above using the weight of the derivative, or by other means known to those skilled in the art.

### Inhibition of Histone Deacetylase

In a third aspect, the invention provides the use of an inhibitor of histone deacetylase according to the invention *in vitro* for treating a cell proliferative disease or condition in a cell in which inhibition of histone deacetylase is desired with an inhibitor of histone deacetylase according to the invention. Because compounds of the invention inhibit histone deacetylase, they are useful research tools for *in vitro* study of the role of histone deacetylase in biological processes. In addition, the compounds of the invention selectively inhibit certain isoforms of HDAC.

Measurement of the enzymatic activity of a histone deacetylase can be achieved using known methodologies. For example, Yoshida et al., J. Biol. Chem., 265: 17174-17179 (1990), describes the assessment of histone deacetylase enzymatic activity by the detection of acetylated histones in trichostatin A treated cells. Taunton et al., Science, 272: 408-411 (1996), similarly describes methods to measure histone deacetylase enzymatic activity using endogenous and recombinant HDAC-1.

In some preferred embodiments, the histone deacetylase inhibitor interacts with and reduces the activity of all histone deacetylases in the cell. In some other preferred embodiments according to this aspect of the invention, the histone deacetylase inhibitor interacts with and reduces the activity of fewer than all histone deacetylases in the cell. In certain preferred embodiments, the inhibitor interacts with and reduces the activity of one histone deacetylase (e.g., HDAC.-1) or a sub-group of histone deacetylases (e.g., HDAC-1, HDAC-2, and HDAC-3) to a greater extent than other histone deacetylases. Where the inhibitor preferentially reduces the activity of a sub-group of histone deacetylases, the reduction in activity of each member of the sub-group may be the same or different. As discussed below, certain particularly preferred histone deacetylase inhibitors are those that interact with, and reduce the enzymatic activity of, histone deacetylases that are involved in tumorigenesis. Certain other preferred histone deacetylase inhibitors interact with and reduce the enzymatic activity of fungal histone deacetylases.

Preferably, the use according to the third aspect of the invention causes an inhibition of cell proliferation of the contacted cells. The phrase "inhibiting cell proliferation" is used to denote an ability of an inhibitor of histone deacetylase to retard the growth of cells contacted with the inhibitor as compared to cells not contacted. An assessment of cell proliferation can be made by counting contacted and non-contacted cells using a Coulter Cell Counter (Coulter, Miami, FL) or a hemacytometer. Where the cells are in a solid growth (e.g., a solid tumor or organ), such an assessment of cell proliferation can be made by measuring the growth with calipers and comparing the size of the growth of contacted cells with non-contacted cells.

Preferably, growth of cells contacted with the inhibitor is retarded by at least 50% as compared to growth of non-contacted cells. More preferably, cell proliferation is inhibited by 100% (i.e., the contacted cells do not increase in number). Most preferably, the phrase "inhibiting cell proliferation" includes a reduction in the number or size of contacted cells, as compared to non-contacted cells. Thus, an inhibitor of histone deacetylase according to the invention that inhibits cell proliferation in a contacted cell may induce the contacted cell to undergo growth retardation, to undergo growth arrest, to undergo programmed cell death (i.e., to apoptose), or to undergo necrotic cell death.

The cell proliferation inhibiting ability of the histone deacetylase inhibitors according to the invention allows the synchronization of a population of asynchronously growing cells. For example, the histone deacetylase inhibitors of the invention may be used to arrest a population of non-neoplastic cells grown in vitro in the G1 or G2 phase of the cell cycle. Such synchronization allows, for example, the identification of gene and/or gene products expressed during the G1 or G2 phase of the cell cycle. Such synchronization of cultured cells may also be useful for testing the efficacy of a new transfection protocol, where transfection efficiency varies and is dependent upon the particular cell cycle phase of the cell to be transfected. Use of the histone deacetylase inhibitors of the invention allows the synchronization of a population of cells, thereby aiding detection of enhanced transfection efficiency.

In some preferred embodiments, the contacted cell is a neoplastic cell. The term "neoplastic cell" is used to denote a cell that shows aberrant cell growth. Preferably, the aberrant cell growth of a neoplastic cell is increased cell growth. A neoplastic cell may be a hyperplastic cell, a cell that shows a lack of contact inhibition of growth in vitro, a benign tumor cell that is incapable of metastasis in vivo, or a cancer cell that is capable of metastasis in vivo and that may recur after attempted removal. The term "tumorigenesis" is used to denote the induction of cell proliferation that leads to the development of a neoplastic growth. In some embodiments, the histone deacetylase inhibitor induces cell differentiation in the contacted cell. Thus, a neoplastic cell, when contacted with an inhibitor of histone deacetylase may be induced to differentiate, resulting in the production of a non-neoplastic daughter cell that is phylogenetically more advanced than the contacted cell.

The invention also provides a histone deacetylase inhibitor of the invention for use in treating a cell proliferative disease or condition in an animal,
comprising administering to an animal in need of such treatment a therapeutically effective amount of a histone deacetylase inhibitor of the invention. Preferably, the animal is a mammal, more preferably a domesticated mammal. Most preferably, the animal is a human.

The term "cell proliferative disease or condition" is meant to refer to any condition characterized by aberrant cell growth, preferably abnormally increased cellular proliferation. Examples of such cell proliferative diseases or conditions include, but are not limited to, cancer, restenosis, and psoriasis. In particularly preferred embodiments, the invention provides a histone deacetylase inhibitor of the invention
for use in inhibiting neoplastic cell proliferation in an animal comprising administering to an animal having at least one neoplastic cell present in its body a therapeutically effective amount of a histone deacetylase inhibitor of the invention.

It is contemplated that some compounds of the invention have inhibitory activity against a histone deacetylases from a protozoal source. Thus, the invention also provides a histone deacetylase inhibitor of the invention for use in treating or preventing a protozoal disease or infection, comprising administering to an animal in need of such treatment a therapeutically effective amount of a histone deacetylase inhibitor of the invention. Preferably the animal is a mammal, more preferably a human. Preferably, the histone deacetylase inhibitor used according to this embodiment of the invention inhibits a protozoal histone deacetylase to a greater extent than it inhibits mammalian histone deacetylases, particularly human histone deacetylases.

The present invention further provides a histone deacetylase inhibitor of the invention for use in treating a fungal disease or infection comprising administering to an animal in need of such treatment a therapeutically effective amount of a histone deacetylase inhibitor of the invention. Preferably the animal is a mammal, more preferably a human. Preferably, the histone deacetylase inhibitor used according to this embodiment of the invention inhibits a fungal histone deacetylase to a greater extent than it inhibits mammalian histone deacetylases, particularly human histone deacetylases.

The term "therapeutically effective amount" is meant to denote a dosage sufficient to cause inhibition of histone deacetylase activity in the cells of the subject, or a dosage sufficient to inhibit cell proliferation or to induce cell differentiation in the subject. Administration may be by any route, including, without limitation, parenteral, oral, sublingual, transdermal, topical, intranasal, intratracheal, or intrarectal. In certain particularly preferred embodiments, compounds of the invention are administered intravenously in a hospital setting. In certain other preferred embodiments, administration may preferably be by the oral route.

When administered systemically, the histone deacetylase inhibitor is preferably administered at a sufficient dosage to attain a blood level of the inhibitor from about 0.01 µM to about 100 µM, more preferably from about 0.05 µM to about 50 µM, still more preferably from about 0.1 µM to about 25 µM, and still yet more preferably from about 0.5 µM to about 25 µM. For localized administration, much lower concentrations than this may be effective, and much higher concentrations may be tolerated. One of skill in the art will appreciate that the dosage of histone deacetylase inhibitor necessary to produce a therapeutic effect may vary considerably depending on the tissue, organ, or the particular animal or patient to be treated.

In certain preferred embodiments of the third aspect of the invention, the *in vitro* use further comprises contacting the cell with an antisense oligonucleotide that inhibits the expression of a histone deacetylase, The combined use of a nucleic acid level inhibitor (e.g., antisense oligonucleotide) and a protein level inhibitor (i.e., inhibitor of histone deacetylase enzyme activity) results in an improved inhibitory effect, thereby reducing the amounts of the inhibitors required to obtain a given inhibitory effect as compared to the amounts necessary when either is used individually. The antisense oligonucleotides according to this aspect of the invention are complementary to regions of RNA or double-stranded DNA that encode HDAC-1, HDAC-2, HDAC-3, HDAC-4, HDAC-5, HDAC-6, HDAC7, and/or HDAC-8 (see e.g., GenBank Accession Number U50079 for HDAC-1, GenBank Accession Number U31814 for HDAC-2, and GenBank Accession Number U75697 for HDAC-3).

For purposes of the invention, the term "oligonucleotide" includes polymers of two or more deoxyribonucleosides, ribonucleosides, or 2'-substituted ribonucleoside residues, or any combination thereof. Preferably, such oligonucleotides have from about 6 to about 100 nucleoside residues, more preferably from about 8 to about 50 nucleoside residues, and most preferably from about 12 to about 30 nucleoside residues. The nucleoside residues may be coupled to each other by any of the numerous known internucleoside linkages. Such internucleoside linkages include without limitation phosphorothioate, phosphorodithioate, alkylphosphonate, alkylphosphonothioate, phosphotriester, phosphoramidate, siloxane, carbonate, carboxymethylester, acetamidate, carbamate, thioether, bridged phosphoramidate, bridged methylene phosphonate, bridged phosphorothioate and sulfone internucleoside linkages. In certain preferred embodiments, these internucleoside linkages may be phosphodiester, phosphotriester, phosphorothioate, or phosphoramidate linkages, or combinations thereof. The term oligonucleotide also encompasses such polymers having chemically modified bases or sugars and/ or having additional substituents, including without limitation lipophilic groups, intercalating agents, diamines and adamantane.

For purposes of the invention the term "2'-substituted ribonucleoside" includes ribonucleosides in which the hydroxyl group at the 2' position of the pentose moiety is substituted to produce a 2'-O-substituted ribonucleoside. Preferably, such substitution is with a lower alkyl group containing 1-6 saturated or unsaturated carbon atoms, or with an aryl or allyl group having 2-6 carbon atoms, wherein such alkyl, aryl or allyl group may be unsubstituted or may be substituted, e.g., with halo, hydroxy, trifluoromethyl, cyano, nitro, acyl, acyloxy, alkoxy, carboxyl, carbalkoxyl, or amino groups. The term "2'-substituted ribonucleoside" also includes ribonucleosides in which the 2'-hydroxyl group is replaced with an amino group or with a halo group, preferably fluoro.

Particularly preferred antisense oligonucleotides utilized in this aspect of the invention include chimeric oligonucleotides and hybrid oligonucleotides.

For purposes of the invention, a "chimeric oligonucleotide" refers to an oligonucleotide having more than one type of internucleoside linkage. One preferred example of such a chimeric oligonucleotide is a chimeric oligonucleotide comprising a phosphorothioate, phosphodiester or phosphorodithioate region, preferably comprising from about 2 to about 12 nucleotides, and an alkylphosphonate or alkylphosphonothioate region (see e.g., Pederson et al. U.S. Patent Nos. 5,635,377 and 5,366,878). Preferably, such chimeric oligonucleotides contain at least three consecutive internucleoside linkages selected from phosphodiester and phosphorothioate linkages, or combinations thereof.

For purposes of the invention, a "hybrid oligonucleotide" refers to an oligonucleotide having more than one type of nucleoside. One preferred example of such a hybrid oligonucleotide comprises a ribonucleotide or 2'-substituted ribonucleotide region, preferably comprising from about 2 to about 12 2'-substituted nucleotides, and a deoxyribonucleotide region. Preferably, such a hybrid oligonucleotide contains at least three consecutive deoxyribonucleosides and also contains ribonucleosides, 2'-substituted ribonucleosides, preferably 2'-O-substituted ribonucleosides, or combinations thereof (see e.g., Metelev and Agrawal, U.S. Patent No. 5,652,355).

The exact nucleotide sequence and chemical structure of an antisense oligonucleotide utilized in the invention can be varied, so long as the oligonucleotide retains its ability to inhibit expression of the gene of interest. This is readily determined by testing whether the particular antisense oligonucleotide is active. Useful assays for this purpose include quantitating the mRNA encoding a product of the gene, a Western blotting analysis assay for the product of the gene, an activity assay for an enzymatically active gene product, or a soft agar growth assay, or a reporter gene construct assay, or an in vivo tumor growth assay, all of which are described in detail in this specification or in Ramchandani et al. (1997) Proc. Natl. Acad. Sci. USA 94: 684-689.

Antisense oligonucleotides utilized in the invention may conveniently be synthesized on a suitable solid support using well known chemical approaches, including H-phosphonate chemistry, phosphoramidite chemistry, or a combination of H-phosphonate chemistry and phosphoramidite chemistry (i.e., H-phosphonate chemistry for some cycles and phosphoramidite chemistry for other cycles). Suitable solid supports include any of the standard solid supports used for solid phase oligonucleotide synthesis, such as controlled-pore glass (CPG) (see, e.g., Pon, R.T. (1993) Methods in Molec. Biol. 20: 465-496).

Particularly preferred oligonucleotides have nucleotide sequences of from about 13 to about 35 nucleotides which include the nucleotide sequences shown in Table A. Yet additional Particularly preferred oligonucleotides have nucleotide sequences of from about 15 to about 26 nucleotides of the nucleotide sequences shown In Table A.

**Table A**

| Oligo | Target | Accession Number | Nucleotide Position | Sequence | position within Gene |
|---|---|---|---|---|---|
| HDAC1 AS1 | Human HDAC1 | U50079 | 1585-1604 | 5'-GAAACGTGAGGGACTCAGCA-3' | 3'-UTR |
| HDAC1 AS2 | Human HDAC1 | U50079 | 1565-1584 | 5'-GGAAGCCAGAGCTGGAGAGG-3' | 3'-UTR |
| HDAC1 MM | Human HDAC1 | U50079 | 1585-1604 | 5'-GTTAGGTGAGGCACTGAGGA-3' | 3'-UTR |
| HDAC2 AS | Human HDAC2 | U31814 | 1643-1622 | 5'-GCTGAGCTGTTCTGATTTGG-3' | 3'-UTR |
| HDAC2 MM | Human HDAC2 | U31814 | 1643-1622 | 5'-CGTGAGCACTTCTCATTTCG-3' | 3'-UTR |
| HDAC3 AS | Human HDAC3 | AF039703 | 1276-1295 | 5'-CGCTTTCCTTGTCATTGACA-3' | 3'-UTR |
| HDAC3 MM | Human HDAC3 | AF039703 | 1276-1295 | 5'-GCCTTTCCTACTCATTGTGT-3' | 3'-UTR |
| HDAC4 AS1 | Numan HDAC4 | AB006626 | 514-33 | 5-GCTGCCTGCCGTGCCCACCC-3' | 5'-UTR |
| HDAC4 MM1 | Human HDAC4 | AB006626 | 514-33 | 5'-CGTGCCTGCGCTGCCCACGG-3' | 5'-UTR |
| HDAC4 AS2 | Human HDAC4 | AB006626 | 7710-29 | 5'-TACAGTCCATGCAACCTCCA-3' | 3'-UTR |
| HDAC4 MM4 | Human HDAC4 | AB006626 | 7710-29 | 5'-ATCAGTCCAACCAACCTCGT-3' | 3'-UTR |
| HDAC5 AS | Human HDAC5 | AF039691 | 2663-2682 | 5'-CTTCGGTCTCACCTGCTTGG-3' | 3'-UTR |
| HDAC6 AS | Human HDAC6 | AJ011972 | 3791-3810 | 5'-CAGGCTGGAATGAGCTACAG-3' | 3'-UTR |
| HDAC6 MM | Human HDAC6 | AJ011972 | 3791-3810 | 5'-GACGCTGCAATCAGGTAGAC-3' | 3'-UTR |
| HDAC7 AS | Human HDAC7 | AF239243 | 2896-2915 | 5'-CTTCAGCCAGGATGCCCACA-3' | 3'-UTR |
| HDAC8 AS1 | Human HDAC8 | AF230097 | 51-70 | 5'-CTCCGGCTCCTCCATCTTCC-3' | 5'-UTR |
| HDAC8 AS2 | Human HDAC8 | AF230097 | 1328-1347 | 5'-AGCCAGCTGCCACTTGATGC-3' | 3'-UTR |

The following examples are intended to further illustrate certain preferred embodiments of the invention, and are not intended to limit the scope of the invention.

### EXAMPLES

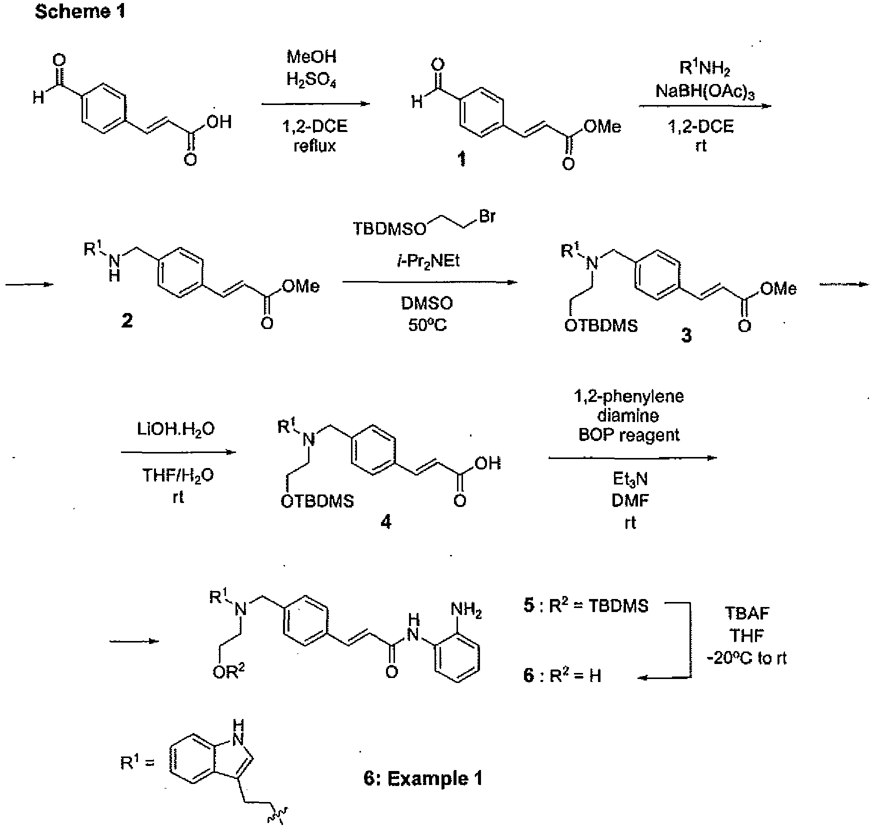

### ExampLe 1: (not in accordance with invention)

### N-(2-Amino-phenyl)-3-[4-({(2-hydroxy-ethyl)-[2-(1H-indol-3-yl)-ethyl]-amino}-methyl)-phenyl]-acrylamide (6)

### Step 1: Methyl 3-(4-formyl-phenyl)-acrylate (1)

To a stirred suspension at room temperature of 4-formylcinnamic acid (15,39 g, 87.36 mmol) in 1,2-dichloroethane (100 mL) was added concentrated sulfuric acid (8 mL) and anhydrous MeOH (15 mL), respectively. The reaction mixture was refluxed for 18 h, cooled to the room temperature and concentrated. The residue was diluted with AcOEt and washed with H₂O, saturated aqueous NaHCO₃, H₂O and brine, dried over MgSO₄, filtered, and concentrated again. The crude product was purified by flash chromatography on silica gel (eluent AcOEt/hexane: 20/80→30/70) to afford the title compound **2** (9.75 g, 51.26 mmol, 59% yield) as a pale yellow powder. ¹H NMR (300 MHz, CDCl₃) δ(ppm): 10.04 (s, 1H), 7.91 (d, J = 7.9 Hz, 2H), 7.80-7.60 (m, 3H), 6.56 (d, J = 15.8 Hz, 1H), 3.84 (s, 3H).

### Step 2: Methyl 3-(4-{[2-(1H-indol-3-yl)-ethylamino]-methyl}-phenyl)-acrylate (2)

To a stirred solution of **1** (3.00 g, 15.77 mmol) and tryptamine (2.78 g, 17.35 mmol) in anhydrous 1,2-dichloroethane (200 mL) under nitrogen was added NaBH(OAc)₃ (3.87 g, 17.35 mmol) at room temperature. The reaction mixture was stirred at room temperature for 39 hours, poured into 10% solution of K₂CO₃ and extracted with CH₂Cl₂. The organic layer was concentrated to form a residue which was purified by flash chromatography on silica gel (MeOH/CH₂Cl₂, 10/90) and co-precipitated in a mixture of AcOEt/hexane to afford the title compound **2** (4.39 g, 13.13 mmol, 83% yield) as a yellow solid. ¹H NMR (400 MHz, DMSO-*d*₆) (δppm) : 10.78 (s, 1H), 7.70-7.62 (m, 3H), 7.49 (d, J = 8.0 Hz, 1H), 7.39 (d, J = 8.2 Hz, 2H), 7.33 (dt, J = 8.0, 0.9 Hz, 1H), 7.13 (d, J = 2.2 Hz, 1H), 7.06 (ddd, J = 7.0, 7.0, 1.2 Hz, 1H), 6.96 (ddd, J = 6.9, 6.9, 1.1 Hz, 1H), 6.62 (d, J = 16.0 Hz, 1H), 3.79 (s, 2H), 3.75 (s, 3H), 2.91-2.78 (m, 4H), 2.18 (bs,1H).

### Step 3: Methyl 3-[4-({[2-(tert-butyl-dimethyl-silanyloxy)-ethyl]-[2-(1H-indol-3-yl)-ethyl]-amino}-methyl)-phenyl]-acrylate (3)

To a stirred solution of **2** (2.82 g, 8.44 mmol) and diisopropylethylamine (2.21 mL, 12.66 mmol) in anhydrous DMSO (22 mL) at room temperature under nitrogen was added (2-bromo-ethoxy)-*tert-*butyl-dimethylsilane (2.17 mL, 10.12 mmol). The reaction mixture was heated at 50-55□C for 24 h, poured into water and extracted with CH₂Cl₂. The organic layer was dried over MgSO₄, filtered, and concentrated. The crude product was purified by flash chromatography on silica gel (AcOEt/CH₂Cl₂, 15/85, plus a few drops of NH₄OH) to afford the title compound **3** (4.06 g, 8.24 mmol, 97% yield) as a dark orange oil. ¹H NMR (300 MHz, CDCl₃) δ(ppm): 7.95 (bs, 1H), 7.70 (d, J = 15.8 Hz, 1H), 7.58-7.30 (m, 6H), 7.18 (t, J = 7.5 Hz, 1H), 7.07 (t, J = 7.5 Hz, 1H), 7.00 (bs, 1H), 6.43 (d, J = 16.2 Hz, 1H), 3.88-3.68 (m, 7H), 3.04-2.66 (m, 6H), 0.88 (bs,9H), 0.04 (bs, 6H).

### Step 4: 3-[4-({[2-(tert-Butyl-dimethyl-silanyloxy)-ethyll]-[2-(1H-indol-3-yl)-ethyl]-amino}-methyl)-phenyl]-acrylic acid (4)

To a stirred solution of compound **3** (3.18 g, 6.45 mmol) in THF (40 mL) was added a solution of LiOH.H₂O (677 mg, 16.14 mmol) in water (20 mL) at room temperature. After 24 h the reaction mixture was concentrated, diluted with water and acidified with 1N HCl until a pH 5-6. A precipitate was formed which was separated by filtration, rinsed with water and dried to afford the title compound **4** (2.43 g, 5.08 mmol, 79% yield) as an off-white solid. ¹H NMR (400 MHz, DMSO-*d*₆) δ(ppm): 12.34 (bs, 1H), 10.75 (s, 1H), 7.63 (d, J = 8.2 Hz, 2H), 7.59 (d, J = 15.8 Hz, 1H), 7.44-7.35 (m, 3H), 7.32 (d, J = 8.0 Hz, 1H), 7.11 (d, J = 2.3 Hz, 1H), 7.05 (td, J = 7.5, 1.0 Hz, 1H), 6.92 (td, J = 7.4, 0.9 Hz, 1H), 6.51 (d, J = 15.8 Hz, 1H), 3.79 (s, 2H), 3.69 (t, J = 6.4 Hz, 2H), 2.93-2.74 (m, 4H), 2.69 (t, J = 6.2 Hz, 2H), 0.88 (s, 9H), 0.05 (s, 6H).

### Step 5: N-(2-Amino-phenyl)-3-[4-({[2-(tert-butyl-dimethyl-silanyloxy)-ethyl]-[2-(1H-indol-3-yl)-ethyl]-amino}-methyl)-phenyl]-acrylamide (5)

To a stirred solution of **4** (1.30 g, 2.72 mmol) in anhydrous DMF (20 mL) at room temperature under nitrogen were added Et₃N (330 µl, 3.26 mmol) and BOP reagent (1.32 g, 2.99 mmol), respectively. After 30 min, a solution of 1,2-phenylenediamine (352 mg, 3.26 mmol), Et₃N (1.14 mL, 8.15 mmol) in anhydrous DMF (3 mL) was added dropwise. After 3 h the reaction mixture was poured into saturated aqueous solution of NH₄Cl, and extracted with AcOEt. The extract was washed with saturated NH₄Cl, water and brine, dried over MgSO₄, filtered, concentrated and purified by flash chromatography on silica gel (MeOH/CH₂Cl₂, 5/95 plus several drops of NH₄OH), to afford the title compound **5** (1.49 g, 2.62 mmol, 96% yield) as a yellow sticky foam. ¹H NMR (300 MHz, DMSO-*d*₆) δ(ppm) : 10.78 (s, 1H), 9.40 (s, 1H), 7.59 (d, J = 8.0 Hz, 2H), 7.58 (d, J = 15.8 Hz, 1H), 7.45 (d, J = 7.9 Hz, 2H), 7.40 (t, J = 7.7 Hz, 2H), 7.35 (d, J = 8.4 Hz, 1H), 7.14 (s, 1H), 7.07 (t, J = 7.5 Hz, 1H), 7.05-6.85 (m, 3H), 6.79 (d, J = 7.9 Hz, 1H), 6.62 (t, J = 7.5 Hz, 1H), 4.98 (bs, 2H), 3.80 (s, 2H), 3.71 (t, J = 6.2 Hz, 2H), 2.95-2.75 (m, 4H), 2.71 (t, J = 6.2 Hz, 2H), 0.89 (s, 9H), 0.05 (s, 6H).

### Step 6: N-(2-Amino-phenyl)-3-[4-({(2-hydroxy-ethyl)-[2-(1H-indol-3-yl)-ethyl]-amino}-methyl)-phenyl]-acrylamide (6)

To a stirred solution at -20□C of **5** (1.49 g, 2.62 mmol) in anhydrous THF (30 mL) under nitrogen was slowly added a solution of TBAF (2.88 mL, 2.88 mmol, 1.OM in THF). The reaction mixture was allowed to warm-up to the room temperature over 1 h and was stirred for additional 22 hours. MeOH was added and the reaction mixture was concentrated, diluted with AcOEt, and successively washed with saturated aqueous solution of NaHCO₃, H₂O, a saturated aqueous solution of NH₄Cl and brine, dried over MgSO₄, filtered, and concentrated. The residue was purified by flash chromatography on silica gel (MeOH/CH₂Cl₂, 5/95→10/90 plus several drops of NH₄OH) and triturated with a mixture of AcOEt/CH₂Cl₂/hexane to afford the title compound **6** (956 mg, 2.10 mmol, 80% yield) as a pale yellow solid. ¹H NMR (400 MHz, DMSO-*d*₆) δ(ppm): 10.76 (s, 1H), 9.39 (s, 1H), AB system (δ_{A} = 7.58, δ_{B} = 7.44, J_{AB} = 8.0 Hz, 4H), 7.56 (d, J = 15.7 Hz, 1H), 7.42-7.34 (m, 2H), 7.33 (d, J = 8.0 Hz, 1H), 7.12 (d, J = 2.3 Hz, 1H), 7.05 (td, J = 7.2, 1.2 Hz, 1H), 6.98-6.90 (m, 2H), 6.90 (d, J = 15.8 Hz, 1H), 6.77 (dd, J = 8.0,1.4 Hz, 1H), 6.60 (ddd, J = 7.5. 7.5, 1.4 Hz, 1H), 4.98 (bs, 2H), 4.43 (t, J = 5.4 Hz, 1H), 3.78 (s, 2H), 3.56 (td, J = 6.3, 5.6 Hz, 2H), 2.94-2.84 (m, 2H), 2.82-2.74 (m, 2H), 2.68 (t, J = 6.5 Hz, 2H).

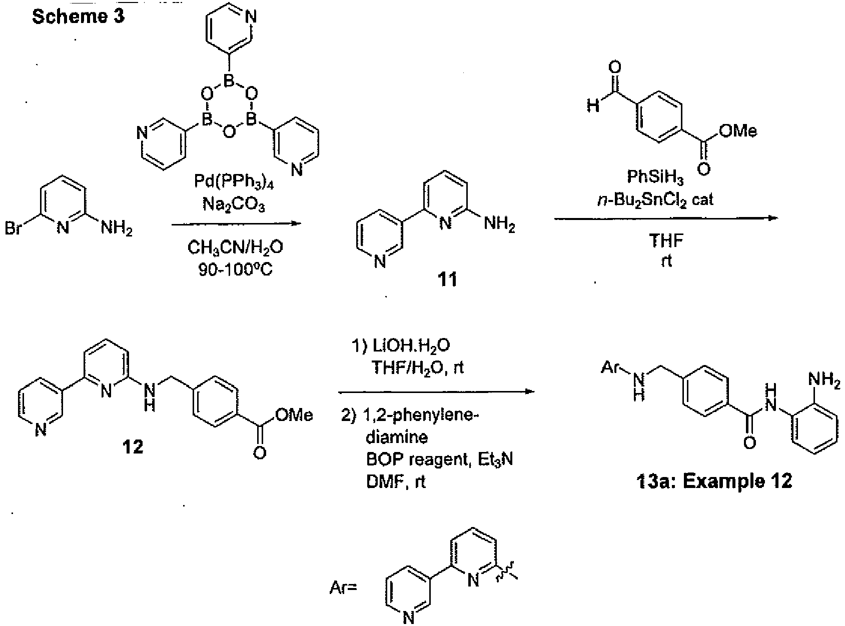

### Example 12: (not in accordance with invention)

### N-(2-Amino-phenyl)-4-([2,3']bipyridinyl-6-ylaminomethyl)-benzamide (13a)

### Step 1: [2,3']Bipyridinyl-6-ylamine (11)

To a stirred degassed suspension of a mixture of 2-amino-6-bromopyridine (5.38 g, 31.09 mmol), 2,4,6-(3-pyridinyl)-cyclotriboroxane (3.80 g, 12.07 mmol) and aqueous Na₂CO₃ (100 mL, 0,4M) in acetonitrile (100 mL) at room temperature Pd(PPh₃)₄ (1.70 g, 1.47 mmol) was added. The reaction mixture was heated at 95□for 1 to 2 days under nitrogen, cooled to the room temperature and filtered. The filtrate was concentrated purified by flash chromatography on silica gel (MeOH/CH₂Cl₂: 5/95→10/90 plus a few drops of NH₄OH) and co-precipitated with a mixture of AcOEt/CH₂Cl₂/hexane to afford the title compound **11** (4.091 g, 23.90 mmol, 77% yield) as a pale yellow solid. ¹H NMR (400 MHz, DMSO-*d*₆) δ(ppm): 9.16 (dd, J = 2.2, 0.8 Hz, 1H), 8.57 (dd, J = 4.7, 1.6 Hz 1H), 8.33-8.28 (m, 1H), 7.54-7.44 (m, 2H), 7.14 (dd, J = 7.3, 0.5 Hz, 1H), 6.49 (dd, J = 8.2, 0.4 Hz 1H), 6.12 (bs, 2H).

### Step 2: Methyl 4-([2,3']bipyridinyl-6-ylaminomethyl)-benzoate (12)

To a stirred suspension of a mixture of **11** (3.00 g, 17.52 mmol), methyl 4-formylbenzoate (4.62 g, 28.11 mmol, 1.5-2.0 equiv.) and dibutyl tin dichloride 160 mg, 0.53 mmol) in anhydrous THF (15 mL) at room temperature was added phenylsilane (2.34 mL, 19.28 mmol) in three portions over two days. After stirring for 2 to 7 days the reaction mixture was filtered, filtrate was concentrated and purified by flash chromatography on silica gel (MeOH/CH₂Cl₂, 2/98→10/90) to afford the title compound **12** (5.50 g, 17.22 mmol, 98% yield) as a pale yellow solid. ¹H NMR (400 MHz, DMSO-d₆) δ(ppm): 9.11 (dd, J = 2.3, 0.7 Hz, 1H), 8.55 (dd, J = 4.7, 1.8 Hz 1H), 8.29-8.24 (m, 1H), 7.93 (d, J = 8.4 Hz, 2H), 7.57-7.40 (m, 5H), 7.18 (d, J = 7.2 Hz, 1H), 6.59 (d, J = 8.2 Hz 1H), 4.69 (d, J = 6.1 Hz, 2H), 3.85 (s, 3H).

### Step 3: N-(2-Amino-phenyl)-4-([2.3']bypyridinyl-6-ylaminomethyl)-benzamide (13a)

The title compound **13a** (Example 12) was obtained from **12** as an off-white solid in two steps following the same procedure as in Example 2, steps 3 and 4 (Scheme 2). ¹H NMR (400 MHz, DMSO-*d*₆) δ(ppm): 9.60 (s, 1H), 9.16 (dd, J = 2.2, 0.9 Hz, 1H), 8.56 (dd, J = 4.8, 1.7 Hz 1H), 8.31 (ddd, J = 7.8, 2.3, 1.7 Hz, 1H), 7.95 (d, J = 8.2 Hz, 2H), 7.57-7.48 (m, 3H), 7.46 (ddd, J = 8.0, 4.7, 0.8 Hz, 1H), 7.42 (t, J = 6.1 Hz, 1H), 7.19 (dd, J = 7.2, 0.6 Hz, 1H), 7.17 (dd, J = 7.3, 1.0 Hz, 1H), 6.98 (td, J = 7.5, 1.4, 1H), 6.79 (dd, J = 7.8, 1.4 Hz 1H), 6.65-6.57 (m, 2H), 4.90 (bs, 2H), 4.69 (d, J = 6.1 Hz, 2H).

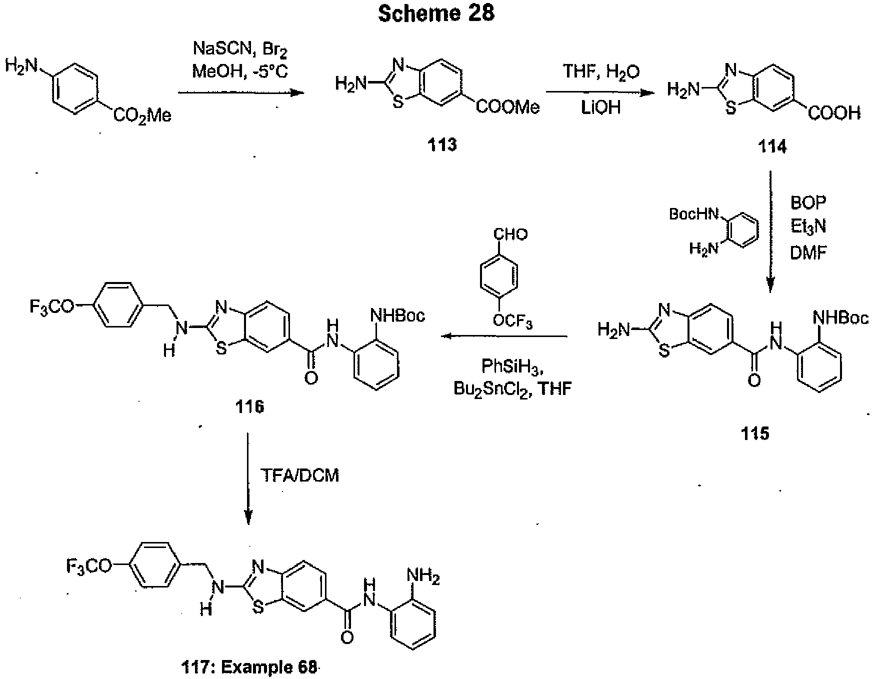

### Example 68 (not in accordance with invention)

### Step 5: 2-(4-Trifluoromethoxy-benzylamino)-benzothiazole-6-carboxylic acid 2-aminophenyl)-amide (117)

### Step 1: 2-Amino-benzothiazole-6-carboxylic acid methyl ester (113)

The title compound was obtained following the procedure described in J.Med. Chem. 1997; 40 (105-111), starting from 4-amino-benzoic acid methyl ester.

### Step 2 : 2-Amino-benzothiazole-6-carboxylic acid (114)

The title compound **114** was obtained following the procedure described in example 1 step 4 (97% yield). ¹H-NMR (DMSO) δ: 12.58 (s, 1H), 8.23 (d, J=1.8 Hz, 1H); 7.85 (s, 2H); 7.78 (dd, J= 8.4,1.8Hz, 1H); 7.33 (d, J=7.8 Hz, 1H).

### Step 3: {2-[(2-Amino-benzothiazole-6-carbonyl)-amino]-phenyl}-carbamic acid tert-butyl ester (115)

The acid **114** (1.80 g, 9.27 mmol) was combined with (2-amino-phenyl)-carbamic acid tert-butyl ester (2.31 g, 11.1 mmol) and BOP (4.91 g, 11.1 mmol) in DMF. To this solution Et₃N (5.16 ml, 37.1 mmol) was added and the mixture was stirred overnight at room temperature under nitrogen, concentrated in *vacuo* and purified by flash column chromatography (30% hexane/EtOAc). To further purify the product, the mixture was partitioned between EtOAc and water, organic layer was separated, dried over MgSO₄ and evaporated give the title compound **115** (1.84 g, 52%). ¹H-NMR (DMSO) δ: 9.72 (s, 1H), 8.66 (m, 1H); 8.22 (d, J=1.8 Hz, 1H); 7.80 (m, 3H); 7.50 (m, 2H); 7.37 (m, 1H); 7.14 (m, 2H); 1.44 (s, 9H).

### Step 4: 2-(4-Trifluoromethoxy-phenyl)-benzothiazole-6-carboxylic acid (2-amino-phenyl)-amide (116)

To a solution of **115** (300 mg, 0.78 mmol), 4-(trifluoromethoxy)benzaldehyde (123 µl, 0.86 mmol), and dibutyltin dichloride (24 mg, 0.08 mmol) in THF was added phenylsilane (106 µl, 0.86 mmol). The mixture was stirred overnight at room temperature under nitrogen, additional aldehyde and phenylsilane were added and the stirring continued until no more starting material was present. The THF was evaporated off the mixture and the residue was purified by flash column chromatography (EtOAc/hexane 30/70, then 50/50), to give the title compound **116** (314 mg, 72%). ¹H-NMR (DMSO) δ: 9.77 (s, 1H), 8.89 (t, J= 5.7 Hz, 1H); 8.69 (s, 1H); 8.29 (d, J= 1.8 Hz, 1H); 7.84 (dd, J= 8.4, 1.8 Hz, 1H); 7.50 (m, 5H); 7.37 (d, J=7.8 Hz, 2H); 7.17 (m, 2H); 4. 69 (d, J=5.7 Hz, 2H); 1.47 (s, 9H).

### Step 5: 2-(4-Trifluoromethoxy-benzylamino)-benzothiazole-6-carboxylic acid (2-amino-phenyl)-amide (117)

To a solution of **116** (306 mg, 0.55 mmol) in DCM was added TFA (2.0 ml). This mixture was stirred at room temperature for 4 hours and concentrated. The residue was dissolved in EtOAc, washed with NaHCO₃, dried over MgSO₄ and concentrated again. The residue was purified by flash column chromatography (30% hexane in EtOAc) to give the title compound 117 as a yellow solid (252 mg, 100%). ¹H-NMR (DMSO) δ: 9.56 (s, 1H), 8.83 (t, J=5.8 Hz, 1H), 8.30 (d, J=1.8 Hz, 1H); 7.85 (dd, J= 8.4, 1.6 Hz, 1H); 7.49 (d, J=8.4 Hz, 2H); 7.43 (d, J=8.4 Hz, 1H); 7.34 (d, J=8.4 Hz, 2H); 7.15 (d, J=7.6 Hz. 1H); 6.94 (brt, J= 7.8 Hz, 1H); 6.77 (d, J=7.8 Hz, 1H); 6.59 (t, J=7.5 Hz, 1H); 4.66 (d, J=5.7 Hz, 2H). LRMS: 458.1 (calc.), 459.2 (obt.)

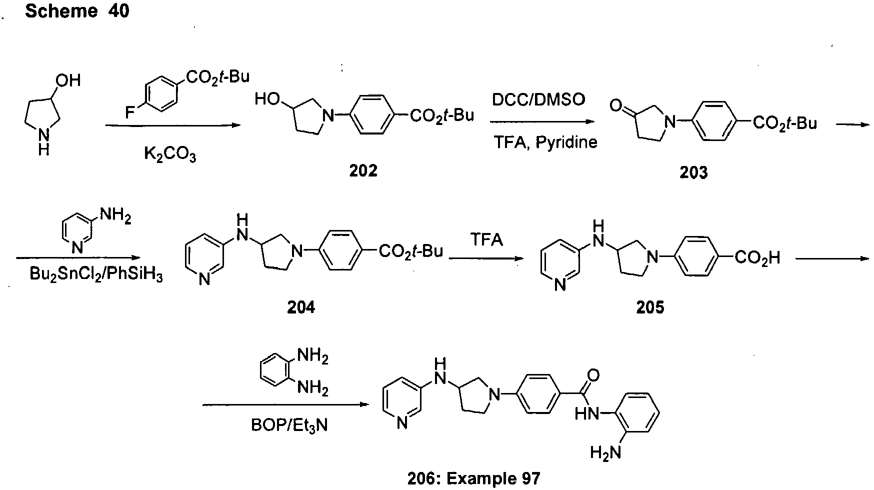

### Example 97

### N-(2-Amino-phenyl)-4-[3-(pyridin-3-ylamino)-pyrrolidin-1-yl]-benzamide (206)

### Step 1: 4-(3-Hydroxy-pyrrolidin-1-yl)-benzoic acid tert-butyl ester (202):

The title compound **202** was obtained following the procedure described in J. Heterocycl. Chem., 1994, 31, 1241, (91% yield). ¹H NMR: (CD₃OD) δ(ppm): 7.77 (d, J= 9.0 Hz, 2H), 6.54 (d, J= 9.0 Hz, 2H), 4.57-4.53 (m, 1H), 3.57-3.50 (m, 2H), 3.45 (td, J= 9.4, 3.3 Hz, 1H), 3.29 (dd, J= 12.7, 1.6 Hz, 1H), 2.22-2.13 (m, 1H), 2.10-2.03 (m, 1H), 1.59 (s, 9H). m/z: 264.4 (MH⁺).

### Step 2: 4-(3-Oxo-pyrrolidin-1-yl)-benzoic acid tert-butyl ester (203):

The title compound **203** was obtained following the procedure described in J. Heterocycl. Chem., 1994, 31, 1241 (73% yield). ¹H NMR: (DMSO-d₆) δ(ppm): 7.74 (d, J= 8.8 Hz, 2H), 6.67(d, J= 9.0 Hz, 2H), 3.75 (s, 2H), 3.69 (t, J= 7.4 Hz, 2H), 2.72 (t, J= 7.6 Hz, 2H), 1.52 (s, 9H). m/z: 262.4 (MH⁺).

### Step 3: 4-[3-(Pyridin-3-ylamino)-pyrrolidin-1-yl]-benzoic acid tert-butyl ester (204):

The title compound **204** was obtained following the procedure as for the reductive amination described in scheme 3, step 2 (example 12) starting from compound **203** and using 3-aminopyridine instead of 6-(pyridin-3-yl)pyridin-2-amine **(11)** (76% yield).¹H NMR: (acetone-d₆) δ (ppm): 8.08 (d, J= 2.7 Hz, 1H), 7.85(dd, J= 4.3, 1.6 Hz, 1H), 7.79 (d, J= 9.0 Hz, 2H), 7.09 (ddd, J= 8.2, 4.5, 0.8 Hz, 1H), 7.06 (ddd, J= 8.2, 2.7, 1.6 Hz, 1H), 6.58 (d, J= 9.0 Hz, 2H), 4.33 (quint, J= 4.9 Hz, 1H), 3.79 (dd, J= 10.2, 6.1 Hz, 1H), 3.60-3.54 (m, 1H), 3.51-3.45 (m, 1H), 3.32 (dd, J= 7.2, 4.1 Hz, 1H), 2.44 (sext., J= 7,6 Hz, 1H), 2.18-2.11 (m, 1H), 1.56 (s, 9H). m/z: 340.4 (MH⁺).

### Step 4: 4-[34-(Pyridin-3-ylamino)-pyrrolidin-1-yl]-benzoic acid 205):

The title compound **205** was obtained following the same procedures as for the Boc cleavage described in scheme 28, step 5 (example 68) using compound **204** as starting material (96% yield). ¹H NMR: (DMSO-d₆) d (ppm): 8.09 (d, J= 2.0 Hz, 1H), 8.02(d, J= 3.5 Hz, 1H), 7.74 (d, J= 8.8 Hz, 2H), 7.68-7.62 (m, 2H), 7.29-7.26 (m, 1H), 6.57 (d, J= 8.8 Hz, 2H), 4.29-4.26 (m, 1H), 3.71 (dd, J= 10.6, 5.7 Hz, 1H), 3.51-3.42 (m, 2H), 3.23 (dd, J= 10.6, 3.5 Hz, 1H), 2.35 (sext., J= 7.2 Hz, 1H), 2.06-1.98 (m, 1H). m/z: 284.4 (MH⁺).

### Step 5: N-(2-Amino-phenyl)-4-[3-(pyridin-3-ylamino)-pyrrolidin-1-yl]-benzamide (206)

The title compound **206** was obtained following the same procedures as for the BOP coupling described in scheme 1, step 5 (example 1) using compound **205** as starting material (10% yield).¹H NMR: (CD₃OD) δ(ppm): 8.14 (s, 1H), 7.96 (d, J= 2.3 Hz, 1H), 7.86 (d, J= 9.0 Hz, 2H), 7.77 (dd, J= 4.7, 1.4 Hz, 1H), 7.61-7.58 (m, 1H), 7.25 (dd, J= 6.1, 3.1 Hz, 1H), 7.18 (ddd, J= 8.4, 4.7, 0.8 Hz, 1H), 7.15 (dd, J= 8.0, 1.6 Hz, 1H), 7,11 (ddd, J= 8.2, 2.7, 1.4 Hz, 1H), 7.05 (td, J= 7.2, 1.4 Hz, 1H), 6.89 (dd, J= 8.0, 1.4 Hz, 1H), 6.76 (td, J= 7.6, 1.4Hz, 1H), 6.64 (d, J= 8.8 Hz, 2H), 4.26 (quint, J= 4.3 Hz, 1H), 3.78 (dd, J= 10.0, 6.1 Hz, 1H), 3.62-3.56 (m, 1H), 3.53-3.47 (m, 1H), 3.31-3.29 (m, 1H), 2.42 (sext., J= 7.4 Hz, 1H), 2.12-2.08 (m, 1H). m/z: 374.4 (MH⁺).

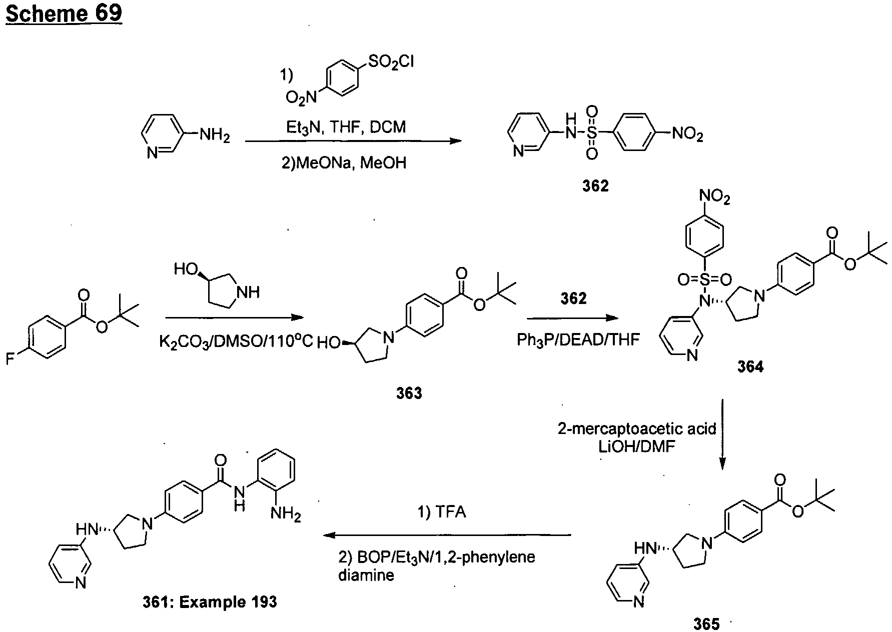

### Example 193:

### (S)-N-(2-Aminophenyl)-4-(3-(pyridin-3-ylamino)pyrrolidin-1-yl)benzamide (361)

### Step 1: N-p-Nosyl-3-pyridine (362):

To a stirred solution of 2-aminopyridine (3.03 g, 32.2 mmol) in THF (15mL) were successively added DCM (30 mL), 4-nitrobenzenesulfonyl chloride (1.50g, 68.7mmol), and Et₃N (9.88mL, 70.9mmol). The solution turned orange and a precipitate formed. The suspension was allowed to stir at room temperature for 1h, solvents were evaporated under reduced pressure and the solid residue was suspended in methanol (200 mL). To the suspension a large excess (>10 eq) of sodium methoxide was added, the mixture was stirred at 50°C for 3 h, quenched with HCl 1N (2mL) and concentrated under reduced pressure at 80°C until the volume became ∼ 50mL. The concentrated solution was further acidified with 1N HCl until neutral pH. A precipitate formed which was collected by filtration to afford the title compound (7.67 g, 85% yield). ¹H NMR (DMSO-d₆) δ (ppm): 10.88 (s, 1H), 8.36 (d, J=9.0 Hz, 2H), 8.28 (dd, J=6.1, 1.4 Hz, 1H), 8.27 (d, J=2.5 Hz, 1H), 7.50 (ddd, J=8.4, 2.7, 1.6 Hz, 1H), 7.30 (ddd, J=8.2, 4.7, 0.8 Hz, 1H). m/z: 280.1 (MH⁺).

### Step 2: tert-Butyl 4-((R)-3-hydroxypyrrolidin-1-yl)benzoate (363):

To a solution of *t-*butyl 4-fluorobenzoate (2.17g, 11.0 mmol) and (R)-(+)-3-pyrrolidinol (1.00g, 11.5 mmol) in DMSO (8 mL) was added potassium carbonate (1.53g, 11.0 mmol). The mixture was stirred at 130°C for 18h and poured into stirring water (100mL) while still hot. The resulting beige precipitate was collected by filtration and dried at 120°C for 1.5h to afford the title compound (2.64g, 91% yield). ¹H NMR (Acetone-d₆) δ (ppm): 7.78 (d, J=9.0 Hz, 2H), 6.54 (d, J=8.8 Hz, 2H), 4.58 (bs, 1H), 4.15 (bs, 1H), 3.55 (dd, J=10.36, 4.7 Hz, 1H), 3.49 (t, J=6.8 Hz, 1H), 3.42 (td, J=9.2, 2.3 Hz, 1H), 3.28 (d, J=10.8 Hz, 1H), 2.21-2.10 (m, 1H), 2.09-2.03 (m, 1H), 1.56 (s, 9H).m/z: 520.3 (MH⁺).

### Step 3: tert-Butyl 4-((S)-3-N-p-nosyl (pyridin-3-ylamino)pyrrolidin-1-yl)benzoate (364)

To a solution of compound **362** (6.00g, 21.5 mmol) in THF (100 mL), were successively added carbinol **363** (5.66g, 21.5 mmol), triphenylphosphine (6.76g, 25.8 mmol) and diethyl azodicarboxylate (4.06mL, 25.8 mmol). The mixture was stirred at room temperature for 18h and the solvent was removed *in vacuo.* The residue was purified by flash chromatography using EtOAc/Hex (40:60) as an eluent to afford the title compound (4.68g, 42% yield). ¹H NMR (DMSO-d₆) d(ppm): 8.58 (dd, J=4.7, 1.4 Hz, 1H), 8.48 (d, J=8.0 Hz, 2H), 8.38 (d, J=2.0 Hz, 1H), 8.13 (d, J=9.0, 2H), 7.72 (d, J=9.0 Hz, 2H), 7.61 (ddd, J=8.0, 2.5, 1.6 Hz, 1H), 7.39 (dd, J=8.2, 4.9 Hz, 1H), 6.43 (d, J=9.0 Hz, 2H), 5.17 (quint, J=8.2 Hz, 1H), 3.77 (dd, J=10.4, 7.2 Hz, 1H), 3.36 (dd, J=10.4, 6.7 Hz, 1H), 3.26 (dd, J=15.1, 7.8 Hz, 1H), 3.06 (td, J=12.3, 3.3 Hz, 1H), 2.43-2.38 (m, 1H), 2.02-1.94 (m, 1H), 1.55 (s, 9H). m/z: 525.3 (MH⁺).

### Step 4: tert-Butyl 4-((S)-3-(pyridin-3-ylamino)pyrrolidin-1-yl)benzoate (365)

To a solution of the nitro compound **364** (4.68g, 8.92 mmol) in DMF (45mL), were successively added lithium hydroxide (1.31g, 31.2 mmol) and thioglycolic acid (930µL, 13.4 mmol). The mixture was stirred for 3 days at room temperature, the solvent was removed in *vacuo* at 80°C and the residue was partitioned between EtOAc and H₂O. Organic layer was collected and extracted with HCl 1N. Acidic layer was collected and neutralized with a saturated NaHCO₃ solution. A white precipitate was formed which was extracted with EtOAc. The EtOAc solution was washed with brine, dried over MgS04, and concentrated *in vacuo* to afford the title compound (1.65g, 54%yield) as a white solid. ¹H NMR: (Acetone-d₆) δ(ppm): 8.08 (d, J=2.2 Hz, 1H), 7.86 (d, J=4.3 Hz, 1H), 7.79 (d, J=9.0 Hz, 2H), 7.09 (dd, J= 8.2, 4.3 Hz, 1H), 7.05 (ddd, J=8.2, 2.7, 1.6 Hz, 1H), 6.57 (d, J= 8.8 Hz, 2H), 5.54 (d, J=6.5 Hz, 1H), 4.32 (sext, J=5.3 Hz, 1H), 3.78 (dd, J=10.2, 5.9 Hz, 1H), 3.56 (dd, J=17.0, 7.2 Hz, 1H), 3.47 (td, J=8.0, 5.1 Hz, 1H), 3.31 (dd, J=10.2, 3.9 Hz, 1H), 2.44 (sext., J=7.8 Hz, 1H), 2.13 (sext, J=5.1 Hz, 1H), 1.56 (s, 9H). m/z: 340.3 (MH⁺).

### Step 5. (S)-N-(2-Aminophenyl)-4-(3-(pyridin-3-ylamino)pyrrolidin-1-yl)benzamide (361)

To a suspension of compound **365** (19mg, 0.56 mmol) in DCM 500µL was added trifluoroacetic acid (200µL). The solution was refluxed at 50°C for 3h and concentrated *in vacuo* to produce a white solid. This material was dissolved in DMF (500µL) and was treated with Et₃N (16µL, 0.118 mmol) and BOP (30mg, 0.067 mmol). The reaction mixture was stirred for 10 min. and 1,2-henylenediamine (7mg, 0.061 mmol) and another portion of Et₃N (23µL, 0.168 mmol) were added. The mixture was stirred for 2h at room temperature and DMF was removed *in vacuo* at 80°C. The residue was partitioned between EtOAc and H₂O. The organic layer was collected and extracted with 1N HCl and neutralized with sat. NaHCO₃. A precipitate formed which was extracted with EtOAc, dried over MgSO₄, and concentrated *in vacuo.* The residue was purified by flash chromatography using MeOH/CHCl₃ (7:93) as the eluent to afford the title compound (11 mg, 52% yield). ¹H NMR: (CD₃OD) δ(ppm): 7.97 (d, J=2.7 Hz, 1H), 7.86 (d, J=8.8 Hz, 2H), 7.78 (dd, J=4.7, 1.0 Hz, 1H), 7.18-7.10 (m, 3H), 7.05 (td, J=7.4, 0.6 Hz, 1H), 6.89 (dd, J=7.8, 1.2 Hz, 1H), 6.76 (td, J=7.4, 1.4 Hz, 1H), 6.64 (d, J=8.8 Hz, 2H), 4.25 (quint, J=4.9 Hz, 1H), 3.77 (dd, J=10.2, 6.1 Hz, 1H), 3.57 (dd, J=17.0, 7.0 Hz, 1H), 3.49 (td, J=8.0, 5.3 Hz, 1H), 3.29 (q, J=6.7Hz, 1H), 2.41 (sext, J=7.2 Hz, 1H), 2.10 (sext, J=4.9 Hz, 1H). m/z: 372.4 (MH⁺).

### Example 194:

### (R)-N-(2-Aminophenyl)-4-(3-(pyridin-3-ylamino)pyrrolidin-1-yl)benzamide (366)

The title compound was obtained following the same procedures described in scheme 69, example 193 but substituting (R)-(+)-3-pyrrolidinol for (S)-(-)-3-pyrrolidinol. (108mg, 21% yield) ¹H NMR: (DMSO-d₆) δ(ppm): 9.34 (s, 1H), 8.00 (d, J=2.3 Hz, 1H), 7.84 (d, J=8.8 Hz, 2H), 7.77 (dd, J=4.7, 1.2 Hz, 1H), 7.12 (d, J=7.6 Hz, 1H), 7.08 (dd, J=8.0, 4.5 Hz, 1H), 6.99-6.97 (m, 1H), 6.92 (t, J=7.8 Hz, 1H), 6.75 (d, J=7.8 Hz, 1H), 6.60-6.56 (m, 3H), 6.17 (d, J=6.8 Hz, 1H), 4.81 (s, 2H), 4.19-4.17 (m, 1H), 3.71 (dd, J=10.2, 6.5 Hz, 1H), 3.53-3.47 (m, 1H), 3.42-3.38 (m, 1H), 3.18 (dd, J=10.4, 4.1 Hz, 1H), 2.32 (sext, J=6.3 Hz, 1H), 1.99 (sext, J=4.7 Hz, 1H). m/z: 374.2 (MH⁺).

### Example 195:

### (S)-N-(2-Aminophenyl)-4-(3-(pyridin-3-yloxy)pyrrolidin-1-yl)benzamide (367)

The title compound was obtained following the same procedures described in scheme 69, example 193 but skipping steps 1 and 4 and substituting compound **362** for 3-hydroxypyridine (24mg, 44% yield) ¹H NMR: (Acetone-d₆) δ(ppm): 8.88 (s, 1H), 8.31 (d, J=2.9 Hz, 1H), 8.19 (d, J=4.7 Hz, 1H), 7.93 (d, J=8.8 Hz, 2H), 7.41 (ddd, J=8.4, 2.9, 1.4 Hz, 1H), 7.31 (ddd, J=8.4, 4.5, 0.6 Hz, 1H), 7.26 (d, J=7.8 Hz, 1H), 6.97 (td, J=7.8, 1.4 Hz, 1H), 6.85 (dd, J=8.0, 1.4 Hz, 1H), 6.66 (d, J=9.0 Hz, 2H), 6.65 (td, J=8.0, 1.4 Hz, 1H), 5.34-5.32 (m, 1H), 4.62 (s, 2H), 3.84 (dd, J=11.3, 4.7 Hz, 1H), 3.61-3.56 (m, 3H), 2.50-2.34 (m, 2H). m/z: 375.2 (MH⁺).

### Example 196:

### (R)-N-(2-Aminophenyl)-4-(3-(pyridin-3-yloxy)pyrrolidin-1-yl)benzamide (368)

The title compound was obtained following the same procedures described in scheme 69, example 193 skipping steps 1 and 4 and substituting compound **362** for 3-hydroxypyridine and (R)-(+)-3-pyrrolidinol for (S)-(-)-3-pyrrolidinol. (14mg, 12% yield) ¹H NMR: (Acetone-d₆) δ(ppm): 8.85 (s, 1H), 8.31 (d, J=2.9 Hz, 1H), 8.19 (dd, J=4.5, 1.2 Hz, 1H), 7.93 (d, J=8.8 Hz, 2H), 7.42 (ddd, J=8.4, 2.9, 1.4 Hz, 1H), 7.31 (ddd, J=8.4, 4.7, 0.8 Hz, 1H), 7.26 (d, J=7.8, 1.6Hz, 1H), 6.97 (td, J=7.2, 1.6 Hz, 1H), 6.85 (dd, J=7.8, 1.2 Hz, 1H), 6.68 (d, J=8.8Hz, 6.66 (td, J= 7.6, 1.4 Hz, 1H), 3.56-5.33 (m, 1H), 4.60 (bs, 2H), 3.86 (dd, J=11.3, 4.7 Hz, 1H), 3.62-3.57 (m, 3H), 2.50-2.35 (m, 2H). m/z: 375.2 (MH⁺).

### Example 197:

### (S)-N-(2-Aminophenyl)-4-(3-(phenylamino)pyrrolidin-1-yl)benzamide (369)

The title compound was obtained following the same procedures described in scheme 69, example 193 but substituting compound 3-aminopyridine for aniline. (7 mg, 16% yield) ¹H NMR: (Acetone-d₆) δ(ppm): 8.84 (s, 1H), 7.91 (d, J=8.8 Hz, 2H), 7.25 (dd, J=7.8, 1.2 Hz, 1H), 7.12 (t, J=7.2 Hz, 2H), 6.96 (dt, J=8.0, 1.4 Hz, 1H), 6.85 (d, J=8.0 Hz, 1H), 6.71 (dd, J=8.8, 1.0 Hz, 2H), 6.66 (t, J=7.8 Hz, 1H), 6.62 (d, J=8.8 Hz, 2H), 5.26 (d, J=7.6 Hz, 1H), 4.60 (bs, 1H), 4.30 (quint, J=5.3 Hz, 1H), 3.79 (dd, J=10.0 Hz, 1H), 3.57 (q, J=9.6 Hz, 1H), 3.50-3.45 (m, 1H), 3.30 (dd, J=10.2, 3.9 Hz, 1H), 2.42 (sext, J=6.8 Hz, 1H). m/z: 373.1 (MH⁺).

### Example 198:

### (R)-N-(2-Aminophenyl)-4-(3-(phenylamino)pyrrolidin-1-yl)benzamide (370)

The title compound was obtained following the same procedures described in scheme 69, example 193 but substituting 3-aminopyridine for aniline and (R)-(+)-3-pyrrolidinol for (S)-3-pyrrolidinol. (22 mg, 23% yield) ¹H NMR (CDCl₃) δ 7.79 (m, 2H), 7.2-7.4 (m, 2H), 7.05 (s, 1H), 6.8 (m, 3H) 6.65 (m, 2H), 6.53(m, 2H), 4.24 (br.s., 1H), 3.9(m, 2H), 3.73 (m, 1H), 3.26 (m, 1H), 2.37 (m, 1H), 2.09 (m, 1H) m/z: 373.3 (MH⁺).

### Example 199:

### (S)-N-(2-Aminophenyl)-4-(3-phenoxypyrrolidin-1-yl)benzamide (371)

The title compound was obtained following the same procedures described in scheme 69, example 193 but skipping steps 1 and 4 and substituting compound **362** for phenol. (50 mg, 33% yield) ¹H NMR (CDCl₃) δ 7.79 (µ, 3H), 7.3 (m, 3H), 7.03 (m, 1H), 6.96 (m, 1H), 6.90 (d, 2H, J=8.8 Hz), 6.80 (m, 2H), 6.54 (d, J=8.8 Hz, 2H), 5.08 (br.s., 1H), 3.71 (dd, J=4.7 Hz, J=11.0 Hz, 1H), 3.6 (m, 3H), 2.41 (m, 1H), 2.31 (m, 1H) m/z: 374.2 (MH⁺).

### Example 200:

### (S)-Methyl-4-(1-(4-(2-aminophenylcarbamoyl)phenyl)pyrrolidin-3-yloxy)benzoate (372)

The title compound was obtained following the same procedures described in scheme 69, example 193 but skipping steps 1 and 4 and substituting compound **362** for methyl 4-hydroxybenzoate. (143 mg, 42% yield) ¹H NMR (CDCl₃) δ 8.0 (m, 2H), 7.81 (m, 2H), 7.72 (s, 1H), 7.25 (m, 1H), 7.06 (m, 1H), 6.91(m, 2H), 6.84 (m, 2H), 6.59 (m, 2H), 5.16 (br.s., 1H), 3.9(s, 3H), 3.78 (m, 1H), 3.60 (m, 3H), 2.4 (m, 2H) m/z: 432.4 (MH⁺).

### Example 201:

### (S)-4-(1-(4-(2-Aminophenyl carbamoyl)phenyl)pyrrolidin-3-yloxy)benzoic acid (373)

A solution of **372** (100 mg, 0.23 mmol) and KOH (100 mg, 1.78 mmol) in 1:1:1 mixture of THF/water/MeOH (6 mL) was stirred at room temperature for 5 days. The reaction mixture was concentrated and partitioned between water (5 mL) and ether (5 mL). Organic phase was discarded and the aqueous phase was acidified to pH=6 using 1M HCl solution and extracted with EtOAc. The extract was dried over Na₂SO4, filtered and concentrated. The residue was purified by flash chromatography, eluent MeOH-DCM (gradient of MeOH from 5 till 20% MeOH) to afford the title compound (20 mg, 21% yield). ¹H NMR (DMSO) δ 9.34 (σ, 1H), 7.85 (m, 4H), 7.11 (d, 1H, J=7.8 Hz), 7.00 (d, J=8.4 Hz, 2H), 6.92 (d, 2H, J=7.7 Hz), 6.75 (d, J=8.0 Hz, 2H), 6.6 (m, 3H), 5.26 (br.s., 1H), 3.75 (m, 1H), [3.34 DMSO, 4H], 2.44 (m, 1H), 2.31 (m, 1H) m/z: 418.4 (MH⁺).

### Example 202

### (S)-N-(2-Aminophenyl)-4-(3-(3,4,5-trimethoxyphenoxy)pyrrolidin-1-yl)benzamide (374)

The title compound was obtained following the same procedures described in scheme 69, example 193 but skipping steps 1 and 4 and substituting compound **362** for 3,4,5-trimethoxyphenol. (30 mg, 21%) ¹H NMR (CDCl₃) δ 7.79 (d, 2H, J=8.8Hz), 7.73 (s, 1H), 7.26 (d, 1H, J=7.4 Hz), 7.05 (t, J=7.7 Hz, 1H), 6.81 (d, 2H, J=7.7 Hz), 6.57 (d, J=8.7 Hz, 2H), 6.14 (s, 2H), 5.04 (br.s., 1H), 3.88 (s, 6H), 3.80 (s, 3H), 3.71 (dd, J=4.7 Hz, J=11.0 Hz, 1H), 3.6 (m, 3H), 2.41 (m, 1H), 2.31 (m, 1H) m/z: 464.4 (MH⁺).

### Example 203:

### (S)-N-(2-Aminophenyl)-4-(3-(benzo[d][1,3]dioxol-5-yloxy)pyrrolidin-1-yl)benzamide (375)

The title compound was obtained following the same procedures described in scheme 69, example 193 but skipping steps 1 and 4 and substituting compound **362** for benzo[d][1,3]dioxol-5-ol. (31 mg, 15%) ¹H NMR (CDCl₃) 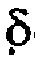 8.95 (s, 1H), 7.87 (d, 2H, J=8.0Hz), 7.80 (d, J=8.7 Hz, 1H), 7.67 (s, 1H), 7.48 (s, .5H) 7.04 (m, 1H), 6.83(m, 1H), 6.71 (m, 1H), 6.57 (d, 2H), 6.48 (s, 1H), 6.33 (m, 1H), 5.93 (s, 2H), 4.96 (br.s., 1H), 3.67(m, 1H), 3.57 (m, 3H), 2.36 (m, 1H), 2.26 (m, 1H) m/z: 418.2 (MH⁺).

### Example 204:

### (S)-N-(2-Aminophenyl)-4-(3-(4-phenoxyphenoxy)pyrrolidin-1-yl)benzamide (376)

The title compound was obtained following the same procedures described in scheme 69, example 193 but skipping steps 1 and 4 and substituting compound **362** for 4-phenoxyphenol. ¹H NMR (CDCl₃) δ 7.81 (m, 2H), 7.70 (s, 1H), 7.67 (s, .5H), 7.2-7.4 (m, 4 H) 6.8-7.2 (m, 10H), 6.6(m, 2H), 5.05 (br.s., 1H), 3.6(m, 1H), 3.5 (m, 3H), 2.41 (m, 1H), 2.32 (m, 1H) m/z: 466.4 (MH⁺).

### Example 205:

### (S)-N-(2-Aminophenyl)-4-(3-(4-nitrophenoxy)pyrrolidin-1-yl)benzamide (377)

The title compound was obtained following the same procedures described in scheme 69, example 193 but skipping steps 1 and 4 and substituting compound **362** for 4-nitrophenol. (12 mg, 6%) ¹H NMR (CDCl₃) δ 8.12 (d, 2H, J=9.1 Hz), 7.72 (d, J=8.8 Hz, 2H), 7.18 (d, J=7.3 Hz, 1H), 6.97 (t, 1H, J=7.7 Hz), 6.87 (d, J=9.1 Hz, 2H), 6.50 (d, 2H, J=8.6Hz), 5.09 (br.s., 1H), 3.71 (dd, J=4.5 Hz, J=11.3 Hz, 1H), 3.6 (m, 3H2.3 (m, 2H) m/z: 419.1 (MH⁺).

### Example 206:

### (S)-N-(2-Aminophenyl)-4-(3-(pyridin-2-ylthio)pyrrolidin-1-yl)benzamide (378)

The title compound was obtained following the same procedures described in scheme 69, example 193 but skipping steps 1 and 4 and substituting compound **362** for pyridine-2-thiol. (22 mg, 28%) ¹H NMR (CDCl₃) δ 8.44 (µ, 1H), 7.78 (d, J=8.8 Hz, 2H), 7.69 (s, 1H), 7.49 (t, 1H, J=7.4 Hz), 7.27 (m, 1H), 7.18 (d, 1H, J=8.0Hz), 7.0-7.1 (m, 2H), 6.82 (d, 7.8 Hz, 2H), 6.55 (d, J=9.0 Hz, 2H), 4.55 (m, 1H), 3.9-4.0 (m, 3H), 3.4-3.6 (m, 4H) 2.6 (m, 1H), 2.2 (m, 1H) m/z: 391.0 (MH⁺).

**Table 13. Characterization of examples 193-206 prepared according to the scheme 69.**

| | | | | | |
|---|---|---|---|---|---|
| 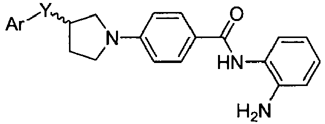 | | | | | |

| **Ex.** | **Cpd** | **Ar** | **Y** | **Name** | **Characterization** |
|---|---|---|---|---|---|
| **193** | **361** | 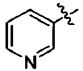 | NH | (S)-*N*-(2-aminophenyl)-4-(3-(pyridin-3-ylamino)pyrrolidin-1-yl)benzamide | ¹H NMR: (CD₃OD) δ(ppm): 7.97 (d, J=2.7 Hz, 1H), 7.86 (d, J=8.8 Hz, 2H), 7.78 (dd, J=4.7, 1.0 Hz, 1H), 7.18-7.10 (m, 3H), 7.05 (td, J=7.4, 0.6 Hz, 1H), 6.89 (dd, J=7.8, 1.2 Hz, 1H), 6.76 (td, J=7.4, 1.4 Hz, 1H), 6.64 (d, J=8.8 Hz, 2H), 4.25 (quint, J=4.9 Hz, 1H), 3.77 (dd, J=10.2, 6.1 Hz, 1H), 3.57 (dd, J=17.0, 7.0 Hz, 1H), 3.49 (td, J=8.0, 5.3 Hz, 1H), 3.29 (q, J=6.7Hz, 1H), 2.41 (sext, J=7.2 Hz, 1H), 2.10 (sext, J=4.9 Hz, 1H). |
| **194** | **366** | 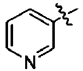 | NH | (R)-*N*-(2-aminophenyl)-4-(3-(pyridin-3-ylamino)pyrrolidin-1-yl)benzamide | ¹H NMR: (DMSO-d₆) δ(ppm): 9.34 (s, 1H), 8.00 (d, J=2.3 Hz, 1H), 7.84 (d, J=8.8 Hz, 2H), 7.77 (dd, J=4.7, 1.2 Hz, 1H), 7.12 (d, J=7.6 Hz, 1H), 7.08 (dd, J=8.0, 4.5 Hz, 1H), 6.99-6.97 (m, 1H), 6.92 (t, J=7.8 Hz, 1H), 6.75 (d, J=7.8 Hz, 1H), 6.60-6.56 (m, 3H), 6.17 (d, J=6.8 Hz, 1H), 4.81 (s, 2H), 4.19-4.17 (m, 1H), 3.71 (dd, J=10.2, 6.5 Hz, 1H), 3.53-3.47 (m, 1H), 3.42-3.38 (m, 1H), 3.18 (dd, J=10.4, 4.1 Hz, 1H), 2.32 (sext, J=6.3 Hz, 1H), 1.99 (sext, J=4.7 Hz, 1H). |

| **Ex.** | **Cpd** | **Ar** | **Y** | **Name** | **Characterization** |
|---|---|---|---|---|---|
| **195** | **367** | 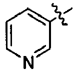 | O | (S)-N-(2-aminophenyl)-4-(3-(pyridin-3-yloxy)pyrrolidin-1-yl)benzamide | ¹H NMR: (Acetone-d₆) δ(ppm): 8.88 (s, 1H), 8.31 (d, J=2.9 Hz, 1H), 8.19 (d, J=4.7 Hz, 1H), 7.93 (d, J=8.8 Hz, 2H), 7.41 (ddd, J=8.4, 2.9, 1.4 Hz, 1H), 7.31 (ddd, J=8.4, 4.5, 0.6 Hz, 1H), 7.26 (d, J=7.8 Hz, 1H), 6.97 (td, J=7.8, 1.4 Hz, 1H), 6.85 (dd, J=8.0, 1.4 Hz, 1H), 6.66 (d, J=9.0 Hz, 2H), 6.65 (td, J=8.0, 1.4 Hz, 1H), 5.34-5.32 (m, 1H), 4.62 (s, 2H), 3.84 (dd, J=11.3, 4.7 Hz, 1H), 3.61-3.56 (m, 3H), 2.50-2.34 (m, 2H). |
| **196** | **368** | 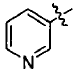 | O | (R)-N-(2-aminophenyl)-4-(3-(pyridin-3-yloxy)pyrrolidin-1-yl)benzamide | ¹H NMR: (Acetone-d₆) δ(ppm): 8.85 (s, 1H), 8.31 (d, J=2.9 Hz, 1H), 8.19 (dd, J=4.5, 1.2 Hz, 1H), 7.93 (d, J=8.8 Hz, 2H), 7.42 (ddd, J=8.4, 2.9, 1.4 Hz, 1H), 7.31 (ddd, J=8.4, 4.7, 0.8 Hz, 1H), 7.26 (d, J=7.8, 1.6Hz, 1H), 6.97 (td, J=7.2, 1.6 Hz, 1H), 6.85 (dd, J=7.8, 1.2 Hz, 1H), 6.68 (d, J=8.8Hz, 6.66 (td, J= 7.6, 1.4 Hz, 1H), 3.56-5.33 (m, 1H), 4.60 (bs, 2H), 3.86 (dd, J=11.3, 4.7 Hz, 1H), 3.62-3.57 (m, 3H), 2.50-2.35 (m, 2H). |
| **197** | **369** | 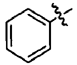 | NH | (S)-N-(2-aminophenyl)-4-(3-(phenylamino)pyrrolidin-1-yl)benzamide | ¹H NMR: (Acetone-d₆) δ(ppm): 8.84 (s, 1H), 7.91 (d, J=8.8 Hz, 2H), 7.25 (dd, J=7.8, 1.2 Hz, 1H), 7.12 (t, J=7.2 Hz, 2H), 6.96 (dt, J=8.0, 1.4 Hz, 1H), 6.85 (d, J=8.0 Hz, 1H), 6.71 (dd, J=8.8, 1.0 Hz, 2H), 6.66 (t, J=7.8 Hz, 1H), 6.62 (d, J=8.8 Hz, 2H), 5.26 (d, J=7.6 Hz, 1H), 4.60 (bs, 1H), 4.30 (quint, J=5.3 Hz, 1H), 3.79 (dd, J=10.0 Hz, 1H), 3.57 (q, J=9.6 Hz, 1H), 3.50-3.45 (m, 1H), 3.30 (dd, J=10.2, 3.9 Hz, 1H), 2.42 (sext, J=6.8 Hz, 1H). |

| **Ex.** | **Cpd** | **Ar** | **Y** | **Name** | **Characterization** |
|---|---|---|---|---|---|
| **198** | **370** | 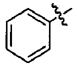 | NH | (R)-N-(2-aminophenyl)-4-(3-(phenylamino)pyrrolidin-1-yl)benzamide | ¹H NMR (CDCl₃) δ (ppm) 7.79 (m, 2H), 7.2-7.4 (m, 2H), 7.05 (s, 1H), 6.8 (m, 3H) 6.65 (m, 2H), 6.53(m, 2H), 4.24 (br.s., 1H), 3.9(m, 2H), 3.73 (m, 1H), 3.26 (m, 1H), 2.37 (m, 1H), 2.09 (m, 1H) |
| **199** | **371** | 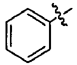 | O | (S)-N-(2-aminophenyl)-4-(3-phenoxypyrrolidin-1-yl)benzamide | ¹H NMR (CDCl₃) δ (ppm) 7.79 ((m, 3H), 7.3 (m, 3H), 7.03 (m, 1H), 6.96 (m, 1H), 6.90 (d, 2H, J=8.8 Hz), 6.80 (m, 2H), 6.54 (d, J=8.8 Hz, 2H), 5.08 (br.s., 1H), 3.71 (dd, J=4.7 Hz, J=11.0 Hz, 1H), 3.6 (m, 3H), 2.41 (m, 1H), 2.31 (m, 1H) |
| **200** | **372** | 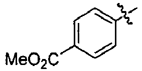 | O | (S)-methyl-4-(1-(4-(2-aminophenyl carbamoyl)phenyl)pyrrolidin-3-yloxy)benzoate | ¹H NMR (CDCl₃) δ (ppm): 8.0 (m, 2H, 7.81 (m, 2H), 7.72 (s, 1H), 7.25 (m, 1H), 7.06 (m, 1H), 6.91(m, 2H), 6.84 (m, 2H), 6.59 (m, 2H), 5.16 (br.s., 1H), 3.9(s, 3H), 3.78 (m, 1H), 3.60 (m, 3H), 2.4 (m, 2H) |
| **201** | **373** | 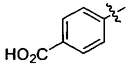 | O | (S)-4-(1-(4-(2-aminophenyl carbamoyl)phenyl)pyrrolidin-3-yloxy)benzoic acid | ¹H NMR: (DMSO-d₆) δ(ppm): 9.34 (s, 1H), 7.85 (m, 4H), 7.11 (d, 1H, J=7.8 Hz), 7.00 (d, J=8.4 Hz, 2H), 6.92 (d, 2H, J=7.7 Hz), 6.75 (d, J=8.0 Hz, 2H), 6.6 (m, 3H), 5.26 (br.s., 1H), 3.75 (m, 1H), [3.34 DMSO, 4H], 2.44 (m, 1H), 2.31 (m, 1H) |
| **202** | **374** | 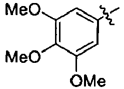 | O | (S)-N-(2-aminophenyl)-4-(3-(3,4,5-trimethoxyphenoxy)pyrrolidin-1-yl)benzamide | ¹H NMR (CDCl₃) δ (ppm) 7.79 (d,2H; , J=8.8Hz), 7.73 (s, 1H), 7.26 (d, 1H, J=7.4 Hz), 7.05 (t, J=7.7 Hz, 1H), 6.81 (d, 2H, J=7.7 Hz), 6.57 (d, J=8.7 Hz, 2H), 6.14 (s, 2H), 5.04 (br.s., 1H), 3.88 (s, 6H), 3.80 (s, 3H), 3.71 (dd, J=4.7 Hz, J=11.0 Hz, 1H), 3.6 (m, 3H), 2.41 (m, 1H), 2.31 (m, 1H) |

| **Ex.** | **Cpd** | **Ar** | **Y** | **Name** | **Characterization** |
|---|---|---|---|---|---|
| **203** | **375** | 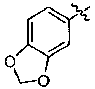 | O | (S)-N-(2-aminophenyl)-4-(3-(benzo[d][1,3]dioxol-5-yloxy)pyrrolidin-1-yl)benzamide | ¹H NMR (CDCl₃) δ (ppm) 8.95 (s, 1H), 7.87 (d, 2H, J=8.0Hz), 7.80 (d, J=8.7 Hz, 1H), 7.67 (s, 1H), 7.48 (s, .5H) 7.04 (m, 1H), 6.83(m, 1H), 6.71 (m, 1H), 6.57 (d, 2H), 6.48 (s, 1H), 6.33 (m, 1H), 5.93 (s, 2H), 4.96 (br.s., 1H), 3.67(m, 1H), 3.57 (m, 3H), 2.36 (m, 1H), 2.26 (m, 1H) |
| **204** | **376** | 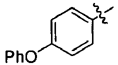 | O | (S)-N-(2-aminophenyl)-4-(3-(4-phenoxyphenoxy)pyrrolidin-1-yl)benzamide | ¹H NMR (CDCl₃) δ (ppm) 7.81 (m, 2H), 7.70 (s, 1H), 7.67 (s, .5H), 7.2-7.4 (m, 4 H) 6.8-7.2 (m, 10H), 6.6(m, 2H), 5.05 (br.s., 1H), 3.6(m, 1H), 3.5 (m, 3H), 2.41 (m, 1H), 2.32 (m, 1H) |
| **205** | **377** | 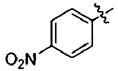 | O | (S)-N-(2-aminophenyl)-4-(3-(4-nitrophenoxy)pyrrolidin-1-yl)benzamide | ¹H NMR (CDCl₃) δ (ppm) 8.12 (d, 2H, J=9.1 Hz), 7.72 (d, J=8.8 Hz, 2H), 7.18 (d, J=7.3 Hz, 1H), 6.97 (t, 1H, J=7.7 Hz), 6.87 (d, J=9.1 Hz, 2H), 6.50 (d, 2H, J=8.6Hz), 5.09 (br.s., 1H), 3.71 (dd, J=4.5 Hz, J=11.3 Hz, 1H), 3.6 (m, 3H2.3 (m, 2H) |
| **206** | **378** | 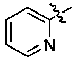 | S | (S)-N-(2-aminophenyl)-4-(3-(pyridin-2-ylthio)pyrrolidin-1-yl)benzamide | ¹H NMR (CDCl₃) δ (ppm) 8.44 (m, 1H), 7.78 (d, J=8.8 Hz, 2H), 7.69 (s, 1H), 7.49 (t, 1H, J=7.4 Hz), 7.27 (m, 1H), 7.18 (d, 1H, J=8.0Hz), 7.0-7.1 (m, 2H), 6.82 (d, 7.8 Hz, 2H), 6.55 (d, J=9.0 Hz, 2H), 4.55 (m, 1H), 3.9-4.0 (m, 3H), 3.4-3.6 (m, 4H) 2.6 (m, 1H), 2.2 (m, 1H) |

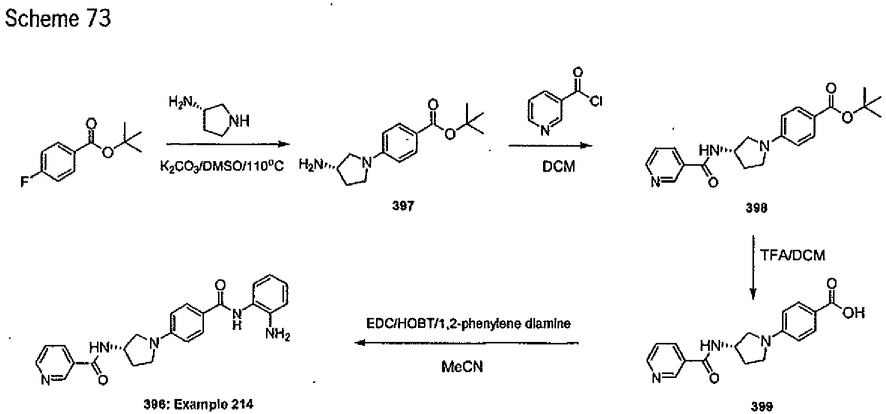

### Example 214:

### (S)-N-(1-(4-(2-Aminophenyl carbamoyl)phenyl)pyrrolidin-3-yl)nicotinamide (396)

### Step 1. (S)-tert-Butyl 4-(3-aminopyrrolidin-1-yl)benzoate (397)

Title compound was obtained similarly to the aminoester **363** using the same procedure as described in step 2, scheme 69. ¹H NMR (CDCl₃) δ 7.83 (d, J=8.8 Hz, 1H), 6.46 (d, J=8.8 Hz, 1H), 3.75 (m, 1H), 3.35-3.6 (m, 3H), 3.06 (dd, J=4.7 Hz, J=9.8 Hz, 1H), 2.26 (m, 1H), 1.85 (m, 1H), 1.57 (s, 9H), LRMS: (calc) 262.1; (found) 263.0 (M+H¹).

### Step 2. (S)-tert-Butyl 4-(3-(nicotinamido)pyrrolidin-1-yl)benzoate (398)

A solution of **397** (100 mg, 0.38 mmol), Et₃N (160 µL, 1.14 mmol) and nicotinoyl chloride HCl salt (68 mg, 0.38 mmol) in DCM (2 mL) was stirred at room temperature for 1 hour and quenched by adding saturated NH₄Cl sat solution (5 mL). The organic phase was separated, dried over Na₂SO₄, filtered and concentrated to produce a residue which was purified by flash chromatography using 5% MeOH in DCM as an eluent to afford the title compound (110 mg, 79% yield). LRMS: (calc) 367.2; (found). 368.1 (M+H¹).

### Step 3. (S)-4-(3-(Nicotinamido)pyrrolidin-1-yl)benzoic acid (399)

The title compound was obtained as the mixture of monosalt and disalt similarly to the compound **117** using the same procedure as described in step 5, scheme 28. LRMS: (calc) 311.1; (found) 312.1 (M+H¹).

### Step 4. (S)-N-(1-(4-(2-AminophenylcarbamoYl)phenyl)pyrrolidin-3-yl)nicotinamide (396)

A solution of **399** (93 mg, 0.3 mmol), phenylene diamine (65 mg, 0.6 mmol), EDC (86 mg, 0.45 mmol), HOBT (53 mg, 0.33 mmol) and Et₃N (125 µL, 91 mg, 0.9 mmol) in acetonitrile (2 mL) was stirred at room temperature overnight. The reaction mixture was concentrated and the residue was purified by flash chromatography using the gradient 5-20% MeOH in DCM as an eluent to afford the title compound (24 mg, 16% yield). ¹H NMR (CDCl₃)
δ 8.97 (s, 1H), 8.66 (d, J=4.9 Hz, 1H), 8.21 (d, J=3.9 Hz, 1H), 7.87 (d, 2H, J=8.8 Hz), 7.52 (dd, J=5.1 Hz, J=8.0 Hz, 1H), 7.15 (d, 1H, J=7.9 Hz), 7.05 (t, J=8.1 Hz, 1H), 6.89 (d, J=7.6 Hz, 1H), 6.76 (t, J=7.3 Hz, 1H), 6.66 (d, J= 9.0 Hz, 2H), 4.78 (m, 1H), 3.80 (dd, J=6.7 Hz, J=10.2 Hz, 1H), 3.61 (m, 1H), 3.49 (m, 1H), 3.41 (m, 1H), 2.4 (m, 1H), 2.2 (m, 1H). LRMS: (calc) 401.2; (found) 402.2 (M+H¹).

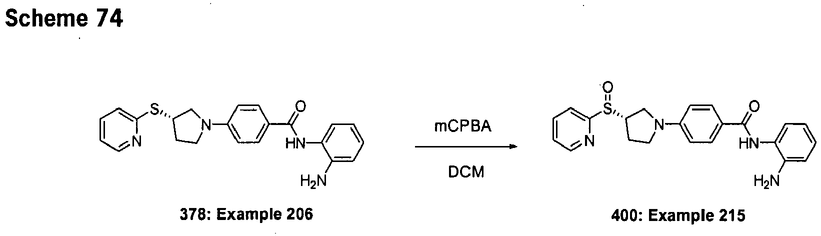

### Example 215:

### N-(2-Aminophenyl)-4-((S)-3-((S)-pyridin-2-ylsulfinyl)pyrrolidin-1-yl)benzamide (400)

A solution of **378** (15 mg, 0.04 mmol) and mCPBA (6 mg, 0.04 mmol) in DCM (2 mL) was stirred at room temperature for 1 hour. The reaction mixture was concentrated and the residue was purified by flash chromatography using the gradient EtOAc to 5% MeOH in DCM as an eluent, to afford the title compound (13 mg, 80% yield). ¹H NMR (CDCl₃) δ ¹H NMR (CDCl₃)
δ 8.63 (m, 1H), 8.58 (m, 1H), 7.6-8.0 (m, 10H), 7.40 (m, 2H), 7.25 (m, 1H), 7.05 (m, 2H), 6.85 (m, 3H), 6.58 (d, J=8.8 Hz, 2H), 6.50 (d, J=11.1 Hz, 2H), 3.7-4.0 (m, 6H), 3.2-3.5 (m, 4H), 2.4-2.8 (m, 3H), 2.95 (m, 1H). LRMS: (calc) 406.1; (found) 407.1 (M+H¹).

### Assay Example 1

### Inhibition of Histone Deacetylase Enzymatic Activity

### 1. Human HDAC-1

Assay 1. HDAC inhibitors were screened against a cloned recombinant human HDAC-1 enzyme expressed and purified from a Baculovirus insect cell expression system. For deacetylase assays, 20,000 cpm of the [³H]-metabolically labeled acetylated histone substrate (M. Yoshida et al., J. Biol. Chem. 265(28): 17174-17179 (1990)) was incubated with 30 µg of the cloned recombinant hHDAC-1 for 10 minutes at 37 °C. The reaction was stopped by adding acetic acid (0.04 M, final concentration) and HCl (250 mM, final concentration). The mixture was extracted with ethyl acetate and the released [³H]-acetic acid was quantified by scintillation counting. For inhibition studies, the enzyme was preincubated with compounds at 4 °C for 30 minutes prior to initiation of the enzymatic assay. IC₅₀ values for HDAC enzyme inhibitors were determined by performing dose response curves with individual compounds and determining the concentration of inhibitor producing fifty percent of the maximal inhibition.
Assay 2. The following protocol was also used to assay the compounds of the invention. In the assay, the buffer used was 25mM HEPES, pH 8.0, 137mM NaCl, 2.7mM KCI, 1mM MgCl₂ and the subtrate was Boc-Lys(Ac)-AMC in a 50mM stock solution in DMSO. The enzyme stock solution was 4.08 µg/mL in buffer. The compounds were pre-incubated (2µl in DMSO diluted to 13 µl in buffer for transfer to assay plate) with enzyme (20µl of 4.08µg/ml) for 10 minutes at room temperature (35µl pre-incubation volume). The mixture was pre-incubated for 5 minutes at room temperature. The reaction was started by bringing the temperature to 37°C and adding 16 µl substrate. Total reaction volume was 50µl. The reaction was stopped after 20 minutes by addition of 50µl developer, prepared as directed by Biomol (Fluor-de-Lys developer, Cat. # KI-105). A plate was incubated in the dark for 10 minutes at room temperature before reading (λ_{Ex}=360nm, λ_{Em}=470nm, Cutoff filter at 435nm).

IC₅₀ values for representative compounds are presented in Table 14. Assay 1 was used to measure HDAC activity of compounds 10c, 13e, 16d, 26b, 44, 47, 61a, 61b, 63, 134, 138, and 308. Assay 2 was used to measure HDAC activity of compounds 361, 366, 367, 368, 369, 370, 371, 372, 373, 374, 375, 376, 377, and 378. In Table 14, "a" indicates activity of ≤ 0.1 µM, "b" indicates activity of ≤ 1 µM, "c" indicates activity of ≤ 5 µM, and "d" indicates activity of > 5 µm. For the H4-Ac T24 EC vs. MS-275 assay in Table 14, "u" indicates less than 1, "v" indicates 1, and "w" indicates greater than 1. For the H3 Ac t24 assay in Table 14, "x" indicates activity of ≤ 1 µM, "y" indicates activity of ≤ 10 µM, and "z" indicates activity of ≤ 20 µM.

### 2. MTT Assay

HCT116 cells (2000/well) were plated into 96-well tissue culture plates one day before compound treatment. Compounds at various concentrations were added to the cells. The cells were incubated for 72 hours at 37°C in 5% CO₂ incubator. MTT (3-[4,5-dimethylthiazol-2yl]-2,5 diphenyl tetrazolium bromide, Sigma) was added at a final concentration of 0.5 mg/ml and incubated with the cells for 4 hours before one volume of solubilization buffer (50% N,N-dimethylformamide, 20% SDS, pH 4.7) was added onto the cultured cells. After overnight incubation, solubilized dye was quantified by colorimetric reading at 570 nM using a reference at 630 nM using an MR700 plate reader (Dynatech Laboratories Inc.). OD values were converted to cell numbers according to a standard growth curve of the relevant cell line. The concentration which reduces cell numbers to 50% of that of solvent treated cells is determined as MTT IC₅₀. IC₅₀ values for representative compounds are presented in Table 14. In Table 14, "a" indicates activity of ≤ 0.1 µM, "b" indicates activity of ≤ 1 µM, and "c" indicates activity of ≤ 5 µM.

### 3. Histone H4 acetylation in whole cells by immunoblots

T24 human bladder cancer cells growing in culture were incubated with HDAC inhibitors for 16 h. Histones were extracted from the cells after the culture period as described by M. Yoshida et al. (J. Biol. Chem. 265(28): 17174-17179 (1990)). 20 g of total histone protein was loaded onto SDS/PAGE and transferred to nitrocellulose membranes. Membranes were probed with polyclonal antibodies specific for acetylated histone H-4 (Upstate Biotech Inc.), followed by horse radish peroxidase conjugated secondary antibodies (Sigma). Enhanced Chemiluminescence (ECL) (Amersham) detection was performed using Kodak films (Eastman Kodak). Acetylated H-4 signal was quantified by densitometry. Representative data are presented in Table 14. Data are presented as the ratio of the concentration effective for reducing the acetylated H-4 signal by 50% (EC₅₀) using the indicated compound to a control compound, MS-275. If the indicated ratio is 1, then the compound is as effective as the MS-275 control compound, If the ratio is less than 1, then the compound is more effective than the MS-275 control compound. Further information regarding the MS-275 compound can be found in Suzuki et al., J. Med. Chem. 1999, pp. 3001-3003.

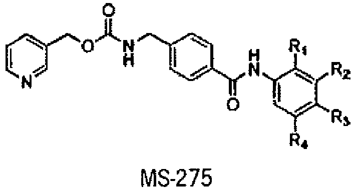

### 4. Histone H3 acetylation assay

T24 human bladder cancer cells growing in culture are incubated with HDAC inhibitors for 16 h. Cell viability is determined by adding 10 µl Alamar Blue (BioSource, DAL1100). Cells are washed once with PBS and fixed with methanol precooled to -20 °C for 10 min. The cells are then washed twice in PBS. The fixed cells are blocked with 50 µl of PBS + 0.1% Triton X-100. Cells are probed with rabbit-anti-acetyl-H3 (Upstate #06-599) as the primary antibody and then with goat-anti-rabbit-HRP (Sigma #A-0545) as the secondary antibody. Fluorescence is read by fluorometer at Ex:550, Em:610, Cutoff:590 (Auto PMT, 15 reads/well) after addition of Amplex-Red. Fluorescence signal is normalized against cell viability derived from Alamar Blue. Data is presented in Table 14 as EC₅₀. Maximum acetylation signal of MS-275 (fluorescence unit) is measured as Eₘₐₓ. The concentration of compound which gives 50% of Eₘₐₓ is EC₅₀. In Table 14, "x" indicates activity of ≤ 1 µM, "y" indicates activity of ≤ 10 µM, and "z" indicates activity of ≤ 20 µM.

**Table 14: In vitro profile of selected HDAC inhibitors.**

| Example | Compd | Structure | HDAC-1 IC₅₀ | MTT HCT116 IC₅₀ | H4-Ac T24 EC vs. MS-275 |
|---|---|---|---|---|---|
| | | | (µM) | (µM) | |
| **4** | **10c*** | 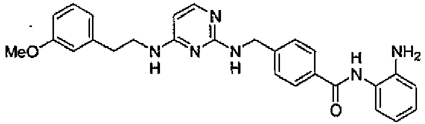 | **c** | **b** | **v** |
| **16** | **13e*** | 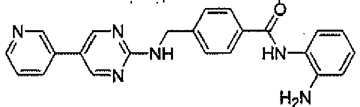 | **c** | **b** | **v** |
| **20** | **16d*** | 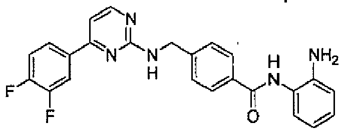 | **b** | **b** | **v** |
| **30** | **26b*** | 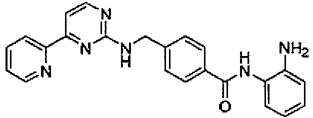 | **c** | **a** | **v** |
| **42** | **44*** | 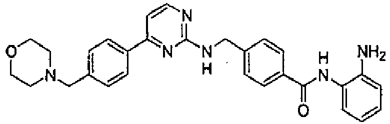 | **c** | **a** | **v** |
| **43** | **47*** | 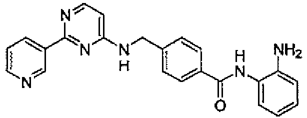 | **c** | **b** | **u** |

| Example | Compd | Structure | HDAC-1 IC₅₀ | MTT HCT116 IC₅₀ | H4-Ac T24 EC vs. MS-275 |
|---|---|---|---|---|---|
| | | | (µM) | (µM) | |
| **48** | **61a*** | 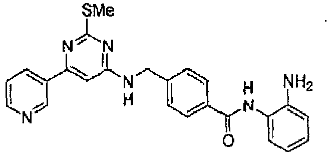 | **c** | **a** | **v** |
| **49** | **61b*** | 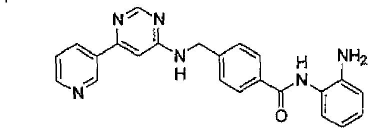 | **c** | **a** | **u** |
| **51** | **63*** | 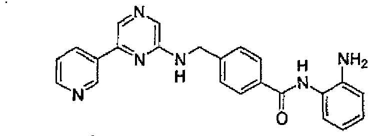 | **c** | **b** | **v** |
| **72** | **134*** | 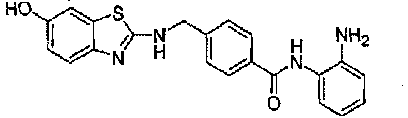 | **c** | **b** | **u** |
| **76** | **138*** | 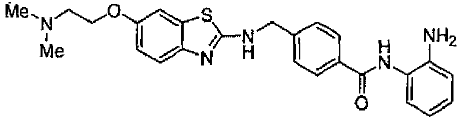 | **c** | **a** | **u** |
| **173** | **308*** | 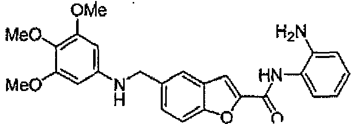 | **c** | **b** | **w** |

| Example | Comp | Structure | HDAC1 IC50 uM | MTT HCT116 IC50 (mM) | H3 Ac t24 (uM) |
|---|---|---|---|---|---|
| 193 | 361 | 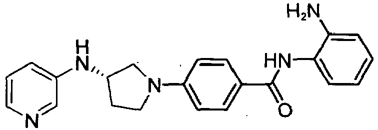 | a | b | y |
| 194 | 366 | 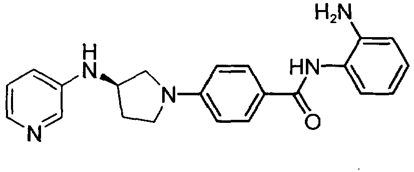 | b | c | z |
| 195 | 367 | 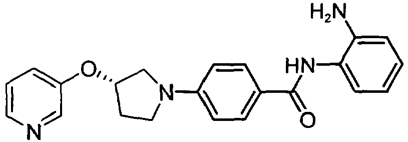 | b | b | y |
| 196 | 368 | 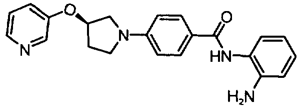 | b | b | |
| 197 | 369 | 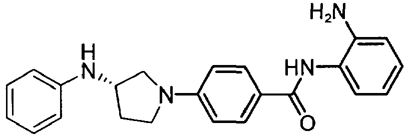 | b | b | x |
| 198 | 370 | 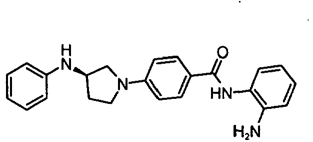 | c | c | |
| 199 | 371 | 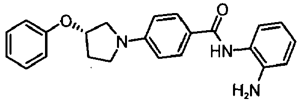 | b | b | |
| 200 | 372 | 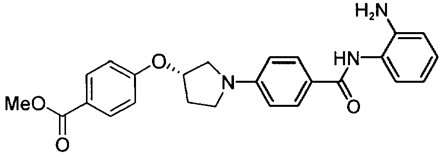 | a | b | |
| 201 | 373 | 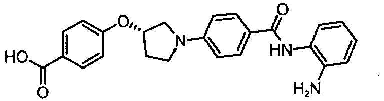 | b | d | |
| 202 | 374 | 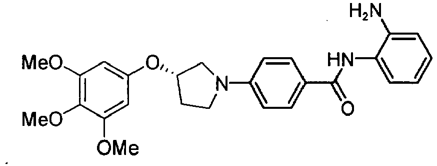 | a | b | x |
| 203 | 375 | 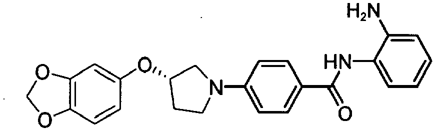 | a | d | x |
| 204 | 376 | 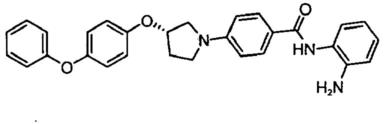 | c | c | |
| 205 | 377 | 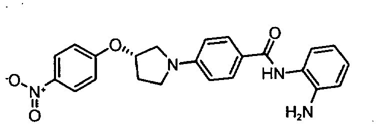 | b | b | |
| 206 | 378 | 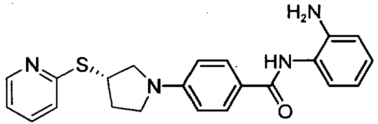 | b | b | y |

| | | | | | |
|---|---|---|---|---|---|
| * = not in accordance with invention | | | | | |

### Assay Example 2

### Antineoplastic Effects of Histone Deacetylase Inhibitors on Human Tumor Xenografts In Vivo

Eight to ten week old female BCD1 mice (Taconic Labs, Great Barrington, NY) were injected subcutaneously in the flank area with 2 x 10⁶ preconditioned HCT116 human colorectal carcinoma cells, SW48 colon cancer cells, and A549 lung cancer cells. Preconditioning of these cells was done by a minimum of three consecutive tumor transplantations in the same strain of nude mice. Subsequently, tumor fragments of approximately 30 mgs were excised and implanted subcutaneously in mice, in the left flank area, under Forene anesthesia (Abbott Labs, Geneva, Switzerland). When the tumors reached a mean volume of 100 mm³, the mice were treated intraperitoneally by daily injection, with a solution of the histone deacetylase inhibitor in DMSO, at a starting dose of 10 mg/kg. The optimal dose of the HDAC inhibitor was established by dose response experiments according to standard protocols. Tumor volume was calculated every second day post infusion according to standard methods (e.g., Meyer et al., Int. J. Cancer 43: 851-856 (1989)). Treatment with the HDAC inhibitors according to the invention caused a significant reduction in tumor weight and volume relative to controls treated with vehicle only (*i*.*e*., no HDAC inhibitor).

## Claims

1. A compound of the formula (1f-1) or a pharmaceutically acceptable salt thereof: 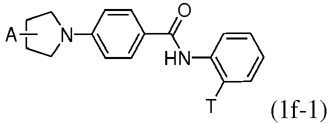 wherein
T is NH₂ or OH, and
A is selected from H, halogen, C₁-C₄ alkyl, optionally substituted alkoxy including aminoalkoxy, haloalkoxy and heteroarylalkoxy, alkoxyalkyl, haloalkyl, amino, nitro, alkylthio, acylamino, carbamoyl,

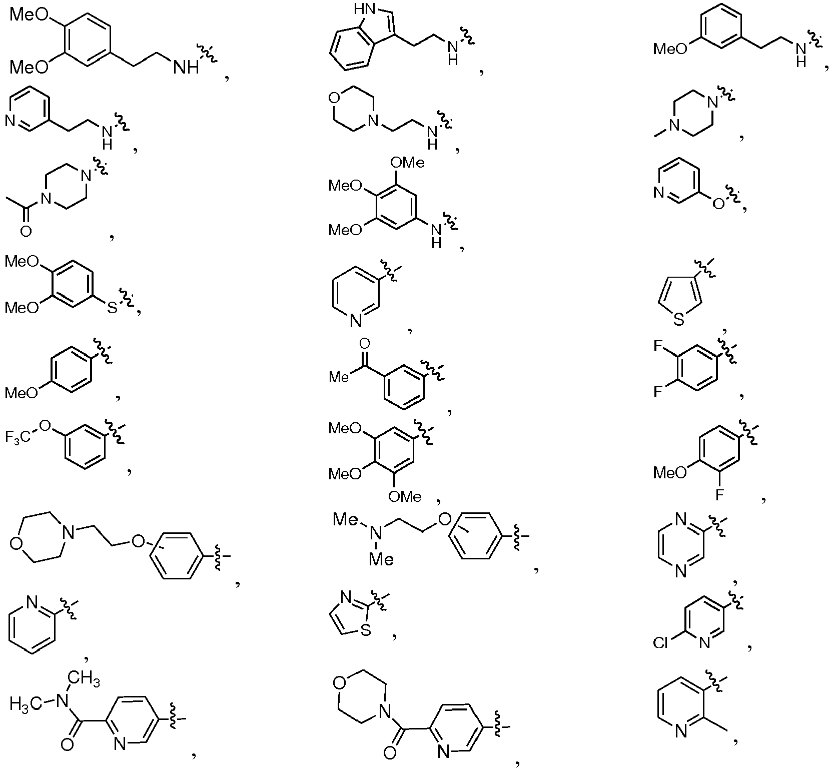

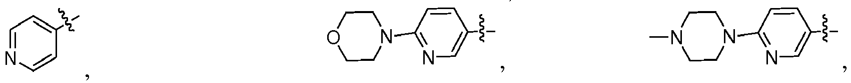

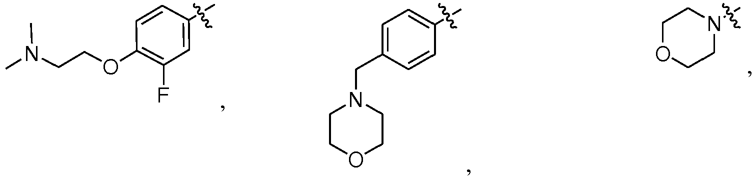

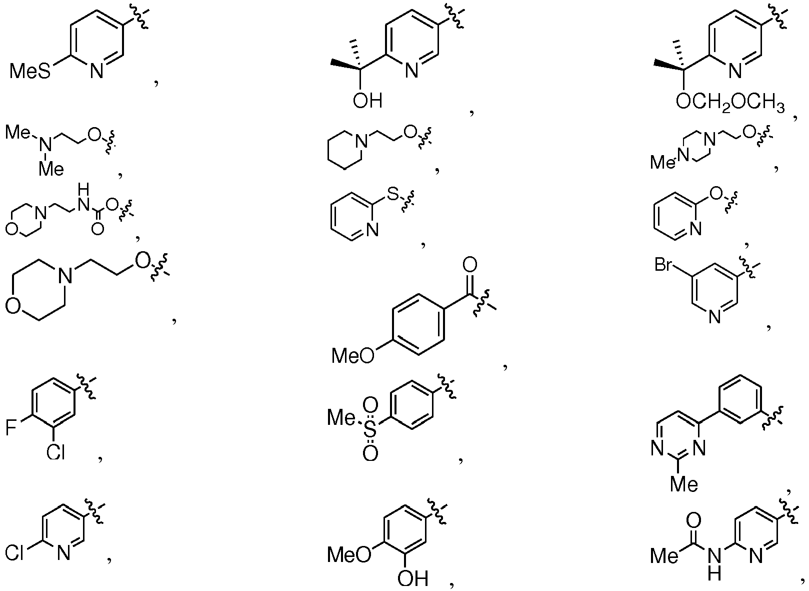

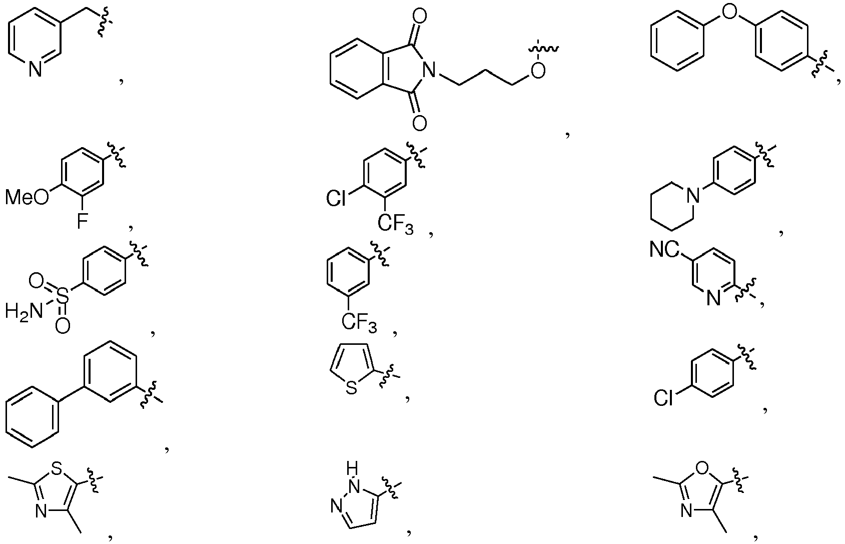

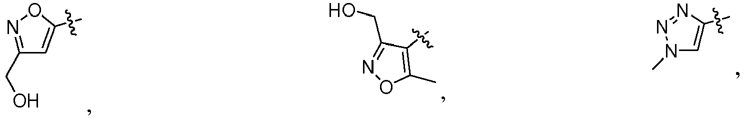

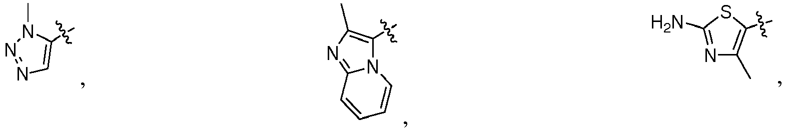

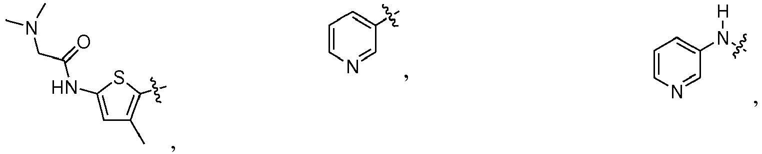

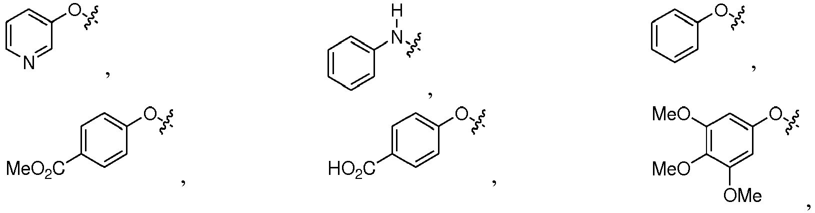

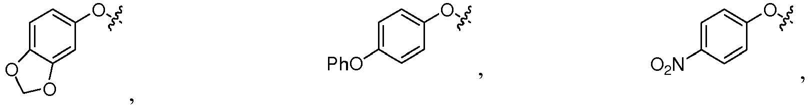

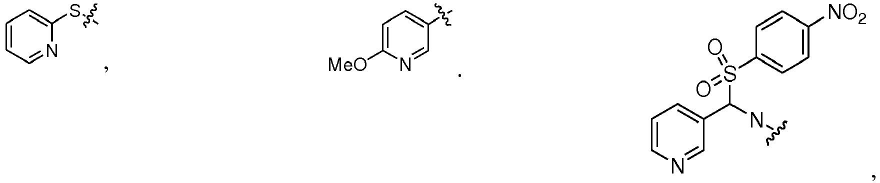

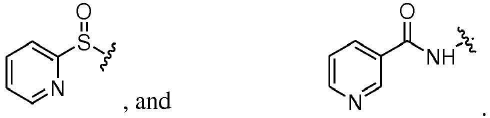

2. A compound according to claim 1 wherein T is NH₂.

3. A compound of formula (2) 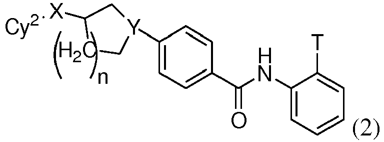 or a pharmaceutically acceptable salt thereof, wherein
Cy² is aryl or heteroaryl, each of which is optionally substituted and wherein each of aryl and heteroaryl is optionally fused to one or more aryl or heteroaryl rings, or to one or more saturated or partially unsaturated cycloalkyl or heterocyclic rings, each of which rings is optionally substituted;
X is selected from the group consisting of: a covalent bond, C₀-C₄-hydrocarbyl, C₀-C₄-hydrocarbyl-(CO)-C₀-C₄-hydrocarbyl, C₀-C₄-hydrocarbyl-(NR⁷)-C₀-C₄-hydrocarbyl, C₀-C₄-hydrocarbyl-(S)-C₀-C₄-hydrocarbyl, C₀-C₄-hydrocarbyl-(O)-C₀-C₄-hydrocarbyl, C₀-C₄-hydrocarbyl-(SO)-C₀-C₄-hydrocarbyl, C₀-C₄-hydrocarbyl-(SO₂)-C₀-C₄-hydrocarbyl, C₀-C₄-hydrocarbyl-(NH)-(CO)-C₀-C₄-hydrocarbyl, C₀-C₄-hydrocarbyl -(CO)-(NH)-C₀-C₄-hydrocarbyl, -NH-CO-NH-, -NH-CS-NH-, -O-CO-O-, -O-CS-O-, -NH-C(NH)-NH-, -S(O)₂-N(R⁷)-, -N(R⁷)-S(O)₂-, -NH-C(O)-O-, and -O-C(O)-NH-,
wherein R⁷ is selected from the group consisting of hydrogen, C₁-C₅-alkyl, aryl, aralkyl, acyl, heterocyclyl, heteroaryl, SO₂-alkyl, SO₂-aryl, CO-alkyl, CO-aryl, CO-NH-alkyl, CO-NH-aryl, CO-O-alkyl and CO-O-aryl, each of which is optionally substituted,
n is 0 to 4,
Y is N or CH, and
T is NH₂ or OH,
wherein
optionally substituted means that the group optionally has from one to four substituents, each selected from
(a) halo, cyano, oxo, alkyl, alkoxy, alkylthio, haloalkoxy, aminoalkyl, aminoalkoxy, carboxy, formyl, nitro, amino, amidino, carbamoyl, guanidino, C₃-C₇ heterocycle, heterocyclylalkyl, heterocyclylcarbonyl, hydroxyalkyl, alkoxyalkyl,
(b) C₁-C₅ alkyl or alkenyl or arylalkyl imino, carbamoyl, carbamate, azido, carboxamido, mercapto, hydroxy, hydroxyalkyl, alkylaryl, arylalkyl, C₁-C₈ alkyl, C₁-C₈ alkenyl, C₁-C₈ alkoxy, C₁-C₈ alkoxycarbonyl, aryloxycarbonyl, C₂-C₈ acyl, C₂-C₈ acylamino, C₁-C₈ alkylthio, arylalkylthio, arylthio, heteroarylthio, C₁-C₈ alkylsulfinyl, arylalkylsulfinyl, arylsulfinyl, C₁-C₈ alkylsulfonyl, arylalkylsulfonyl, arylsulfonyl, C₀-C₆ *N*-alkyl carbamoyl, C₂-C₁₅ *N*,*N*-dialkylcarbamoyl, C₃-C₇ cycloalkyl, aroyl, aryloxy, heteroaryloxy, arylalkyl ether, C₃-C₇ heterocyclylalkylether, aryl, aryl fused to a cycloalkyl or heterocycle or another aryl ring, C₃-C₇ heterocycle, heteroaryl, arylcarbamoyl, or any of these rings fused or spiro-fused to a cycloalkyl, heterocyclyl, or aryl, wherein any of the foregoing which are additionally substitutable is further optionally substituted with one more moieties listed in (a), above; and
(c) -(CH₂)ₛ-NR³⁰R³¹, wherein s is from 0 (in which case the nitrogen is directly bonded to the moiety that is substituted) to 6, and R³⁰ and R³¹ are each independently hydrogen, cyano, oxo, carboxamido, amidino, C₁-C₈ hydroxyalkyl, C₁-C₃ alkylaryl, aryl-C₁-C₃ alkyl, C₁-C₈ alkyl, C₁-C₈ alkenyl, C₁-C₈ alkoxy, C₁-C₈ alkoxycarbonyl, aryloxycarbonyl, aryl-C₁-C₃ alkoxycarbonyl, C₂-C₈ acyl, C₁-C₈ alkylsulfonyl, arylalkylsulfonyl, arylsulfonyl, aroyl, aryl, cycloalkyl, heterocyclyl, or heteroaryl, wherein each of the foregoing is further optionally substituted with one more moieties listed in (a), above; or R³⁰ and R³¹ taken together with the N to which they are attached form a heterocyclyl or heteroaryl, each of which is optionally substituted with from 1 to 3 substituents from (a), above.

4. A compound according to claim 3 having S stereochemistry.

5. A compound according to claim 3 having R stereochemistry.

6. A compound according to claim 3, of the formula, 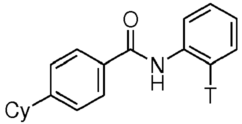 or a pharmaceutically acceptable salt thereof, wherein
Cy is 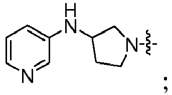 and
T is NH₂ or OH.

7. A compound according to claim 3, selected from the group consisting of:
*N*-(2-Amino-phenyl)-4-[3-(pyridin-3-ylamino)-pyrrolidin-1-yl]-benzamide;
(S)-*N*-(2-Aminophenyl)-4-(3-(pyridin-3-ylamino)pyrrolidin-1-yl)benzamide;
(R)-*N*-(2-aminophenyl)-4-(3-(pyridin-3-ylamino)pyrrolidin-1-yl)benzamide;
(S)-N-(2-Aminophenyl)-4-(3-(pyridin-3-yloxy)pyrrolidin-1-yl)benzamide
(R)-N-(2-Aminophenyl)-4-(3-(pyridin-3-yloxy)pyrrolidin-1-yl)benzamide;
(S)-N-(1-(4-(2-Aminophenyl carbamoyl)phenyl)pyrrolidin-3-yl)nicotinamide;
(S)-N-(2-Aminophenyl)-4-(3-(pyridin-2-ylthio)pyrrolidin-1-yl)benzamide;
N-(2-Aminophenyl)-4-((S)-3-((S)-pyridin-2-ylsulfinyl)pyrrolidin-1-yl)benzamide;
(S)-N-(2-aminophenyl)-4-(3-(phenylamino)pyrrolidin-1-yl)benzamide;
(R)-N-(2-aminophenyl)-4-(3-(phenylamino)pyrrolidin-1-yl)benzamide;
(S)-N-(2-aminophenyl)-4-(3-phenoxypyrrolidin-1-yl)benzamide;
(S)-methyl 4-(1-(4-(2-aminophenylcarbamoyl)phenyl)pyrrolidin-3-yloxy)benzoate;
(S)-4-(1-(4-(2-aminophenylcarbamoyl)phenyl)pyrrolidin-3-yloxy)benzoic acid;
(S)-N-(2-aminophenyl)-4-(3-(3,4,5-trimethoxyphenoxy)pyrrolidin-1-yl)benzamide;
(S)-N-(2-aminophenyl)-4-(3-(benzo[*d*][1,3]dioxol-5-yloxy)pyrrolidin-1-yl)benzamide;
(S)-N-(2-aminophenyl)-4-(3-(4-phenoxyphenoxy)pyrrolidin-1-yl)benzamide; and
(S)-N-(2-aminophenyl)-4-(3-(4-nitrophenoxy)pyrrolidin-1-yl)benzamide.

8. A composition comprising a mixture of enantiomers of compounds of any one of claims 1 - 7.

9. The composition of claim 8 wherein the mixture is racemic.

10. The composition of claim 8 wherein the mixture is enantiomerically enriched.

11. A composition comprising one or more compounds according to any one of claims 1 - 7 and a pharmaceutically acceptable carrier.

12. Use of a composition *in vitro* for treating a cell proliferative disease or condition in a cell, comprising one or more compounds according to any one of claims 1-7 and a pharmaceutically acceptable carrier.

13. A compound according to any one of claims 1-7 or a composition comprising one or more compounds according to any one of claims 1-7 and a pharmaceutically acceptable carrier for use in treating a cell proliferative disease or condition in an animal.

14. The compound or composition for use according to claim 13 wherein the animal is a mammal.

15. The compound or composition for use according to claim 13 wherein the animal is a human.

16. The compound or composition for use according to claim 13 wherein the cell proliferative disease or condition is neoplastic cell proliferation.

17. The compound or composition for use according to claim 13 wherein the cell proliferative disease or condition is cancer, restenosis or psoriasis.

## Patentansprüche

1. Verbindung der Formel (1f-1) oder ein pharmazeutisch unbedenkliches Salz davon: 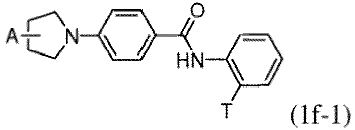 wobei
T für NH₂ oder OH steht und
A aus H, Halogen, C₁-C₄-Alkyl, gegebenenfalls substituiertem Alkoxy einschließlich Aminoalkoxy, Halogenalkoxy und Heteroarylalkoxy, Alkoxyalkyl, Halogenalkyl, Amino, Nitro, Alkylthio, Acylamino, Carbamoyl, 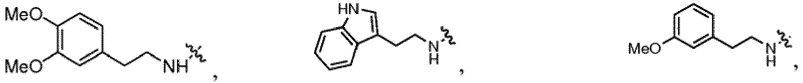 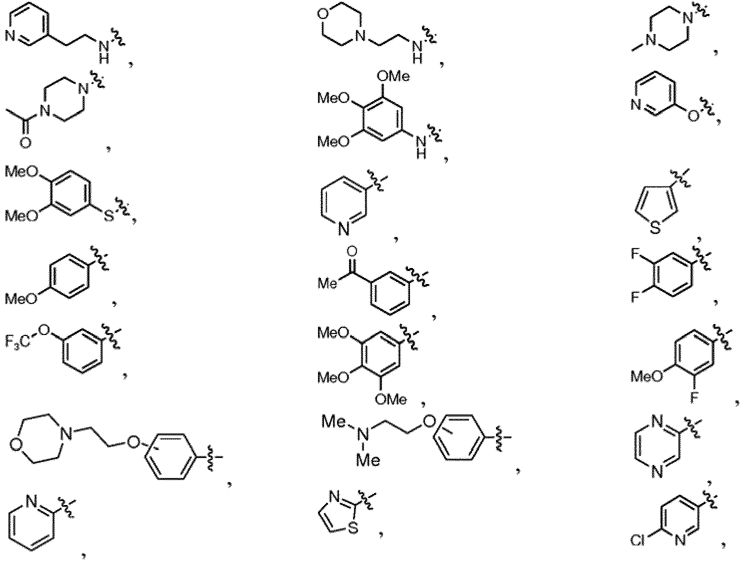 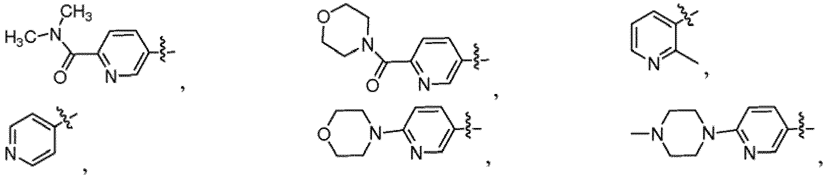 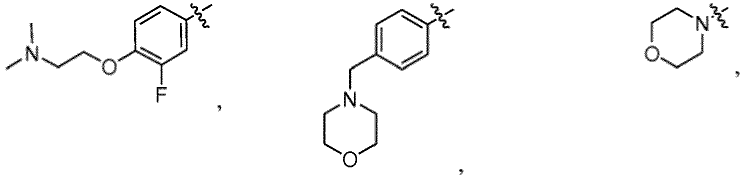 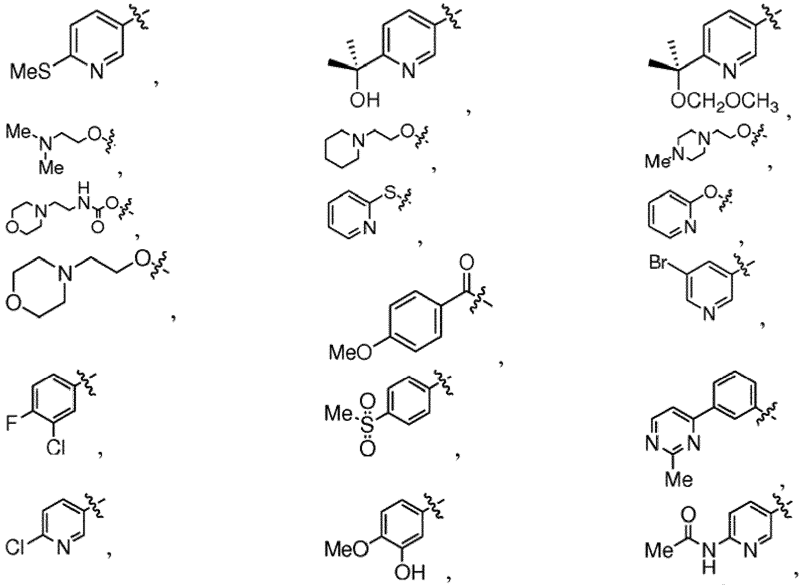 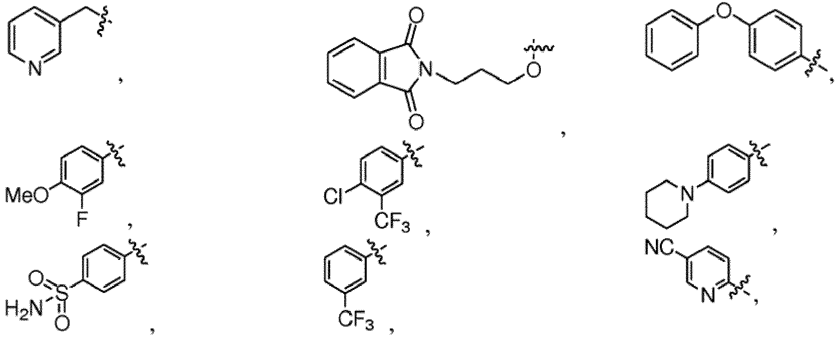 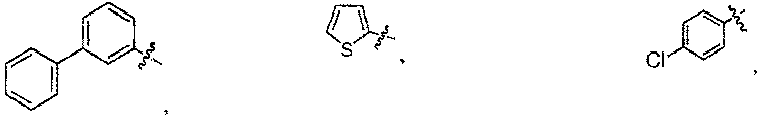 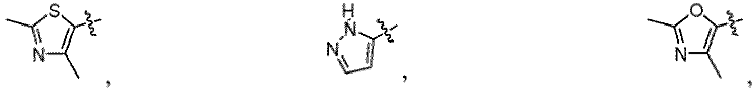 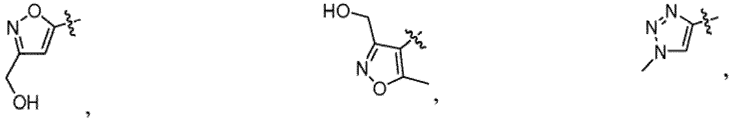 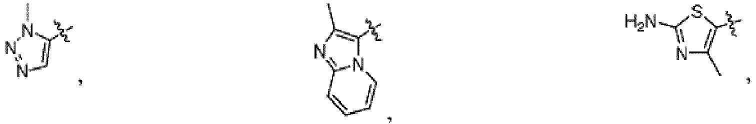 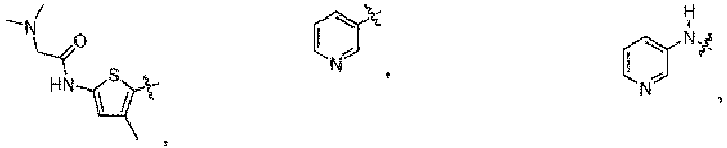 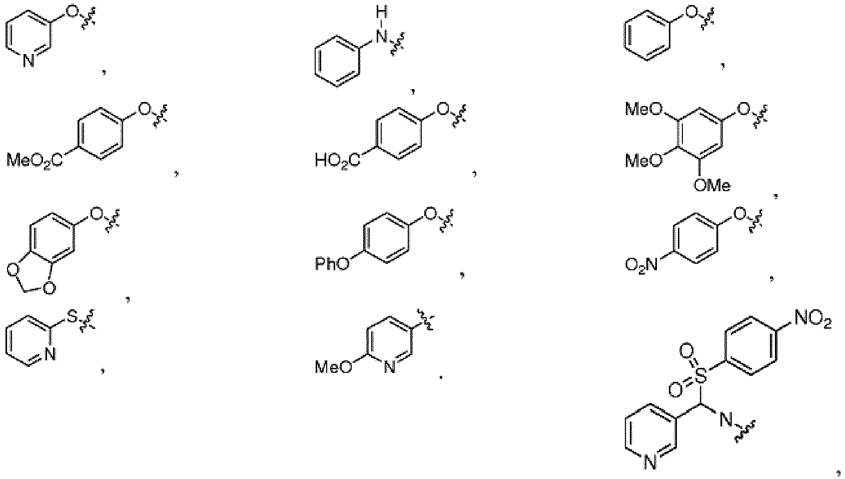 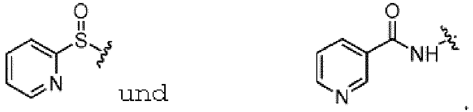 ausgewählt ist.

2. Verbindung nach Anspruch 1, wobei T für NH₂ steht.

3. Verbindung der Formel (2) 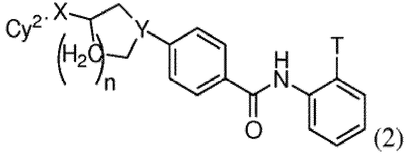 oder ein pharmazeutisch unbedenkliches Salz davon, wobei
Cy² für Aryl oder Heteroaryl steht, wobei jede dieser Gruppen gegebenenfalls substituiert ist und Aryl und Heteroaryl jeweils gegebenenfalls an einen oder mehrere Aryl- oder Heteroarylringe oder an einen oder mehrere gesättigte oder teilweise ungesättigte Cycloalkyringe oder heterocyclische Ringe kondensiert sind, wobei jeder dieser Ringe gegebenenfalls substituiert ist;
X aus der Gruppe bestehend aus einer kovalenten Bindung, C₀-C₄-Hydrocarbyl, C₀-C₄-Hydrocarbyl-(CO)-C₀-C₄-hydrocarbyl, C₀-C₄-Hydrocarbyl-(NR⁷)-C₀-C₄-hydrocarbyl, C₀-C₄-Hydrocarbyl-(S)-C₀-C₄-hydrocarbyl, C₀-C₄-Hydrocarbyl-(O)-C₀-C₄-hydrocarbyl, C₀-C₄-Hydrocarbyl-(SO)-C₀-C₄-hydrocarbyl, C₀-C₄-Hydrocarbyl-(SO₂)-C₀-C₄-hydrocarbyl, C₀-C₄-Hydrocarbyl-(NH)-(CO)-C₀-C₄-hydrocarbyl, C₀-C₄-Hydrocarbyl-(CO)-(NH)-C₀-C₄-hydrocarbyl, -NH-CO-NH-, -NH-CS-NH-, -0-CO-O-, -O-CS-O-, -NH-C(NH)-NH-, -S(O)₂-N(R⁷)-, -N(R⁷)-S(O)₂-, -NH-C(O)-O- und -O-C(O)-NH- ausgewählt ist,
wobei R⁷ aus der Gruppe bestehend aus Wasserstoff, C₁-C₅-Alkyl, Aryl, Aralkyl, Acyl, Heterocyclyl, Heteroaryl, SO₂-Alkyl, SO₂-Aryl, CO-Alkyl, CO-Aryl, CO-NH-Alkyl, CO-NH-Aryl, CO-O-Alkyl und CO-O-Aryl ausgewählt ist, wobei jede dieser Gruppen gegebenenfalls substituiert ist,
n für 0 bis 4 steht,
Y für N oder CH steht und
T für NH₂ oder OH steht,
wobei
gegebenenfalls substituiert bedeutet, dass die Gruppe gegebenenfalls einen bis vier Substituenten aufweist, die jeweils aus
(a) Halogen, Cyano, Oxo, Alkyl, Alkoxy, Alkylthio, Halogenalkoxy, Aminoalkyl, Aminoalkoxy, Carboxy, Formyl, Nitro, Amino, Amidino, Carbamoyl, Guanidino, C₃-C₇-Heterocyclus, Heterocyclylalkyl, Heterocyclylcarbonyl, Hydroxyalkyl, Alkoxyalkyl,
(b) C₁-C₅-Alkyl- oder Alkenyl- oder Arylalkylimino, Carbamoyl, Carbamat, Azido, Carboxamid, Mercapto, Hydroxy, Hydroxyalkyl, Alkylaryl, Arylalkyl, C₁-C₈-Alkyl, C₁-C₈-Alkenyl, C₁-C₈-Alkoxy, C₁-C₈-Alkoxycarbonyl, Aryloxycarbonyl, C₂-C₈-Acyl, C₂-C₈-Acylamino, C₁-C₈-Alkylthio, Arylalkylthio, Arylthio, Heteroarylthio, C₁-C₈-Alkylsulfinyl, Arylalkylsulfinyl, Arylsulfinyl, C₁-C₈-Alkylsulfonyl, Arylalkylsulfonyl, Arylsulfonyl, C₀-C₆-*N*-Alkylcarbamoyl, C₂-C₁₅-*N*,*N*-Dialkylcarbamoyl, C₃-C₇-Cycloalkyl, Aroyl, Aryloxy, Heteroaryloxy, Arylalkylether, C₃-C₇-Heterocyclylalkylether, Aryl, Aryl, das an ein Cycloalkyl oder einen Heterocyclus oder einen weiteren Arylring kondensiert ist, C₃-C₇-Heterocyclus, Heteroaryl, Arylcarbamoyl oder einen beliebigen dieser Ringe, der an ein Cycloalkyl, Heterocyclyl oder Aryl kondensiert oder spiro-kondensiert ist, wobei jede der oben aufgeführten Gruppen, die zusätzlich substituierbar sind, ferner gegebenenfalls durch eine oder mehrere in (a) oben aufgelistete Gruppierungen substituiert ist; und
(c) -(CH₂)ₛ-NR³⁰R³¹, wobei s für 0 (wobei in diesem Fall der Stickstoff direkt an die substituierte Gruppierung gebunden ist) bis 6 steht und R³⁰ and R³¹ jeweils unabhängig für Wasserstoff, Cyano, Oxo, Carboxamido, Amidino, C₁-C₈-Hydroxyalkyl, C₁-C₃-Alkylaryl, Aryl-C₁-C₃-alkyl, C₁-C₈-Alkyl, C₁-C₈-Alkenyl, C₁-C₈-Alkoxy, C₁-C₈-Alkoxycarbonyl, Aryloxycarbonyl, Aryl-C₁-C₃-alkoxycarbonyl, C₂-C₈-Acyl, C₁-C₈-Alkylsulfonyl, Arylalkylsulfonyl, Arylsulfonyl, Aroyl, Aryl, Cycloalkyl, Heterocyclyl oder Heteroaryl stehen, wobei jede der oben aufgeführten Gruppen ferner gegebenenfalls durch eine oder mehrere in (a) oben aufgelistete Gruppierungen substituiert ist; oder R³⁰ und R³¹ zusammen mit dem N, an das sie gebunden sind, ein Heterocyclyl oder Heteroaryl bilden, wobei jede dieser Gruppen gegebenenfalls durch 1 bis 3 Substituenten aus (a) oben substituiert ist;
ausgewählt sind.

4. Verbindung nach Anspruch 3 mit S-Stereochemie.

5. Verbindung nach Anspruch 3 mit R-Stereochemie.

6. Verbindung nach Anspruch 3 der Formel 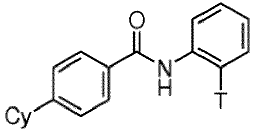 oder ein pharmazeutisch unbedenkliches Salz davon, wobei
Cy für 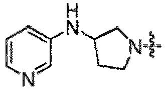 steht und
T für NH₂ oder OH steht.

7. Verbindung nach Anspruch 3, ausgewählt aus der Gruppe bestehend aus:
*N*-(2-Aminophenyl)-4-[3-(pyridin-3-ylamino)-pyrrolidin-1-yl]benzamid;
(S)-*N*-(2-Aminophenyl)-4-(3-(pyridin-3-ylamino)-pyrrolidin-1-yl)benzamid;
(R)-*N*-(2-Aminophenyl)-4-(3-(pyridin-3-ylamino)-pyrrolidin-1-yl)benzamid;
(S)-N-(2-Aminophenyl)-4-(3-(pyridin-3-yloxy)-pyrrolidin-1-yl)benzamid;
(R)-N-(2-Aminophenyl)-4-(3-(pyridin-3-yloxy)-pyrrolidin-1-yl)benzamid;
(S)-N-(1-(4-(2-Aminophenylcarbamoyl)phenyl)-pyrrolidin-3-yl)nicotinamid;
(S)-N-(2-Aminophenyl)-4-(3-(pyridin-2-ylthio)-pyrrolidin-1-yl)benzamid;
N-(2-Aminophenyl)-4-((S)-3-((S)-pyridin-2-yl-sulfinyl)pyrrolidin-1-yl)benzamid;
(S)-N-(2-Aminophenyl)-4-(3-(phenylamino)-pyrrolidin-1-yl)benzamid;
(R)-N-(2-Aminophenyl)-4-(3-(phenylamino)-pyrrolidin-1-yl)benzamid;
(S)-N-(2-Aminophenyl)-4-(3-phenoxypyrrolidin-1-yl)benzamid;
(S)-4-(1-(4-(2-Aminophenylcarbamoyl)phenyl)-pyrrolidin-3-yloxy)benzoesäuremethylester;
(S)-4-(1-(4-(2-Aminophenylcarbamoyl)phenyl)-pyrrolidin-3-yloxy)benzoesäure;
(S)-N-(2-Aminophenyl)-4-(3-(3,4,5-trimethoxy-phenoxy)pyrrolidin-1-yl)benzamid;
(S)-N-(2-Aminophenyl)-4-(3-(benzo[*d*][1,3]dioxol-5-yloxy)pyrrolidin-1-yl)benzamid;
(S)-N-(2-Aminophenyl)-4-(3-(4-phenoxyphenoxy)-pyrrolidin-1-yl)benzamid und
(S)-N-(2-Aminophenyl)-4-(3-(4-nitrophenoxy)-pyrrolidin-1-yl)benzamid.

8. Zusammensetzung, umfassend ein Gemisch von Enantiomeren von Verbindungen nach einem der Ansprüche 1-7.

9. Zusammensetzung nach 8, wobei das Gemisch racemisch ist.

10. Zusammensetzung nach Anspruch 8, wobei das Gemisch enantiomerenangereichert ist.

11. Zusammensetzung, umfassend eine oder mehrere Verbindungen nach einem der Ansprüche 1-7 und einen pharmazeutisch unbedenklichen Träger.

12. Verwendung einer Zusammensetzung in vitro zur Behandlung einer Zellproliferationserkrankung oder eines Zellproliferationsleidens in einer Zelle, umfassend eine oder mehrere Verbindungen nach einem der Ansprüche 1-7 und einen pharmazeutisch unbedenklichen Träger.

13. Verbindung nach einem der Ansprüche 1-7 oder Zusammensetzung, umfassend eine oder mehrere Verbindungen nach einem der Ansprüche 1-7 und einen pharmazeutisch unbedenklichen Träger, zur Verwendung bei der Behandlung einer Zellproliferationserkrankung oder eines Zellproliferationsleidens bei einem Tier.

14. Verbindung oder Zusammensetzung zur Verwendung nach Anspruch 13, wobei es sich bei dem Tier um ein Säugetier handelt.

15. Verbindung oder Zusammensetzung zur Verwendung nach Anspruch 13, wobei es sich bei dem Tier um einen Menschen handelt.

16. Verbindung oder Zusammensetzung zur Verwendung nach Anspruch 13, wobei es sich bei der Zellproliferationserkrankung oder dem Zellproliferationsleiden um neoplastische Zellproliferation handelt.

17. Verbindung oder Zusammensetzung zur Verwendung nach Anspruch 13, wobei es sich bei der Zellproliferationserkrankung oder dem Zellproliferationsleiden um Krebs, Restenose oder Psoriasis handelt.

## Revendications

1. Composé de formule (1f-1) ou sel pharmaceutiquement acceptable de celui-ci : 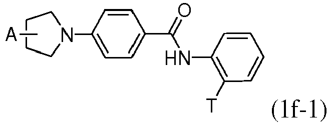 où
T représente NH₂ ou OH, et
A est choisi parmi H, les atomes d'halogène et les groupements alkyle en C₁-C₄, alkoxy éventuellement substitué y compris aminoalkoxy, halogénoalkoxy et hétéroarylalkoxy, alkoxyalkyle, halogénoalkyle, amino, nitro, alkylthio, acylamino, carbamoyle,

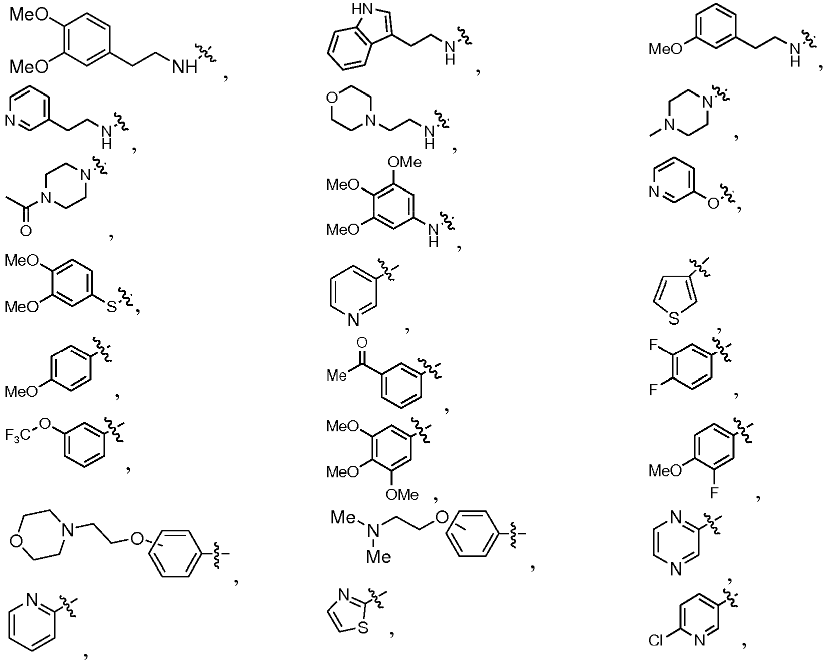

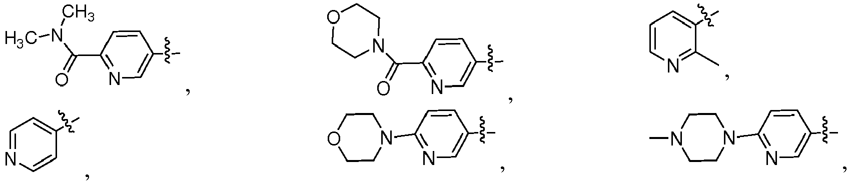

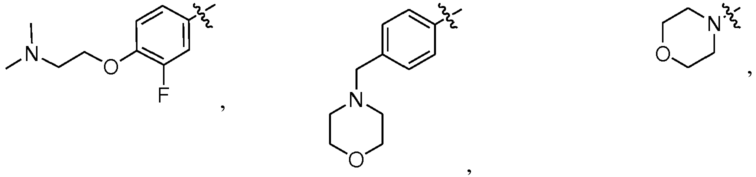

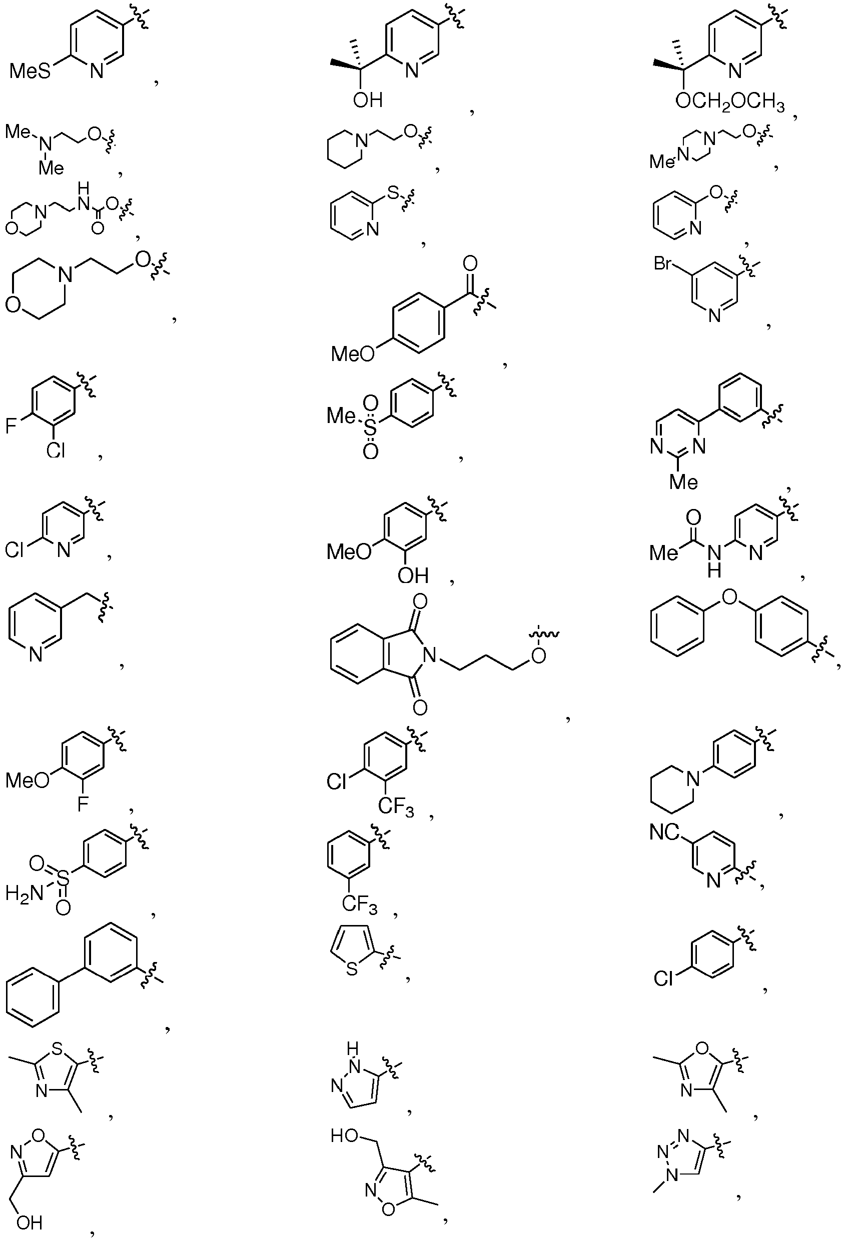

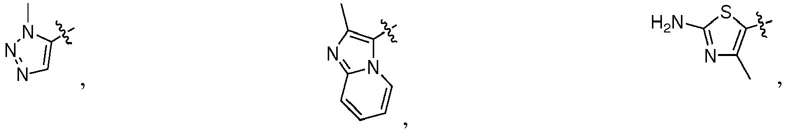

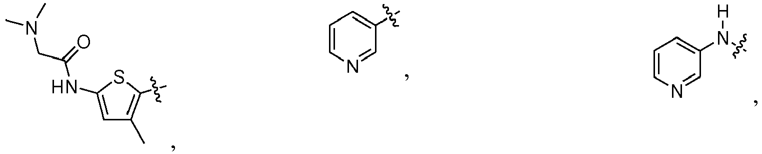

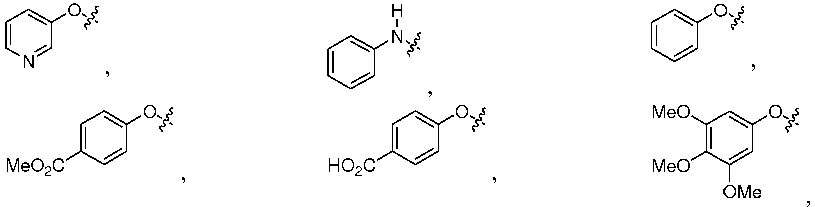

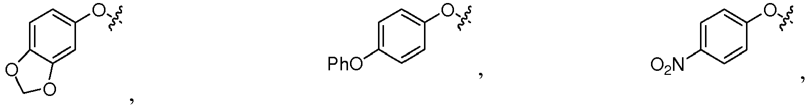

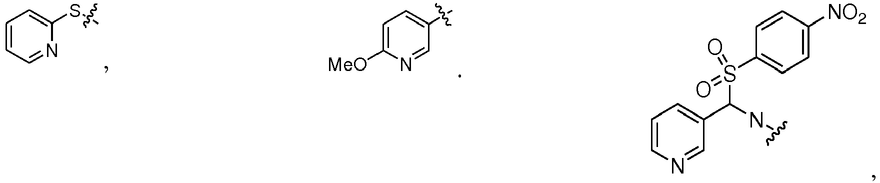

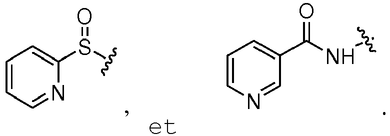

2. Composé selon la revendication 1, où T représente NH₂.

3. Composé de formule (2) 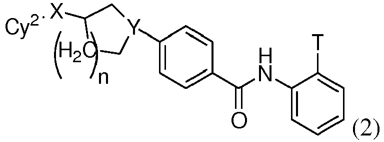 ou sel pharmaceutiquement acceptable de celui-ci, où
Cy² représente un groupement aryle ou hétéroaryle, chacun étant éventuellement substitué et où chacun des groupements aryle et hétéroaryle est éventuellement fusionné à un ou plusieurs cycles aryle ou hétéroaryle, ou à un ou plusieurs cycles cycloalkyle ou hétérocycliques saturés ou partiellement insaturés, chacun desdits cycles étant éventuellement substitué ;
X est choisi dans le groupe constitué par : une liaison covalente ou un groupement hydrocarbyle en C₀-C₄, (hydrocarbyle en C₀-C₄)-(CO)-(hydrocarbyle en C₀-C₄), (hydrocarbyle en C₀-C₄)-(NR⁷)-(hydrocarbyle en C₀-C₄), (hydrocarbyle en C₀-C₄)-(S)-(hydrocarbyle en C₀-C₄), (hydrocarbyle en C₀-C₄)-(O)-(hydrocarbyle en C₀-C₄), (hydrocarbyle en C₀-C₄)-(SO)-(hydrocarbyle en C₀-C₄), (hydrocarbyle en C₀-C₄)-(SO₂)-(hydrocarbyle en C₀-C₄), (hydrocarbyle en C₀-C₄)-(NH)-(CO)-(hydrocarbyle en C₀-C₄), (hydrocarbyle en C₀-C₄)-(CO)-(NH)-(hydrocarbyle en C₀-C₄), -NH-CO-NH-, -NH-CS-NH-, -O-CO-O-, -O-CS-O-, -NH-C(NH)-NH-, -S(O)₂-N(R⁷)-, -N(R⁷)-S(O)₂-, -NH-C(O)-O- et - O-C(O)-NH-,
où R⁷ est choisi dans le groupe constitué par l'atome d'hydrogène et les groupements alkyle en C₁-C₅, aryle, arylalkyle, acyle, hétérocyclyle, hétéroaryle, SO₂-alkyle, SO₂-aryle, CO-alkyle, CO-aryle, CO-NH-alkyl, CO-NH-aryle, CO-O-alkyle et CO-O-aryle, chacun étant éventuellement substitué,
n est compris entre 0 et 4,
Y représente N ou CH, et
T représente NH₂ ou OH,
où
éventuellement substitué signifie que le groupement comporte éventuellement entre un et quatre substituants, chacun étant choisi parmi les suivants :
(a) halogéno, cyano, oxo, alkyle, alkoxy, alkylthio, halogénoalkoxy, aminoalkyle, aminoalkoxy, carboxy, formyle, nitro, amino, amidino, carbamoyle, guanidino, hétérocycle en C₃-C₇, hétérocyclylalkyle, hétérocyclylcarbonyle, hydroxyalkyle, alkoxyalkyle,
(b) alkyle en C₁-C₅ ou alcényle ou arylalkyle imino, carbamoyle, carbamate, azido, carboxamido, mercapto, hydroxy, hydroxyalkyle, alkylaryle, arylalkyle, alkyle en C₁-C₈, alcényle en C₁-C₈, alkoxy en C₁-C₈, (alkoxy en C₁-C₈)-carbonyle, aryloxycarbonyle, acyle en C₂-C₈, (acyle en C₂-C₈)-amino, (alkyle en C₁-C₈)-thio, arylalkylthio, arylthio, hétéroarylthio, (alkyle en C₁-C₈)-sulfinyle, arylalkylsulfinyle, arylsulfinyle, (alkyle en C₁-C₈)-sulfonyle, arylalkylsulfonyle, arylsulfonyle, N-(alkyle en C₀-C₆)-carbamoyle, N, N-(dialkyle en C₂-C₁₅)-carbamoyle, cycloalkyle en C₃-C₇, aroyle, aryloxy, hétéroaryloxy, arylalkyléther, (hétérocyclylalkyle en C₃-C₇)-éther, aryle, aryle fusionné à un groupement cycloalkyle ou hétérocycle ou à un autre cycle aryle, hétérocycle en C₃-C₇, hétéroaryle, arylcarbamoyle, ou l'un quelconque de ces cycles fusionnés ou spiro-fusionnés à un groupement cycloalkyle, hétérocyclyle, ou aryle, où l'un quelconque des groupements précédents qui peuvent être substitués plus avant est en outre éventuellement substitué par une plusieurs entités répertoriées en (a), ci-avant ; et
(c) -(CH₂)ₛ-NR³⁰R³¹, où s est compris entre 0 (auquel cas l'azote est lié directement à l'entité substituée) et 6, et chacun des radicaux R³⁰ et R³¹ représente indépendamment un atome d'hydrogène ou un groupement cyano, oxo, carboxamido, amidino, hydroxyalkyle en C₁-C₈, (alkyle en C₁-C₃)-aryle, aryle-(alkyle en C₁-C₃), alkyle en C₁-C₈, alcényle en C₁-C₈, alkoxy en C₁-C₈, (alkoxy en C₁-C₈)-carbonyle, aryloxycarbonyle, aryl-(alkoxy en C₁-C₃)-carbonyle, acyle en C₂-C₈, (alkyle en C₁-C₈)-sulfonyle, arylalkylsulfonyle, arylsulfonyle, aroyle, aryle, cycloalkyle, hétérocyclyle ou hétéroaryle, où chacun des groupements précédents est en outre éventuellement substitué par une ou plusieurs entités répertoriées en (a) ci-avant ; ou R³⁰ et R³¹ forment ensemble et avec l'atome N auquel ils sont liés un groupement hétérocyclyle ou hétéroaryle, chacun étant éventuellement substitué par entre 1 et 3 substituants de (a) ci-avant.

4. Composé selon la revendication 3, présentant une stéréochimie S.

5. Composé selon la revendication 3, présentant une stéréochimie R.

6. Composé selon la revendication 3, répondant à la formule 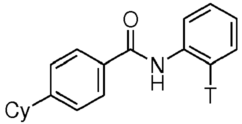 ou sel pharmaceutiquement acceptable de celui-ci, où
Cy représente 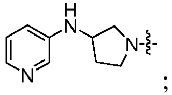 et
T représente NH₂ ou OH.

7. Composé selon la revendication 3, choisi dans le groupe constitué par les suivants :
N-(2-Amino-phényl)-4-[3-(pyridin-3-ylamino)-pyrrolidin-1-yl]-benzamide ;
(S)-N-(2-Aminophényl)-4-(3-(pyridin-3-ylamino)pyrrolidin-1-yl)benzamide ;
(R)-N-(2-aminophényl)-4-(3-(pyridin-3-ylamino)pyrrolidin-1-yl)benzamide ;
(S)-N-(2-Aminophényl)-4-(3-(pyridin-3-yloxy)pyrrolidin-1-yl)benzamide
(R)-N-(2-Aminophényl)-4-(3-(pyridin-3-yloxy)pyrrolidin-1-yl)benzamide ;
(S)-N-(1-(4-(2-Aminophénylcarbamoyl)phényl)pyrrolidin-3-yl)nicotinamide ;
(S)-N-(2-Aminophényl)-4-(3-(pyridin-2-ylthio)pyrrolidin-1-yl)benzamide ;
N-(2-Aminophényl)-4-((S)-3-((S)-pyridin-2-ylsulfinyl)pyrrolidin-1-yl)benzamide ;
(S)-N-(2-aminophényl)-4-(3-(phénylamino)pyrrolidin-1-yl)benzamide ;
(R)-N-(2-aminophényl)-4-(3-(phénylamino)pyrrolidin-1-yl)benzamide ;
(S)-N-(2-aminophényl)-4-(3-phénoxypyrrolidin-1-yl)benzamide ;
(S)-4-(1-(4-(2-aminophénylcarbamoyl)phényl)pyrrolidin-3-yloxy)benzoate de méthyle ;
acide (S)-4-(1-(4-(2-aminophénylcarbamoyl)phényl)pyrrolidin-3-yloxy)benzoïque ;
(S)-N-(2-aminophényl)-4-(3-(3,4,5-triméthoxyphénoxy)pyrrolidin-1-yl)benzamide ;
(S)-N-(2-aminophényl)-4-(3-(benzo[d][1,3]dioxol-5-yloxy)pyrrolidin-1-yl)benzamide ;
(S)-N-(2-aminophényl)-4-(3-(4-phénoxyphénoxy)pyrrolidin-1-yl)benzamide ; et
(S)-N-(2-aminophényl)-4-(3-(4-nitrophénoxy)pyrrolidin-1-yl)benzamide.

8. Composition comprenant un mélange d'énantiomères de composés selon l'une quelconque des revendications 1 à 7.

9. Composition selon la revendication 8, où le mélange est racémique.

10. Composition selon la revendication 8, où le mélange est énantiomériquement enrichi.

11. Composition comprenant un ou plusieurs composés selon l'une quelconque des revendications 1 à 7 ainsi qu'un vecteur pharmaceutiquement acceptable.

12. Utilisation d'une composition *in vitro* dans le traitement d'une maladie proliférative cellulaire ou d'un état pathologique au sein d'une cellule, comprenant un ou plusieurs composés selon l'une quelconque des revendications 1 à 7 ainsi qu'un vecteur pharmaceutiquement acceptable.

13. Composé selon l'une quelconque des revendications 1 à 7 ou composition comprenant un ou plusieurs composés selon l'une quelconque des revendications 1 à 7 et vecteur pharmaceutiquement acceptable pour utilisation dans le traitement d'une maladie proliférative des cellules ou d'un état pathologique chez un animal.

14. Composé ou composition pour utilisation selon la revendication 13, où l'animal est un mammifère.

15. Composé ou composition pour utilisation selon la revendication 13, où l'animal est un humain.

16. Composé ou composition pour utilisation selon la revendication 13, où la maladie proliférative cellulaire ou l'état pathologique est une prolifération de cellules néoplasiques.

17. Composé ou composition pour utilisation selon la revendication 13, où la maladie proliférative cellulaire ou l'état pathologique est le cancer, la resténose ou le psoriasis.
